(19) Europäisches Patentamt
European Patent Office
Office européen des brevets

(11) EP 4 660 185 A1

(12) **EUROPEAN PATENT APPLICATION**
published in accordance with Art. 153(4) EPC

(43) Date of publication:
**10.12.2025 Bulletin 2025/50**

(21) Application number: **24749441.2**

(22) Date of filing: **30.01.2024**

(51) International Patent Classification (IPC):
***C07C 233/64*** (2006.01) ***C07C 15/00*** (2006.01)
***C07C 15/12*** (2006.01) ***C07D 213/00*** (2006.01)
***C07D 401/00*** (2006.01) ***C07D 403/00*** (2006.01)
***A61K 31/166*** (2006.01) ***A61K 31/435*** (2006.01)
***A61K 31/495*** (2006.01) ***A61K 31/609*** (2006.01)
***A61P 25/02*** (2006.01) ***A61P 25/04*** (2006.01)
***A61P 29/00*** (2006.01)

(52) Cooperative Patent Classification (CPC):
**A61K 31/166; A61K 31/435; A61K 31/495; A61K 31/609; A61P 25/02; A61P 25/04; A61P 29/00; C07C 15/00; C07C 15/12; C07C 233/64; C07D 213/00; C07D 401/00; C07D 403/00**

(86) International application number:
**PCT/BR2024/050028**

(87) International publication number:
**WO 2024/159288 (08.08.2024 Gazette 2024/32)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **30.01.2023 US 202363482226 P**

(71) Applicants:
- **Eurofarma Laboratórios S.A.**
  **06696-000 São Paulo - SP (BR)**
- **Universidade Federal Do Rio De Janeiro - UFRJ**
  **21941-853 Rio de Janeiro -RJ (BR)**

(72) Inventors:
- **BARREIRO, Gabriela**
  **04514-032 São Paulo - SP (BR)**
- **SANT'ANA, Danilo Pereira De**
  **06709-320 São Paulo - SP (BR)**
- **MONTEIRO, Júlia Lammoglia**
  **06704-175 São Paulo - SP (BR)**
- **GAMBA, Luis Eduardo Reina**
  **06404-326 São Paulo - SP (BR)**
- **FRAGA, Carlos Alberto Manssour**
  **(deceased) (BR)**
- **BARREIRO, Eliezer Jesus De Lacerda**
  **(deceased) (BR)**
- **LIMA, Lídia Moreira**
  **21931-220 Rio de Janeiro - RJ (BR)**

(74) Representative: **Engelhard, Markus**
**Boehmert & Boehmert**
**Anwaltspartnerschaft mbB**
**Pettenkoferstrasse 22**
**80336 München (DE)**

(54) **NAV1.7- AND/OR NAV1.8-INHIBITING AMIDES, PROCESSES FOR THE PREPARATION THEREOF, COMPOSITIONS, USES, METHODS FOR TREATMENT USING SAME, AND KITS**

(57) The present invention relates to Nav 1.7 and/or Nav 1.8 blocking amides. More specifically, the present invention reports amides comprising Formula (I), in which the substituents $R_1$ to $R_{27}$ are selected independently of the groups defined in the specification, as well as their processes of obtaining, compositions comprising at least one of these compounds, uses, treatment methods to treat or prevent pain-related pathologies, and kits. The present invention belongs to the fields of medicinal chemistry, organic synthesis, as well as the treatment of pain-related diseases.



Processed by Luminess, 75001 PARIS (FR)

**(Cont. next page)**

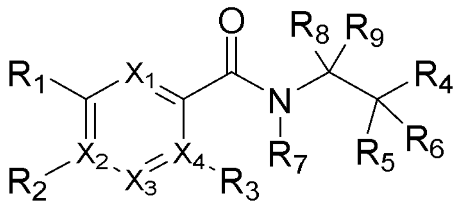

Formula (Ia)

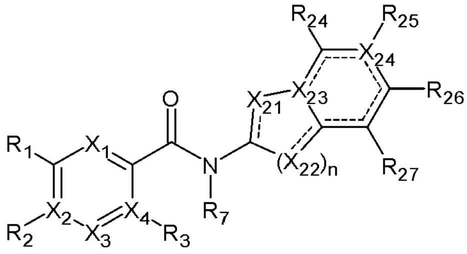

Formula (Ib)

## Description

### FIELD OF INVENTION

The present invention refers to Nav 1.7 and/or Nav 1.8 blocking amides, their preparation processes, compositions containing these, uses, kits and treatment methods to treat or prevent pain-related pathologies. The present invention belongs to the fields of medicinal chemistry, organic synthesis, as well as the treatment of pain-related diseases.

### BACKGROUND OF INVENTION

Physiological pain is an important protective mechanism designed to alert the body to real or potential injuries that can put its integrity at risk. Broadly speaking, physiological pain can be classified into nociceptive pain and inflammatory pain. Nociceptive pain is characterized by having a high activation threshold, which remains until the stimulus that generated it is eliminated. Inflammatory pain, which arises as a response to tissue damage, is characterized by having a low activation threshold and is a consequence of the activity of molecular mediators of the inflammatory process in sensitizing nociceptors (Schaible. Langenbecks Arch. Surg. 2004, 389, 237). When these nociceptive processes remain in the absence of noxious stimuli or in response to non noxious stimuli, the protective and repair role of pain loses its functionality, configuring a maladaptive picture of neural plasticity and, as a consequence, a pathological state of chronic pain. Among the syndromes included in this classification, neuropathic pain has a high prevalence and impact today (Smith. Pain. 2020, 161, 1:S127; Cavalli. Int. J. Immunopathol. Pharmacol. 2019, 33:2058738419838383; Bouhassira. Rev Neurol (Paris). 2019, 175(1-2):16; Scholz. Nature Neurosci., 2002, 5,1062; Costigan. Annu. Rev. Neurosci., 2009, 32, 1).

Neuropathic pain is defined by the International Association for the Study of Pain (IASP) as pain initiated or caused by a primary dysfunction or injury in the central and/or peripheral nervous system (Dworkin. Clin. J. Pain, 2002, 18(6), 343). Central neuropathic pain comes from spinal cord injuries or central nervous system diseases such as multiple sclerosis or Parkinson's disease (Ducreux. Brain, 2006, 129, 963). Peripheral neuropathic pain, on the other hand, can be caused by trauma, metabolic disorders, chemical neurotoxicity, infection, or tumor invasion, among others. Among the most common syndromes of neuropathic pain are chemotherapy-induced neuropathic pain, complex regional pain, neuropathy related to viral infection, neuropathy secondary to tumor infiltration, diabetic neuropathy, phantom limb pain, postherpetic neuralgia, trigeminal neuralgia, and postsurgical neuralgia (Pak. Curr. Pain Headache Rep., 2018, 22(2), 9).

Currently, there is no specific treatment for the control of pathologies related to neuropathic pain, however, the first-line alternative consists of the use of opioid analgesics, and - as adjuvants - local anesthetics, anticonvulsants and antidepressants. However, the adverse effects and low efficacy drastically limit the use of these agents in the control of various pain-related pathologies (Kushnarev. Expert Opin. Investig. Drugs, 2020, 29(3), 259; Emery. Expert Opin. Ther. Targets, 2016, 20(8), 975).

Voltage-gated sodium channels (Nav) play a key role in the transmission of pain-related stimuli. These channels are activated in response to membrane depolarization, allowing the generation and propagation of action potentials in neurons (and other electrically excitable cells), by controlling the flow of sodium ions through the membranes. Structurally, voltage-gated sodium channels are heteromeric transmembrane proteins consisting of one $\alpha$ subunit and two $\beta$ helper subunits. The $\alpha$ subunit is organized into four homologous domains (I-IV), each with six transmembrane segments (S1-S6). The S4 segment of each domain is characterized by presenting a conserved region of arginine residues, which act as sensors of the intra- and extracellular electrical environment of the neuron. This mechanism makes it possible to transform changes in the cellular electric field into specific conformational changes that, in turn, regulate the activation, deactivation and inactivation of voltage-gated sodium channels (Catterall. Nat. Chem. Biol., 2020, 16, 1314; Wisedchaisri. Cell., 2019, 178(4), 993; Clairfeuille. Science, 2019, 363, 1302).

In mammals, nine subunits $\alpha$ (Nav 1.1 - Nav 1.9) and four auxiliary subunits $\beta$ ($\beta$1-$\beta$4) have been identified. The $\alpha$ subunits can further be classified according to their susceptibility to blocking by tetrodotoxin (TTX), being classified as sensitive to tetrodotoxin (Nav 1.1, Nav 1.2, Nav 1.3, Nav 1.4, Nav 1.6 and Nav 1.7) or resistant to tetrodotoxin (Nav 1.5, Nav 1.8 and Nav 1.9) (Lera-Ruiz. J. Med. Chem., 2015, 58(18), 7093; Bagal. J. Med. Chem., 2013, 56(3), 593). Each of these subunits $\alpha$ has a different profile of expression and function, so that some of them are essential for the proper functioning of organs such as the heart and/or brain. Thus, the non-selective blockage of these channels is related to several types of adverse effects, such as migraine, epilepsy, paralysis and muscle and cardiac syndromes, among others (Bagal. J. Med. Chem., 2013, 56(3), 593; Bagal. Channels, 2015, 9(6), 360).

Broadly speaking, sodium channels are distributed mainly in the central and peripheral nervous system, in neurons and glia. Nav channels 1.1, 1.2, and 1.3 are primarily expressed in the brain. The Nav 1.4 and Nav 1.5 channels are found primarily in skeletal and cardiac muscles, respectively. Nav 1.6 channels are expressed in the central and peripheral nervous systems, while Nav 1.9 channels are selectively expressed in C-type nociceptive fibers in the dorsal root ganglion. On the other hand, the Nav 1.7 and Nav 1.8 channels are mainly found in the peripheral nervous system and

are directly related to the processes of pain transmission (Law. Drug Discovery Today, 2019, 24(7), 1389; Bagal. Channels (Austin), 2015, 9(6), 360; Lera-Ruiz. J. Med. Chem., 2015, 58(18), 7093).

Nav 1.7 sodium channels are expressed broadly in the olfactory epithelium, sympathetic ganglion, and dorsal root ganglion, predominantly in nociceptive fibers C and A$\delta$. A large amount of evidence supports the important role of Nav 1.7 sodium channels in pain transmission processes. For example, gain-of-function related mutations in the gene (SCN9A), which encodes sodium channel Nav 1.7, are associated with extreme pain disorders such as congenital pain insensitivity, paroxysmal extreme pain disorder, and primary erythromelalgia. On the other hand, mutations related to loss of gene function (SCN9A) are related to congenital insensitivity to pain in individuals who, in general terms, are free of motor or cognitive impairment (Vetter. Pharmacology & Therapeutics, 2017, 172, 73; Ahuja. Science, 2015, 350(6267), 1491; Kingwell. Nat. Rev. Drug Discov., 2019, 18, 321; Safina. J. Med. Chem., 2021, 64, 2953; Luo. J. Med. Chem., 2019, 62, 831; Bankar. Cell Reports, 2018, 24, 3133).

Nav 1.8 sodium channels are most expressed in the peripheral nervous system, widely (but not exclusively) in C-type nociceptive fibers in the dorsal root ganglion. Recent evidence including elevated expression levels of Nav 1.8 in chronic pain states, data with Nav 1.8 *knockout* animals, and analgesic activity of Nav 1.8-specific desensitizing oligodeoxynucleotides, among others (Brown. Bioorg. Med. Chem., 2019, 27(1), 230; Payne. Br. J. Pharmacol., 2015, 172(10), 2654; Bagal. Med. Chem. Lett. 2015, 6(6) 650; Kort. J. Med. Chem. 2008, 51, 407; Zhang. Neuropharmacology, 2010, 59, 201 and 207), support the role of the sodium channel Nav 1.8 in the development and process of pain-related pathologies (Kingwell. Nat. Rev. Drug Discov., 2019, 18, 321; Law. Drug Discovery Today, 2019, 24(7), 1389; Bagal. Channels (Austin), 2015, 9(6), 360; Lera-Ruiz. J. Med. Chem., 2015, 58(18), 7093).

Thus, the voltage-gated sodium channels Nav 1.7 and Nav 1.8 are considered promising therapeutic targets for the treatment of neuropathic pain-related dysfunctions (Kornecook. J. Pharmacol. Exp. Ther., 2017, 362, 146; Kingwell. Nat. Rev. Drug Discov., 2019, 18, 321; Bagal. Channels (Austin), 2015, 9(6), 360; Lera-Ruiz. J. Med. Chem., 2015, 58(18), 7093; Deuis. Neuropharmacology, 2017, 127, 87 and 108; Kushnarev. Expert Opin. Investig. Drugs, 2020, 29(3), 259; McKerrall. Bioorganic & Medicinal Chemistry Lett., 2018, 28, 3141; Emery. Expert Opin. Ther. Targets, 2016, 20(8), 975; Bagal. Bioorganic & Medicinal Chemistry Lett., 2014, 24, 3690).

A large number of compounds have been described in the literature for their ability to act as blockers of Nav 1.7 and 1.8 sodium channels, however, they present a great structural diversity, a fact that does not allow the establishment of a common pharmacophoric group.

The patent literature contains several examples of compounds that act as sodium channel blockers. In particular, Nav 1.7 selective sodium channel blockers are described in US10550080, US9765029, and US10000475. Additionally, some documents describe selective blockers of Nav 1.8 sodium channels as WO2020261114, WO2020092667, US9163042, WO2014120808, WO2014120815, WO2018213426, WO2019014352, WO2015006280, and US7928107. These documents reveal compounds with different structures from the present invention.

In addition, there are patent documents that describe dual Nav blockers 1.7 and 1.8, including WO2018235851, US8629149, JP2017001991 that reveal, respectively, pyridyl amines, oxopiperazine derivatives and benzoxazolons. However, all these documents reveal compounds with structures and physicochemical characteristics different from the present invention.

In this context, it is advantageous to develop new alternatives of compounds that can act as Nav 1.7 and/or Nav 1.8 blockers that have adequate pharmacological action and, preferably, provide mitigated adverse effects. Therefore, the present invention refers to amides as an alternative and/or complement to the treatment of pain-related diseases.

## SUMMARY OF THE INVENTION

The present invention discloses amides with blocking activity of Nav 1.7 and/or 1.8 channels, against pain-related pathologies, as well as related compositions, uses, kits, treatment methods and preparation processes.

The present invention refers to compound(s) of Formula (Ia):

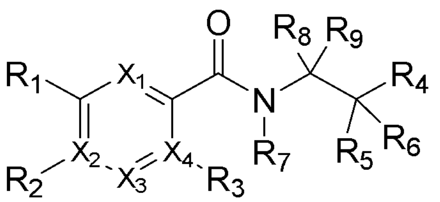

Formula (Ia)

or a pharmaceutically acceptable salt, hydrate, solvate, ester and isomer thereof, wherein:

- $X_1$, $X_2$, $X_3$, and $X_4$ are independently selected from the group consisting of carbon, CH, or nitrogen, wherein

- when at least one of the substituents $X_2$, $X_4$ is nitrogen or CH, the corresponding R ($R_2$, $R_3$) is null,
- when two of the substituents $X_1$, $X_2$, $X_3$ and $X_4$ are nitrogen, the remaining substituents $X_1$, $X_2$, $X_3$ or $X_4$ must be carbon or CH.
- $R_1$ is:

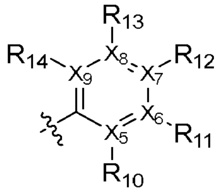
,

wherein:

- $X_5$, $X_6$, $X_7$, $X_8$, and $X_9$ are independently selected from the group consisting of carbon, CH, or nitrogen, wherein
- when at least one of the substituents $X_5$-$X_9$ is nitrogen or CH, the corresponding R ($R_{10}$-$R_{14}$) is null,
- $R_{10}$, $R_{11}$, $R_{12}$, $R_{13}$, and $R_{14}$ are independently selected from the group consisting of hydrogen, halogen, linear or branched $C_1$-$C_6$ alkyl, alkoxy $C_1$-$C_6$ linear or branched, hydroxy, hydroxyalkyl $C_1$-$C_4$, haloaloxy $C_1$-$C_6$ linear or branched, $-OCD_2CD_3$ or $-OCD_3$;
- $R_2$ and $R_3$ are independently selected from the group consisting of hydrogen, halogen, amine, $C_1$-$C_6$ alkoxy, and $C_1$-$C_6$;
- $R_4$ is:

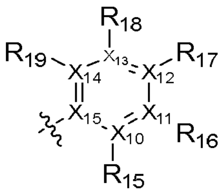
or
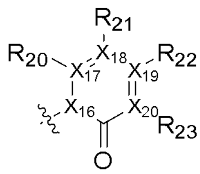
,

wherein

- $X_{10}$, $X_{11}$, $X_{12}$, $X_{13}$, $X_{14}$, $X_{15}$, $X_{16}$, $X_{17}$, $X_{18}$, $X_{19}$ and $X_{20}$ are independently selected from the group consisting of carbon, CH or nitrogen, wherein
- when at least one of the substituents $X_{10}$, $X_{11}$, $X_{12}$, $X_{13}$, $X_{14}$, $X_{17}$, $X_{18}$, $X_{19}$ or $X_{20}$ is N or CH, the corresponding R ($R_{15}$-$R_{23}$) is null;
- $R_{15}$, $R_{16}$, $R_{17}$, $R_{18}$, $R_{19}$, $R_{20}$, $R_{21}$, $R_{22}$, $R_{23}$ are independently selected from the group consisting of hydrogen, halogen, oxygen, alkyl $C_1$-$C_6$ linear or branched, $C_1$-$C_6$ linear or branched alkoxy, hydroxyalkyl $C_{1-}C_4$ linear or branched, $-OCD_2CD_3$ or $-OCD_3$;
- $R_5$ and $R_6$ are independently selected from the group consisting of hydrogen, hydroxy, carbonyl, amine, linear or branched $C_1$-$C_6$alkyl, wherein
- when $R_5$ or $R_6$ are carbonyl, the other R must be null;
- or $R_4$, $R_5$, and $R_6$ together form one of the following bicycles:

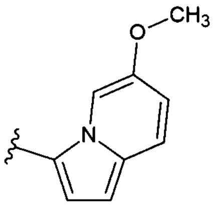
,
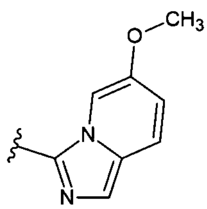
or
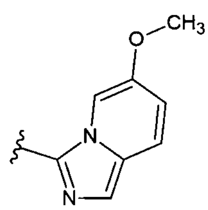
;

and

- $R_7$, $R_8$, and $R_9$ are independently selected from the group consisting of hydrogen and linear or branched $C_1$-$C_6$ alkyl.

The present invention further refers to compound(s) of Formula (Ib):

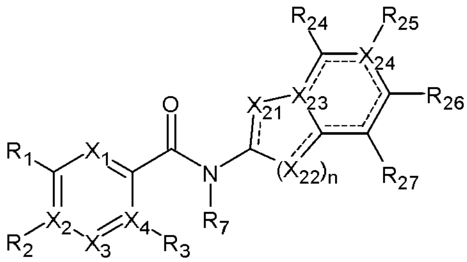

Formula (Ib)

or a pharmaceutically acceptable salt, hydrate, solvate, ester and isomer thereof, in which

- $X_1$, $X_2$, $X_3$, and $X_4$ are independently selected from the group consisting of carbon, CH, or nitrogen, wherein
- when at least one of the substituents $X_2$, $X_4$ is nitrogen or CH, the corresponding R ($R_2$, $R_3$) is null;
- when two of the substituents $X_1$, $X_2$, $X_3$ and $X_4$ are nitrogen, the remaining substituents $X_1$, $X_2$, $X_3$ or $X_4$ must be carbon or CH.
- $R_1$ is:

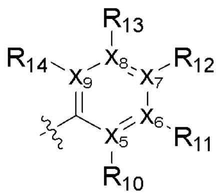

,

wherein

- $X_5$, $X_6$, $X_7$, $X_8$, and $X_9$ are independently selected from the group consisting of carbon, CH, or nitrogen, wherein
- when at least one of the substituents $X_5$-$X_9$ is nitrogen or CH, the corresponding R ($R_{10}$-$R_{14}$) is null,
- $R_{10}$, $R_{11}$, $R_{12}$, $R_{13}$, and $R_{14}$ are independently selected from the group consisting of hydrogen, halogen, linear or branched $C_1$-$C_6$ alkyl, alkoxy $C_1$-$C_6$ linear or branched, hydroxyalkyl $C_1$-$C_4$, haloalkoxy $C_1$-$C_6$ linear or branched, $-OCD_2CD_3$ or $-OCD_3$;
- $R_2$ and $R_3$ are independently selected from the group consisting of hydrogen, halogen, amine, $C_1$-$C_6$ alkoxy, and $C_1$-$C_6$ alkyl linear or branched;
- $R_7$ is selected from a group consisting of hydrogen and $C_1$-$C_6$ alkyl linear or branched;
- $X_{21}$, $X_{22}$, $X_{23}$, and $X_{24}$ are independently selected from the group consisting of carbon, CH, nitrogen, oxygen, or sulfur or $NCH_3$ wherein
- when $X_{24}$ is nitrogen or CH, $R_{25}$ is null.
- n is 1 or 2;
- $R_{24}$, $R_{25}$, $R_{26}$, and $R_{27}$ are independently selected from the groups consisting of hydrogen, halogen, and alkoxy $C_1$-$C_6$ linear or branched.

In addition, the present invention further refers to compositions comprising one or more compounds of Formula (Ia) or Formula (Ib) or a salt, hydrate, solvate and pharmaceutically acceptable isomer thereof; and one or more pharmaceutically acceptable excipients.

In addition, kits in accordance with this invention may comprise such compositions and application devices, which may include ampoules, syringes and others. Alternatively, the kits according to this invention comprise more than one compound of Formula (Ia) or Formula (Ib) arranged in one or more dosage forms, including, without limitation, tablets, accompanied by administration instructions.

The present invention further refers to methods of treatment, prevention, relief, suppression and/or control of diseases related to neuropathic pain. Uses of Formula (Ia) or Formula (Ib) compound(s) to prepare a drug for the treatment of pathologies related to neuropathic pain are also taught. Finally, the present invention teaches processes for obtaining compounds of Formula (Ia) or Formula (Ib).

**DETAILED DESCRIPTION OF THE INVENTION**

The present invention presents, in a first embodiment, the compounds of Formula (Ia):

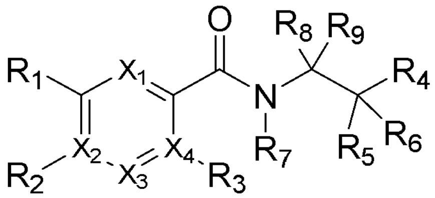

Formula (Ia)

or a pharmaceutically acceptable salt, hydrate, solvate, ester and isomer thereof, wherein:

- $X_1$, $X_2$, $X_3$, and $X_4$ are independently selected from the group consisting of carbon, CH, or nitrogen, wherein:
    - when at least one of the substituents $X_2$, $X_4$ is nitrogen or CH, the corresponding R ($R_2$, $R_3$) is null,
    - when two of the substituents $X_1$, $X_2$, $X_3$ and $X_4$ are nitrogen, the remaining substituents $X_1$, $X_2$, $X_3$ or $X_4$ must be carbon or CH;
    - $R_1$ is:

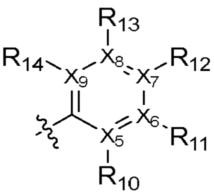

,

wherein:

- $X_5$, $X_6$, $X_7$, $X_8$, and $X_9$ are independently selected from the group consisting of carbon, CH, or nitrogen, wherein:
    - when at least one of the substituents $X_5$-$X_9$ is nitrogen or CH, the corresponding R ($R_{10}$-$R_{14}$) is null; and
    - $R_{10}$, $R_{11}$, $R_{12}$, $R_{13}$, and $R_{14}$ are independently selected from the group consisting of hydrogen, halogen, linear or branched alkyl $C_1$-$C_6$, alkoxy $C_1$-$C_6$ linear or branched, hydroxy, hydroxyalkyl $C_1$-$C_4$, haloalkoxy $C_1$-$C_6$ linear or branched, $-OCD_2CD_3$, $-OCD_3$;
    - $R_2$ and $R_3$ are independently selected from the group consisting of hydrogen, halogen, amine, $C_1$-$C_6$ linear or branched alkoxy, and $C_1$-$C_6$ linear or branched alkyl;
    - $R_4$ is:

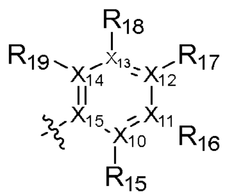

or

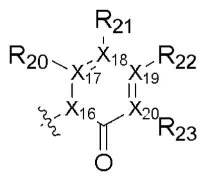

,

wherein:

- $X_{10}$, $X_{11}$, $X_{12}$, $X_{13}$, $X_{14}$, $X_{15}$, $X_{16}$, $X_{17}$, $X_{18}$, $X_{19}$ and $X_{20}$ are independently selected from the group consisting of carbon, CH or nitrogen, where:
    - when at least one of the substituents $X_{10}$, $X_{11}$, $X_{12}$, $X_{13}$, $X_{14}$, $X_{17}$, $X_{18}$, $X_{19}$ or $X_{20}$ is N or CH, the corresponding R ($R_{15}$-$R_{23}$) is null;
    - $R_{15}$, $R_{16}$, $R_{17}$, $R_{18}$, $R_{19}$, $R_{20}$, $R_{21}$, $R_{22}$, $R_{23}$ are independently selected from the group consisting of hydrogen, halogen, oxygen, $C_1$-$C_6$ alkyl linear or branched, alkoxy $C_1$-$C_6$ linear or branched,

haloalkoxy, $C_1$-$C_6$ linear or branched, hydroxyalkyl $C_1$-$C_4$ linear or branched, $-OCD_2CD_3$ or $-OCD_3$;

- $R_5$ and $R_6$ are independently selected from the group consisting of hydrogen, hydroxy, carbonyl, amine, alkyl $C_1$-$C_6$ linear or branched, wherein when $R_5$ or $R_6$ are carbonyl, the other R must be null;
- or $R_4$, $R_5$, and $R_6$ together form one of the following bicycles:

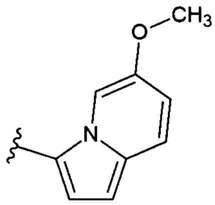
,
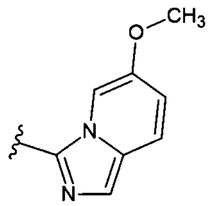
or
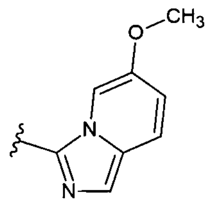
;

and

- $R_7$, $R_8$, and $R_9$ are independently selected from the group consisting of hydrogen and linear or branched $C_1$-$C_6$ alkyl.

The present invention presents, in a second embodiment, the compounds of Formula (Ib):

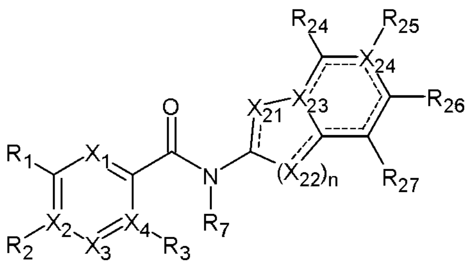
Formula (Ib)

or a pharmaceutically acceptable salt, hydrate, solvate, ester and isomer thereof, wherein:

- $X_1$, $X_2$, $X_3$, and $X_4$ are independently selected from the group consisting of carbon, CH, or nitrogen, wherein
- when at least one of the substituents $X_2$, $X_4$ is nitrogen or CH, the corresponding R ($R_2$, $R_3$) is null,
- when two of the substituents $X_1$, $X_2$, $X_3$ and $X_4$ are nitrogen, the remaining substituents $X_1$, $X_2$, $X_3$ or $X_4$ must be carbon or CH;
- $R_1$ is:

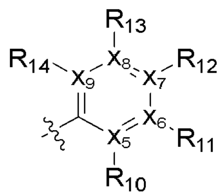
,

wherein

- $X_5$, $X_6$, $X_7$, $X_8$, and $X_9$ are independently selected from the group consisting of carbon, CH, or nitrogen, wherein
- when at least one of the substituents $X_5$-$X_9$ is nitrogen or CH, the corresponding R ($R_{10}$-$R_{14}$ ) is null,
- $R_{10}$, $R_{11}$, $R_{12}$, $R_{13}$, and $R_{14}$ are independently selected from the group consisting of hydrogen, halogen, linear or branched $C_1$-$C_6$ alkyl, alkoxy $C_1$-$C_6$ linear or branched, hydroxyalkyl $C_1$-$C_4$, haloalkoxy $C_1$-$C_6$ linear or branched, $-OCD_2CD_3$ or $-OCD_3$;
- $R_2$ and $R_3$ are independently selected from the group consisting of hydrogen, halogen, amine, $C_1$-$C_6$ alkoxy, and $C_1$-$C_6$ alkyl linear or branched;
- $R_7$ is selected from a group consisting of hydrogen and alkyl $C_1$-$C_6$ linear or branched;

- $X_{21}$, $X_{22}$, $X_{23}$, and $X_{24}$ are independently selected from the group consisting of carbon, CH, $CH_2$, nitrogen, oxygen, or sulfur, where
- when $X_{24}$ is nitrogen or CH, $R_{25}$ is null;
- n is 1 or 2;
- $R_{24}$, $R_{25}$, $R_{26}$, and $R_{27}$ are independently selected from the groups consisting of hydrogen, halogen, and alkoxy $C_1$-$C_6$ linear or branched.

As described herein, the compounds of the present invention comprise multiple variable groups (R, $X_1$, etc.). As a person skilled in the art will recognize, the group combinations contemplated by this invention are those combinations that result in the formation of stable or chemically viable compounds. The term "stable" in this context refers to compounds that are not substantially altered when subjected to conditions that allow their production, detection and preferably their recovery, purification and use for one or more of the purposes disclosed herein. In some embodiments, a stable compound or chemically viable compound is one that is not substantially altered when kept at a temperature of 40 °C or less, in the absence of moisture or other chemically reactive conditions, for at least one week.

Additionally, the chemical structures drawn here are intended to be understood as they would be understood by a person skilled in the art. For example, with respect to the formulas III, IV, X, a person skilled in the art would understand that $X_2$ and $X_3$ are connected by a simple bond, even though the bonds between these groups can be hidden by the labels of the atoms in the chemical structures.

In an implementation of the first embodiment, $X_1$, $X_2$, $X_3$, and $X_4$ are independently selected from the group consisting of carbon, CH, or nitrogen, where when at least one of the substituents $X_2$, $X_4$ is nitrogen or CH, the corresponding R ($R_2$, $R_3$) is null; and when two of the substituents $X_1$, $X_2$, $X_3$, and $X_4$ are nitrogen, the remaining substituents $X_1$, $X_2$, $X_3$ or $X_4$ must be carbon or CH. $R_1$ is

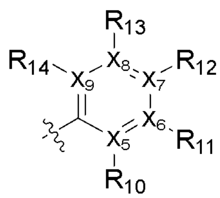,

where: $X_5$, $X_6$, $X_7$, $X_8$, and $X_9$ are independently selected from the group consisting of carbon, CH, or nitrogen, wherein when at least one of the substituents $X_{5-9}$ is nitrogen or CH, the corresponding R ($R_{10}$-. $R_{14}$) is null; $R_{10}$, $R_{11}$, $R_{12}$, $R_{13}$, and $R_{14}$ are independently selected from the group consisting of hydrogen, halogen, alkyl $C_1$-$C_6$ linear or branched, $C_1$-$C_6$ linear or branched alkoxy, hydroxy, hydroxyalkyl $C_1$-$C_4$, haloalkoxy $C_1$-$C_6$ linear or branched, $-OCD_2CD_3$ or $-OCD_3$;

In an embodiment, $R_2$ and $R_3$ are independently selected from the group consisting of hydrogen, halogen, amine, $C_1$-$C_6$ alkoxy linear or branched, and $C_1$-$C_6$ linear or branched alkyl;

In an embodiment, $R_4$ is:

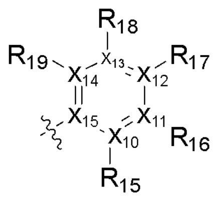

or

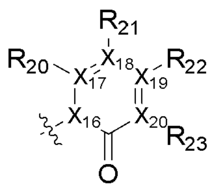,

wherein: $X_{10}$, $X_{11}$, $X_{12}$, $X_{13}$, $X_{14}$, $X_{15}$, $X_{16}$, $X_{17}$, $X_{18}$, $X_{19}$ and $X_{20}$ are independently selected from the group consisting of carbon, CH or nitrogen, where: when at least one of the substituents $X_{10}$, $X_{11}$, $X_{12}$, $X_{13}$, $X_{14}$, $X_{17}$, $X_{18}$, $X_{19}$ or $X_{20}$ is nitrogen or CH, the corresponding R ($R_{15}$-$R_{23}$) is null; and $R_{15}$, $R_{16}$, $R_{17}$, $R_{18}$, $R_{19}$, $R_{20}$, $R_{21}$, $R_{22}$, $R_{23}$ are independently selected

from the group consisting of hydrogen, halogen, oxygen, C $_1$-$C_6$ linear or branched alkyl, alkoxy $C_1$-$C_6$ linear or branched, hydroxyalkyl $C_{1-}C_4$ linear or branched, -$OCD_2CD_3$ or -$OCD_3$; $R_5$ and $R_6$ are independently selected from the group consisting of hydrogen, hydroxy, carbonyl, amine,alkyl linear or branched $C_1$-$C_6$, where: when $R_5$ or $R_6$ are carbonyl, the other R must be null; and $R_7$, $R_8$, and $R_9$ are independently selected from the group consisting of hydrogen and $C_1$-$C_6$ linear or branched alkyl.

In an embodiment, $X_1$, $X_2$, $X_3$, and $X_4$ are independently selected from the group consisting of carbon and nitrogen. In an embodiment, $R_1$ is:

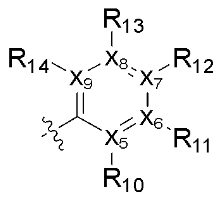

where $X_5$, $X_6$, $X_7$, $X_8$, and $X_9$ are independently selected from the group consisting of carbon and nitrogen. In a preferred embodiment, $X_5$ and $X_8$ are carbon or nitrogen, and $X_6$, $X_7$, and $X_9$ are carbon.

In an embodiment, $R_{10}$, $R_{11}$, $R_{12}$, $R_{13}$ and $R_{14}$ are independently selected from hydrogen, fluorine, chlorine, bromine, methyl, ethyl, propyl, isopropyl, butyl, sec-butyl, tert-butyl, methoxyl, ethoxyl, propoxyl, isopropoxyl, hydroxyl, trifluoromethoxy, difluoromethoxy, fluorometoxy, chloromethoxy, dichloromethoxy, trichloromethoxy, 2-hydroxyethyl, 1-hydroxyethyl, hydroxymethyl, 1-hydroxypropyl, 2-hydroxypropyl, 1-hydroxybutyl, 2-hydroxybutyl, -$OCD_2CD_3$ or -$OCD_3$. In a preferred embodiment, $R_{10}$ is methyl, fluorine, or hydrogen; $R_{11}$ and $R_{13}$ are hydrogen; $R_{12}$ is 1-hydroxyethyl, 1-hydroxypropyl, methoxyl, ethoxyl, isopropoxy, difluoromethoxy; $R_{14}$ is methyl or hydrogen.

$R_2$ and $R_3$ are independently selected hydrogen, fluorine, chlorine, bromine, amine, methyl, ethyl, propyl, isopropyl, butyl, *sec*-butyl, *tert*-butyl, methoxyl, ethoxyl, propoxyl, isopropoxy. In a preferred embodiment $R_2$ is hydrogen or null when $X_2$ is nitrogen; and $R_3$ is hydrogen or null when $X_4$ is nitrogen.

In an embodiment, $R_4$ is:

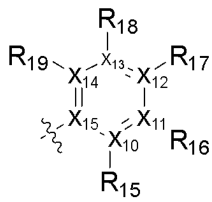

,

or

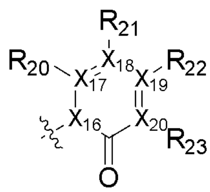

.

In an embodiment, $X_{10}$, $X_{11}$, $X_{12}$, $X_{13}$, $X_{14}$, $X_{15}$, $X_{16}$, $X_{17}$, $X_{18}$, $X_{19}$, and $X_{20}$ are independently selected from the group consisting of carbon, CH, or nitrogen. In a preferred embodiment, $X_{10}$, $X_{11}$, $X_{12}$, $X_{13}$, $X_{14}$, $X_{15}$, $X_{17}$, $X_{18}$, $X_{19}$, and $X_{20}$ are carbon or nitrogen. In a preferred embodiment, $X_{16}$ is nitrogen.

In an embodiment, $R_{13}$, $R_{16}$, $R_{17}$, $R_{18}$, $R_{19}$, $R_{20}$, $R_{21}$, $R_{22}$, $R_{23}$ are independently selected from the group consisting of hydrogen, fluorine, chlorine, bromine, oxygen, methyl, ethyl, propyl, isopropyl, butyl, *sec*-butyl, *tert*-butyl, methoxyl, ethoxyl, propoxyl, isopropoxyl, difluoromethoxy, trifluoromethoxy, 2-hydroxyethyl, 1-hydroxyethyl, hydroxymethyl, 1-hydroxypropyl, 2- hydroxypropyl, 1-hydroxybutyl, 2-hydroxybutyl, -$OCD_2CD_3$, or -$OCD_3$. In a preferred embodiment, $R_{15}$ is methyl, fluorine, hydrogen, or null when $X_{10}$ is nitrogen; $R_{16}$ is hydrogen, methoxyl, -$OCD_3$ or null when $X_{11}$ is nitrogen; $R_{17}$ is hydrogen, methoxyl or null when $X_{12}$ is nitrogen; $R_{13}$ is hydrogen, 1-hydroxyethyl, methoxyl, -$OCD_3$, or null when $X_{13}$ is nitrogen, $R_{19}$ is hydrogen, fluorine or null when X14 is nitrogen; $R_{20}$, $R_{22}$, and $R_{23}$ is hydrogen; $R_{21}$ is hydrogen or methoxyl.

In an embodiment, $R_5$ and $R_6$ are independently selected from the group consisting of hydrogen, hydroxy, carbonyl, amine, methyl, ethyl, propyl, isopropyl, isopropyl, butyl, *sec*-butyl, *tert*-butyl. In a preferred embodiment, $R_5$ and

$R_6$ are hydrogen, carbonyl, methyl, amine, and hydroxyl, whereas, when $R_5$ or $R_6$ are carbonyl, the other R will be null.

In an embodiment, $R_7$, $R_8$, and $R_9$ are independently selected from the group consisting of hydrogen, methyl, ethyl, propyl, isopropyl, butyl, *sec*-butyl, and *tert*-butyl. In a preferred embodiment, $R_7$ is hydrogen, $R_8$, and $R_9$ are methyl or hydrogen.

In an implementation of the second embodiment, $X_1$, $X_2$, $X_3$, and $X_4$ are independently selected from the group consisting of carbon, CH, or nitrogen, where when at least one of the substituents $X_2$, $X_4$ is N or CH, the corresponding R ($R_2$, $R_3$) is null; and when two of the substituents $X_1$, $X_2$, $X_3$ and $X_4$ are N, the remaining substituents $X_1$, $X_2$, $X_3$ or $X_4$ must be carbon or CH. $R_1$ is:

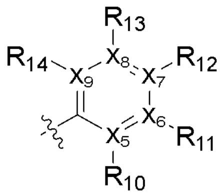

where $X_5$, $X_6$, $X_7$, $X_8$, and $X_9$ are independently selected from the group consisting of carbon and nitrogen. In a preferred embodiment, $X_5$ and $X_8$ are carbon or nitrogen, and $X_6$, $X_7$, and $X_9$ are carbon.

In an embodiment, $R_{10}$, $R_{11}$, $R_{12}$, $R_{13}$ and $R_{14}$ are independently selected from hydrogen, fluorine, chlorine, bromine, methyl, ethyl, propyl, isopropyl, butyl, sec-butyl, tert-butyl, methoxyl, ethoxyl, propoxyl, isopropoxyl, hydroxyl, trifluoromethoxy, difluoromethoxy, fluoromethoxy, chloromethoxy, dichloromethoxy, trichloromethoxy, 2-hydroxyethyl, 1-hydroxyethyl, hydroxymethyl, 1-hydroxypropyl, 2-hydroxypropyl, 1-hydroxybutyl, 2-hydroxybutyl, $-OCD_2CD_3$ or - $OCD_3$. In a preferred embodiment, $R_{10}$ is methyl, fluorine, or hydrogen; $R_{11}$ and $R_{13}$ are hydrogen; $R_{12}$ is 1-hydroxyethyl, 1-hydroxypropyl, methoxyl, ethoxyl, isopropoxy, difluoromethoxy; $R_{14}$ is methyl or hydrogen.

In an embodiment, $R_2$ and $R_3$ are independently selected hydrogen, fluorine, chlorine, bromine, amine, methyl, ethyl, propyl, isopropyl, butyl, *sec*-butyl, *tert*-butyl, methoxyl, ethoxyl, propoxyl, isopropoxy. In a preferred embodiment $R_2$ is hydrogen or null when $X_2$ is nitrogen; and $R_3$ is hydrogen or null when $X_4$ is nitrogen.

In an embodiment, R4 is selected from the group consisting of hydrogen, methyl, ethyl, propyl, isopropyl, butyl, *sec*-butyl, *tert*-butyl. In a preferred embodiment, hydrogen or methyl.

In an embodiment, $X_{21}$, $X_{22}$, $X_{23}$, and $X_{24}$ are independently selected from the group consisting of carbon, CH, $CH_2$, nitrogen, oxygen, or sulfur. In a preferred embodiment, $X_{21}$ is CH, nitrogen, and oxygen; $X_{22}$ is CH, $CH_2$, nitrogen, oxygen, and sulfur; $X_{23}$ is carbon or nitrogen; $X_{24}$ is CH or nitrogen.

In an embodiment, n is 1 or 2.

In an embodiment, $R_{24}$, $R_{25}$, $R_{26}$, $R_{27}$ are independently selected from the group consisting of hydrogen, fluorine, chlorine, bromine, methoxyl, ethoxyl, propoxyl, isopropoxyl or null when the respective $X_{21}$, $X_{22}$, $X_{23}$ or $X_{24}$ is CH, $CH_2$, nitrogen, oxygen or sulfur. In a preferred embodiment, $R_{24}$ is hydrogen, fluorine, methoxyl; $R_{25}$ is hydrogen, methoxyl, null when $X_{24}$ is CH or nitrogen; $R_{26}$ is hydrogen; $R_{27}$ is hydrogen, methoxyl, fluorine.

In a preferred implementation of the first and second embodiments, the Formula (Ia) and Formula (Ib) compound(s) are selected from the group(s) consisting of:

*N*-(3,5-dimethoxyphenethyl)-6-(4-isopropoxyphenyl)pyrazine-2-carboxamide (Compound 1);
6-(4-(difluoromethoxy)phenyl)-*N*-(3,5-dimethoxyphenethyl)pyrazine-2-carboxamide (Compound 2);
*N*-(3,5-dimethoxyphenethyl)-6-(4-ethoxy-2-fluorophenyl)pyrazine-2-carboxamide (Compound 3);
*N*-(3,5-dimethoxyphenethyl)-6-(4-ethoxy-2-methylphenyl)pyrazine-2-carboxamide (Compound 4);
6-(4-ethoxyphenyl)-*N*-(3-methoxyphenethyl)pyrazine-2-carboxamide (Compound 5);
6-(4-ethoxyphenyl)-*N*-(2-fluorophenethyl)pyrazine-2-carboxamide (Compound 6);
6-(4-ethoxyphenyl)-*N*-(2-(2-oxopiridine-1(2*H*)-yl)ethyl)pyrazine-2-carboxamide (Compound 7);
6-(4-ethoxyphenyl)-*N*-(2-(5-methoxy-2-oxopiridine-1(2*H*)-yl)ethyl)pyrazine-2-carboxamide (Compound 8);
6-(4-ethoxyphenyl)-*N*-(2-(2-fluorophenyl)-2-oxoethyl)pyrazine-2-carboxamide (Compound 9);
*N*-(3,5-dimethoxyphenethyl)-6-(4-ethoxyphenyl)pyrazine-2-carboxamide (Compound 10);
6-(4-ethoxyphenyl)-*N*-(2-(2-fluoro-5-methoxyphenyl)-2-oxoethyl)pyrazine-2-carboxamide (Compound 11);
6-(4-ethoxyphenyl)-*N*-(3-(1-hydroxyethyl)-5-methoxyphenethyl)pyrazine-2-carboxamide (Compound 12);
6-(4-ethoxyphenyl)-*N*-(3-(1-hydroxyethyl)phenethyl)pyrazine-2-carboxamide (Compound 13);
6-(4-ethoxyphenyl)-*N*-(2-(2-fluoro-5-(1-hydroxyethyl)pyridine-3-yl)ethyl)pyrazine-2-carboxamide (Compound 14);
6-(4-ethoxyphenyl)-*N*-(2-fluoro-5-(1-hydroxyethyl)phenethyl)pyrazine-2-carboxamide (Compound 15);
6-(4-ethoxyphenyl)-*N*-(2-(2-fluoro-5-methoxypyridin-3-yl)ethyl)pyrazine-2-carboxamide (Compound 16);
6-(4-ethoxyphenyl)-*N*-(2-fluoro-5-methoxyphenethyl)pyrazine-2-carboxamide (Compound 17);
6-(4-ethoxyphenyl)-*N*-(2-(5-methoxy-2-methylpyridine-3-yl)ethyl)pyrazine-2-carboxamide (Compound 18);

6-(4-ethoxyphenyl)-*N*-(5-methoxy-2-methylphenethyl)pyrazine-2-carboxamide (Compound 19);
(*S*)-*N*-(1-(3,5-dimethoxyphenyl)propan-2-yl)-6-(4-ethoxyphenyl)pyrazine-2-carboxamide (Compound 20);
(*R*)-N-(1-(3,5-dimethoxyphenyl)propan-2-yl)-6-(4-ethoxyphenyl)pyrazine-2-carboxamide (Compound 21);
(*R*)-*N*-(2-(3,5-dimethoxyphenyl)propyl)-6-(4-ethoxyphenyl)pyrazine-2-carboxamide (Compound 22);
(*S*)-*N*-(2-(3,5-dimethoxyphenyl)propyl)-6-(4-ethoxyphenyl)pyrazine-2-carboxamide (Compound 23);
(*R*)-*N*-(2-(3,5-dimethoxyphenyl)-2-hydroxyethyl)-6-(4-ethoxyphenyl)pyrazine-2-carboxamide (Compound 24);
(*S*)-*N*-(2-(3,5-dimethoxyphenyl)-2-hydroxyethyl)-6-(4-ethoxyphenyl)pyrazine-2-carboxamide (Compound 25);
(*R*)-*N*-(2-amino-2-(3,5-dimethoxyphenyl)ethyl)-6-(4-ethoxyphenyl)pyrazine-2-carboxamide (Compound 26);
(*S*)-*N*-(2-amino-2-(3,5-dimethoxyphenyl)ethyl)-6-(4-ethoxyphenyl)pyrazine-2-carboxamide (Compound 27);
*N*-(3,5-dimethoxyphenethyl)-6-(6-isopropoxypyridine-3-yl)pyrazine-2-carboxamide (Compound 28);
6-(6-(difluoromethoxy)pyridine-3-yl)-*N*-(3,5-dimethoxyphenethyl)pyrazine-2-carboxamide (Compound 29);
*N*-(3,5-dimethoxyphenethyl)-6-(6-ethoxy-4-methylpyridine-3-yl)pyrazine-2-carboxamide (Compound 30);
*N*-(3,5-dimethoxyphenethyl)-6-(6-ethoxy-2-methylpyridine-3-yl)pyrazine-2-carboxamide (Compound 31);
6-(6-ethoxypyridine-3-yl)-*N*-(2-(2-oxopiridine-1(2H)-yl)ethyl)pyrazine-2-carboxamide (Compound 32);
6-(6-ethoxypyridine-3-yl)-*N*-(2-(5-methoxy-2-oxopiridine-1(2*H*)-yl)ethyl)pyrazine-2-carboxamide (Compound 33);
6-(6-ethoxypyridine-3-yl)-*N*-(2-(2-fluoro-5-methoxypyridine-3-yl)ethyl)pyrazine-2-carboxamide (Compound 34);
6-(6-ethoxypyridine-3-yl)-*N*-(2-fluoro-5-methoxyphenethyl)pyrazine-2-carboxamide (Compound 35);
6-(6-ethoxypyridine-3-yl)-*N*-(2-(5-methoxy-2-methylpyridine-3-yl)ethyl)pyrazine-2-carboxamide (Compound 36);
6-(6-ethoxypyridine-3-yl)-*N*-(5-methoxy-2-methylphenethyl)pyrazine-2-carboxamide (Compound 37);
6-(6-ethoxypyridine-3-yl)-*N*-(2-(2-fluorophenyl)-2-oxoethyl)pyrazine-2-carboxamide (Compound 38);
6-(6-ethoxypyridine-3-yl)-*N*-(2-(2-fluorophenyl)-2-oxoethyl)pyrazine-2-carboxamide (Compound 39);
6-(6-ethoxypyridine-3-yl)-*N*-(3-(1-hydroxyethyl)-5-methoxyphenethyl)pyrazine-2-carboxamide (Compound 40);
6-(6-ethoxypyridine-3-yl)-*N*-(3-(1-hydroxyethyl)phenethyl)pyrazine-2-carboxamide (Compound 41);
6-(6-ethoxypyridine-3-yl)-*N*-(2-(2-fluoro-5-(1-hydroxyethyl)pyridine-3-yl)ethyl)pyrazine-2-carboxamide (Compound 42);
6-(6-ethoxypyridine-3-yl)-*N*-(2-fluoro-5-(1-hydroxyethyl)phenethyl)pyrazine-2-carboxamide (Compound 43);
N-(3,5-dimethoxyphenethyl)-6-(6-ethoxypyridine-3-yl)pyrazine-2-carboxamide (Compound 44);
6-(6-ethoxypyridine-3-yl)-*N*-(3-methoxyphenethyl)pyrazine-2-carboxamide (Compound 45);
6-(6-ethoxypyridine-3-yl)-*N*-(2-fluorophenethyl)pyrazine-2-carboxamide (Compound 46);
6-(6-(difluoromethoxy)pyridine-3-yl)-*N*-(2-fluorophenethyl)pyrazine-2-carboxamide (Compound 47);
*N*-(2-fluorophenethyl)-6-(6-methoxypyridine-3-yl)pyrazine-2-carboxamide (Compound 48);
*N*-(3,5-dimethoxyphenethyl)-6-(4-methoxyphenyl)pyrazine-2-carboxamide (Compound 49);
6-(4-(difluoromethoxy)phenyl)-*N*-(2-(2-fluoro-5-methoxypyridine-3-yl)ethyl)pyrazine-2-carboxamide (Compound 50);
6-(6-(difluoromethoxy)pyridine-3-yl)-*N*-(2-(2-fluoro-5-methoxypyridine-3-yl)ethyl)pyrazine-2-carboxamide (Compound 51);
*N*-(2-(2-fluoro-5-methoxypyridine-3-yl)ethyl)-6-(4-methoxyphenyl)pyrazine-2-carboxamide (Compound 52);
*N*-(2-(2-fluoro-5-methoxypyridin-3-yl)ethyl)-6-(6-methoxypyridine-3-yl)pyrazine-2-carboxamide (Compound 53);
(*S*)-6-(4-(difluoromethoxy)phenyl)-N-(2-(3,5-dimethoxyphenyl)-2-hydroxyethyl)pyrazine-2-carboxamide (Compound 54);
(*S*)-N-(2-(3,5-dimethoxyphenyl)-2-hydroxyethyl)-6-(4-methoxyphenyl)pyrazine-2-carboxamide (Compound 55);
*N*-(2-(2-fluoro-5-methoxypyridinee-3-yl)ethyl)-6-(4-(2-hydroxypropane-2-yl)phenyl)pyrazine-2-carboxamide (Compound 56);
*N*-(2-(2-fluoro-5-methoxypyridine-3-yl)ethyl)-6-(6-(2-hydroxypropane-2-yl)pyridine-3-yl)pyrazine-2-carboxamide (Compound 57);
*N*-(2-(2-fluoro-5-methoxypyridine-3-yl)ethyl)-6-(6-(1-hydroxyethyl)pyridine-3-yl)pyrazine-2-carboxamide (Compound 58);
*N*-(2-(2-fluoro-5-methoxypyridine-3-yl)ethyl)-6-(4-(1-hydroxyethyl)phenyl)pyrazine-2-carboxamide (Compound 59);
6-(4-ethoxyphenyl)-*N*-(4-methoxy-1*H*-indole-2-yl)pyrazine-2-carboxamide (Compound 60);
6-(6-ethoxypyridine-3-yl)-*N*-(4-fluoro-7-methoxybenzo[d]thiazole-2-yl)pyrazine-2-carboxamide (Compound 61);
6-(6-ethoxypyridine-3-yl)-*N*-(7-fluoro-4-methoxybenzo[d]oxazole-2-yl)pyrazine-2-carboxamide (Compound 62);
6-(6-ethoxypyridine-3-yl)-*N*-(5-fluoro-8-methoxyimidazo[1,2-a]pyridine-2-yl)pyrazine-2-carboxamide (Compound 63);
6-(6-ethoxypyridine-3-yl)-*N*-(4-fluoro-7-methoxy-1*H*-benzo[d]imidazole-2-yl)pyrazine-2-carboxamide (Compound 64);
6-(6-ethoxypyridine-3-yl)-*N*-(4-fluoro-7-methoxybenzo[d]oxazole-2-yl)pyrazine-2-carboxamide (Compound 65);
6-(6-ethoxypyridine-3-yl)-*N*-(7-fluoro-4-methoxy-1-methyl-1*H*-benzo[d]imidazole-2-yl)pyrazine-2-carboxamide (Compound 66);

6-(6-ethoxypyridine-3-yl)-*N*-(4-fluoro-7-methoxyoxazole[4,5-c]pyridine-2-yl)pyrazine-2-carboxamide (Compound 67);
6-(6-ethoxypyridine-3-yl)-*N*-(8-fluoro-5-methoxyimidazo[1,2-a]pyridine-2-yl)pyrazine-2-carboxamide (Compound 68);
6-(6-ethoxypyridine-3-yl)-*N*-(4-fluoro-7-methoxy-1*H*-indole-2-yl)pyrazine-2-carboxamide (Compound 69);
6-(6-ethoxypyridine-3-yl)-*N*-(4-fluoro-7-methoxy-1-methyl-1*H*-indole-2-yl)pyrazine-2-carboxamide (Compound 70);
6-(6-ethoxypyridine-3-yl)-*N*-(4-fluoro-7-methoxy-1*H*-indole-2-yl)-*N*-methylpyrazine-2-carboxamide (Compound 71);
6-(6-ethoxypyridine-3-yl)-*N*-(7-methoxy-1*H*-indole-2-yl)pyrazine-2-carboxamide (Compound 72);
6-(6-ethoxypyridine-3-yl)-*N*-(4-methoxy-1*H-i*ndole-2-yl)pyrazine-2-carboxamide (Compound 73);
6-(6-ethoxypyridine-3-yl)-*N*-(7-fluoro-4-methoxy-1*H*-indole-2-yl)pyrazine-2-carboxamide (Compound 74);
6-(6-ethoxypyridine-3-yl)-*N*-(7-fluoro-4-methoxy-1-methyl-1*H*-indole-2-yl)pyrazine-2-carboxamide (Compound 75);
6-(4-ethoxyphenyl)-*N*-(4-fluoro-7-methoxy-1*H*-indole-2-yl)pyrazine-2-carboxamide (Compound 76);
6-(4-ethoxyphenyl)-*N*-(7-fluoro-4-methoxy-1*H*-indole-2-yl)pyrazine-2-carboxamide (Compound 77);
(*S*)-6-(6-ethoxypyridine-3-yl)-*N*-(8-fluoro-5-methoxy-1,2,3,4-tetrahydronaphthalen-2-yl)pyrazine-2-carboxamide (Compound 78);
(*R*)-6-(6-ethoxypyridine-3-yl)-*N*-(8-fluoro-5-methoxy-1,2,3,4-tetrahydronaphthalen-2-yl)pyrazine-2-carboxamide (Compound 79);
6-(6-ethoxypyridine-3-yl)-*N*-((6-methoxyindolizine-3-yl)methyl)pyrazine-2-carboxamide (Compound 80);
(*S*)-6-(6-ethoxypyridine-3-yl)-*N*-(4-fluoro-7-methoxy-2,3-dihydro-1*H*-inden-2-yl)pyrazine-2-carboxamide (Compound 81);
(*R*)-6-(6-ethoxypyridine-3-yl)-*N*-(4-fluoro-7-methoxy-2,3-dihydro-1*H*-inden-2-yl)pyrazine-2-carboxamide (Compound 82);
(*S*)-6-(6-ethoxypyridine-3-yl)-*N*-(1-fluoro-4-methoxy-6,7-dihydro-5*H*-cyclopenta[c]pyridin-6-yl)pyrazine-2-carboxamide (Compound 83);
(*R*)-6-(6-ethoxypyridine-3-yl)-*N*-(1-fluoro-4-methoxy-6,7-dihydro-5*H*-cyclopenta[c]pyridine-6-yl)pyrazine-2-carboxamide (Compound 84);
6-(6-ethoxypyridine-3-yl)-*N*-((6-methoxyimidazo[1,5-a]pyridine-3-yl)methyl)pyrazine-2-carboxamide (Compound 85);
6-(6-ethoxypyridine-3-yl)-*N*-((6-methoxyimidazo[1,2-a]pyridine-3-yl)methyl)pyrazine-2-carboxamide (Compound 86);
(*S*)-6-(6-(ethoxy-$d_5$)pyridine-3-yl)-*N*-(2-(2-fluorophenyl)-2-hydroxyethyl)pyrazine-2-carboxamide (Compound 87);
(*R*)-6-(6-(ethoxy-$d_5$)pyridine-3-yl)-*N*-(2-(2-fluorophenyl)-2-hydroxyethyl)pyrazine-2-carboxamide (Compound 88);
*N*-(2-fluorophenethyl)-6-(6-(methoxy-d3)pyridin-3-yl)pyrazine-2-carboxamide (Compound 89);
*N*-(2-(2-fluoro-5-methoxypyridine-3-yl)ethyl)-6-(6-(methoxy-d3)pyridine-3-yl)pyrazine-2-carboxamide (Compound 90);
(*S*)-*N*-(2-(3,5-bis(methoxy-d3)phenyl)-2-hydroxyethyl)-6-(6-(difluoromethoxy)pyridine-3-yl)pyrazine-2-carboxamide (Compound 91);
(*S*)-N-(2-(3,5-bis(methoxy-d3)phenyl)-2-hydroxyethyl)-6-(6-(methoxy-d3)pyridine-3-yl)pyrazine-2-carboxamide (Compound 92);
(*S*)-*N*-(2-(3,5-dimethoxyphenyl)-2-hydroxyethyl)-6-(4-(methoxy-d3)phenyl)pyrazine-2-carboxamide (Compound 93);
*N*-(2-(2-fluoro-5-methoxypyridine-3-yl)ethyl)-6-(4-(methoxy-d3)phenyl)pyrazine-2-carboxamide (Compound 94);
*N*-(3,5-dimethoxyphenethyl)-6-(4-(methoxy-d3)phenyl)pyrazine-2-carboxamide (Compound 95);
(*S*)-*N*-(2-(3,5-bis(methoxy-d3)phenyl)-2-hydroxyethyl)-6-(4-difluoromethoxy)phenyl)pyrazine-2-carboxamide (Compound 96);
(*S*)-6-(4-(ethoxy-ds)phenyl)-*N*-(2-(2-fluoro-5-(methoxy-d3)pyridine-3-yl)-2-hydroxyethyl)pyrazine-2-carboxamide (Compound 97);
(*R*)-6-(4-(ethoxy-$d_5$)phenyl)-*N*-(2-(2-fluoro-5-(methoxy-d3)pyridine-3-yl)-2-hydroxyethyl)pyrazine-2-carboxamide (Compound 98);
(*S*)-*N*-(2-(3,5-bis(methoxy-d3)phenyl)-2-hydroxyethyl)-6-(4-(methoxy-d3)phenyl)pyrazine-2-carboxamide (Compound 99);
6-(4-ethoxyphenyl)-*N*-(2-(2-fluoro-5-methoxypyridine-3-yl)ethyl)pyridazine-4-carboxamide (Compound 100);
5-(4-ethoxyphenyl)-*N*-(2-(2-fluoro-5-methoxypyridine-3-yl)ethyl)pyridazine-3-carboxamide (Compound 101);
5-(4-ethoxyphenyl)-*N*-(2-(2-fluoro-5-methoxypyridine-3-yl)ethyl)nicotinamide (Compound 102);
6-(4-ethoxyphenyl)-*N*-(2-(2-fluoro-5-methoxypyridine-3-yl)ethyl)picolinamide (Compound 103);
2-(4-ethoxyphenyl)-*N*-(2-(2-fluoro-5-methoxypyridine-3-yl)ethyl)isonicotinamide (Compound 104);
4-(4-ethoxyphenyl)-*N*-(2-(2-fluoro-5-methoxypyridine-3-yl)ethyl)picolinamide (Compound 105);

2-(4-ethoxyphenyl)-*N*-(2-(2-fluoro-5-methoxypyridine-3-yl)ethyl)pyrimidine-4-carboxamide (Compound 106);
4-(4-ethoxyphenyl)-*N*-(2-(2-fluoro-5-methoxypyridine-3-yl)ethyl)pyrimidine-2-carboxamide (Compound 107);
6'-ethoxy-*N*-(2-(2-fluoro-5-methoxypyridine-3-yl)ethyl)-[2,3'-bipyridine]-6-carboxamide (Compound 108);
6'-ethoxy-*N*-(2-(2-fluoro-5-methoxypyridine-3-yl)ethyl)-[2,3'-bipyridine]-4-carboxamide (Compound 109);
2-(6-ethoxypyridine-3-yl)-*N*-(2-(2-fluoro-5-methoxypyridine-3-yl)ethyl)pyrimidine-4-carboxamide (Compound 110);
6-(6-ethoxypyridine-3-yl)-*N*-(2-(2-fluoro-5-methoxypyridine-3-yl)ethyl)pyridazine-4-carboxamide (Compound 111);
5-(6-ethoxypyridine-3-yl)-*N*-(2-(2-fluoro-5-methoxypyridine-3-yl)ethyl)pyridazine-3-carboxamide (Compound 112);
6'-ethoxy-*N*-(2-(2-fluoro-5-methoxypyridine-3-yl)ethyl)-[3,3'-bipyridine]-5-carboxamide (Compound 113);
6-ethoxy-*N*-(2-(2-fluoro-5-methoxypyridine-3-yl)ethyl)-[3,4'-bipyridine]-2'-carboxamide (Compound 114), and
4-(6-ethoxypyridine-3-yl)-*N*-(2-(2-fluoro-5-methoxypyridine-3-yl)ethyl)pyrimidine-2-carboxamide (Compound 115).

In an implementation of the first embodiment and second embodiment, the compounds of Formula (Ia) and Formula (Ib) are voltage-gated sodium channel blockers Nav 1.7 and/or Nav 1.8. In a preferred implementation of the first embodiment, the Formula (Ia) or Formula (Ib) compounds are dual blockers of voltage-gated sodium channels Nav 1.7 and Nav 1.8.

In an implementation of the first embodiment or second embodiment, Formula (Ia) or Formula (Ib) compounds may have a basic nature and, consequently, pharmaceutically acceptable salts may be obtained by the addition of organic or inorganic acids. Non-limiting examples of organic acids that can be used are fumaric, maleic, benzoic, lactic acids, among others. Among the inorganic acids, we can mention hydrochloric acid, phosphoric acid, hydrobromic acid, sulfuric acid, nitric acid, among others.

In an implementation of the first embodiment or second embodiment, the compounds of Formula (Ia) or Formula (Ib) can be obtained in the form of crystals, which can be optionally presented as pharmaceutically acceptable solvates, in which the solvent is incorporated in stoichiometric proportions or not into the crystal lattice. In further embodiments, the crystallization solvent is water, resulting in pharmaceutically acceptable hydrates.

Finally, in an implementation of the first embodiment or second embodiment, Formula (Ia) or Formula (Ib) compounds can have more than one isomer, including without limitation, spatial isomerism, such as geometric and optical isomerism.

**DEFINITIONS:**

In order to clarify or elucidate the terms used in this invention, the following definitions are presented, whereby the scope is not limited thereto.

The term "halogen" refers to the elements of the 7th family of the periodic table, which are: fluorine (F), chlorine (Cl), bromine (Br), iodine (I), astatine (At) and tennessine (Ts).

The term "linear or branched $C_1$-$C_6$ alkyl refers to saturated hydrocarbon groups of linear or branched chain, such as methyl, ethyl, propyl, isopropyl, butyl, isobutyl, sec-butyl, tert-butyl, but not limited to the same.

The term "linear or branched $C_1$-$C_6$ alkoxy" refers to alkyl groups attached to an oxygen radical, such as methoxyl, ethoxyl, propoxyl, isopropoxyl, butoxyl, butoxyl *sec*-butoxyl, *tert*-butoxyl but not limited to the same.

The term "linear or branched haloalkoxy $C_1$-$C_6$" refers to alkyl groups attached to a root oxygen and at least one halogen, such as, but not limited to, chloromethoxyl, dichloromethoxyl, trichloromethoxyl, fluoromethoxyl, difluoromethoxyl and trifluoromethoxyl.

The term "linear or branched $C_2$-$C_4$" refers to saturated, linear or branched alkyl groups replaced with at least one hydroxyl, such as 1-hydroxyethyl, 2-hydroxyethyl, 1-hydroxypropyl, 2-hydroxypropyl, 3-hydroxypropyl, isopropoxyl, but not limited to the same.

The term "$-OCD_3$" or "$-OCD_2CD_3$" refers to alkyl groups attached to an oxygen radical where D refers to a deuterium atom.

The term "$NCH_3$" refers to a nitrogen bonded to a $C_1$ alkyl group (methyl) by means of a single bond, in which the nitrogen will bond to two other atoms in the ring by means of two single bonds.

The term "carbonyl" refers to the functional group consisting of a carbon atom bonded to an oxygen atom by means of a double bond.

In a third embodiment, the present invention presents a composition comprising a therapeutically effective amount of compound(s) of Formula (Ia) or Formula (Ib) of the present invention or a salt, hydrate, solvate and pharmaceutically acceptable isomer thereof; and one or more pharmaceutically acceptable excipients.

Pharmaceutically acceptable excipients are any substance, other than the active pharmaceutical ingredient, that has been evaluated for the safety thereof and that is intentionally added to the dosage form. Such excipients are selected according to the pharmaceutical dosage form of interest, its route of administration, physicochemical compatibility with the active ingredient, and the effect on efficacy.

In addition, these excipients are widely known in the state of the art and are classified according to their function,

including without limitation diluents, binders, disintegrants or disaggregators, lubricants, suspending agents, thickeners, solvents, surfactants, sliders, anti-caking agents or flow agents, glazing agents, plasticizers, sweeteners, isotonicity agents, dyes and pigments, preservatives, antioxidants, modifying agents or pH control, complexing agents, chelating agents, flavorings, viscosity modifying agents, opacifiers, permeation promoters, among others.

In an implementation of the third embodiment pharmaceutical compositions can be administered by various routes including oral, sublingual, nasal, parenteral, injectable, submuscular, topical, transdermal, ocular, rectal, but not limited to these.

In a fourth embodiment, the present invention presents the use of compound(s) of Formula (Ia) or Formula (Ib) or a salt, hydrate, solvate and pharmaceutically acceptable isomer thereof to prepare a drug to treat pathologies related to neuropathic pain.

In a preferred implementation of the fourth embodiment, said pathologies are selected from the group consisting of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia, and postsurgical neuralgia.

In a fifth embodiment, the present invention presents a method of treatment, prevention, relief, suppression and/or control of pathologies related to neuropathic pain comprising the administration of an effective amount of Formula (Ia) or Formula (Ib) compound(s) or a salt, hydrate, solvate and pharmaceutically acceptable isomer thereof. In a preferred embodiment, the method is for the treatment, prevention, relief, suppression and/or control of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia and postsurgical neuralgia. In another preferred implementation, the administration of at least one compound of Formula (Ia) or Formula (Ib) is selected from the group comprising oral, sublingual, nasal, parenteral, injectable, submuscular, topical, transdermal, ocular, and rectal routes.

In a sixth embodiment of the invention, processes for obtaining Formula (Ia) or Formula (Ib) compounds are presented, comprising the following steps:

(a) Formation of the Formula III intermediate:

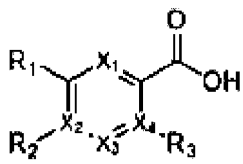

Formula III

from the basic hydrolysis reaction of a Formula IV intermediate:

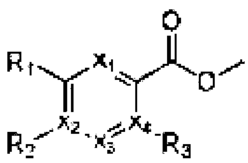

Formula IV

(b) obtaining Formula Ia and Formula Ib compounds:

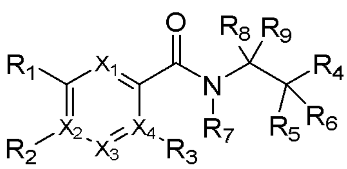

Formula (Ia)

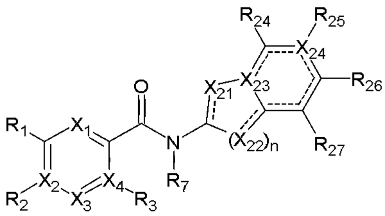

Formula (Ib)

from the amide coupling of Formula II intermediates:

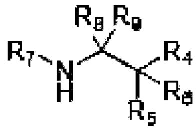

Formula II

and Formula III with or without the presence of a catalyst and an aprotic solvent;
wherein,

- $X_1$, $X_2$, $X_3$, and $X_4$ are independently selected from the group consisting of carbon, CH, or nitrogen, wherein:
    - when at least one of the substituents $X_2$, $X_4$ is nitrogen or CH, the corresponding R ($R_2$, $R_3$) is null,
    - when two of the substituents $X_1$, $X_2$, $X_3$ and $X_4$ are nitrogen, the remaining substituents $X_1$, $X_2$, $X_3$ or $X_4$ must be carbon or CH;
    - $R_1$ is:

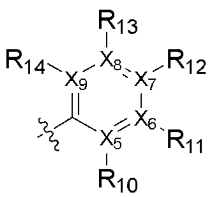

,

where:

- $X_5$, $X_6$, $X_7$, $X_8$, and $X_9$ are independently selected from the group consisting of carbon, CH, or nitrogen, wherein:
    - when at least one of the substituents $X_5$-$X_9$ is nitrogen or CH, the corresponding R ($R_{10}$-$R_{14}$) is null,
    - $R_{10}$, $R_{11}$, $R_{12}$, $R_{13}$, and $R_{14}$ are independently selected from the group consisting of hydrogen, halogen, linear or branched $C_1$-$C_6$ alkyl, alkoxy $C_1$-$C_6$ linear or branched, hydroxy, hydroxyalkyl $C_1$-$C_4$, haloalkoxy $C_1$-$C_6$ linear or branched, $-OCD_2CD_3$ or $-OCD_3$;
    - $R_2$ and $R_3$ are independently selected from the group consisting of hydrogen, halogen, amine, $C_1$-$C_6$ alkoxy, and alkyl $C_1$-$C_6$;
    - $R_4$ is:

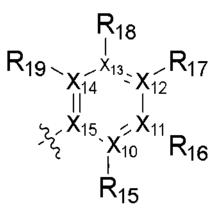

or

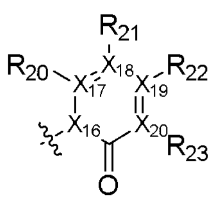

,

where

- $X_{10}$, $X_{11}$, $X_{12}$, $X_{13}$ $X_{14}$, $X_{15}$, $X_{16}$, $X_{17}$, $X_{18}$, $X_{19}$ and $X_{20}$ are independently selected from the group consisting of carbon, CH or nitrogen, wherein
- when at least one of the substituents $X_{10}$, $X_{11}$, $X_{12}$, $X_{13}$, $X_{14}$, $X_{17}$, $X_{18}$, $X_{19}$ or $X_{20}$ is nitrogen or CH, the corresponding R ($R_{15}$-$R_{23}$) is null;
- $R_{13}$, $R_{16}$, $R_{17}$, $R_{18}$, $R_{19}$, $R_{20}$, $R_{21}$, $R_{22}$, $R_{23}$ are independently selected from the group consisting of hydrogen, halogen, oxygen, alkyl $C_1$-$C_6$ linear or branched, $C_1$-$C_6$ linear or branched alkoxy, hydroxyalkyl $C_{1-}C_4$ linear or branched, $-OCD_2CD_3$ or $-OCD_3$;
- $R_5$ and $R_6$ are independently selected from the group consisting of hydrogen, hydroxy, carbonyl, amine, linear or branched $C_1$-$C_6$ alkyl, wherein
- when $R_5$ or $R_6$ are carbonyl, the other R must be null;
- or $R_4$, $R_5$, and $R_6$ together form one of the following bicycles:

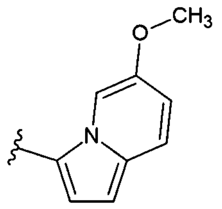 , 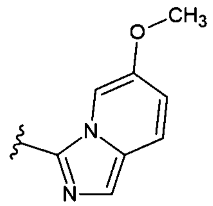 or 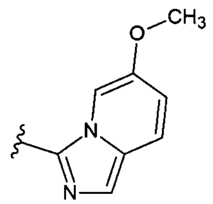 ;

  - $X_{21}$, $X_{22}$, $X_{23}$, and $X_{24}$ are independently selected from the group consisting of carbon, CH, $CH_2$, nitrogen, oxygen, sulfur, or $NCH_3$ wherein
  - when $X_{24}$ is nitrogen or CH, $R_{25}$ is null;
  - n is 1 or 2; and
  - $R_{24}$, $R_{25}$, $R_{26}$, and $R_{27}$ are independently selected from the groups consisting of hydrogen, halogen, and alkoxy $C_1$-$C_6$ linear or branched, or null when the respective $X_{21}$, $X_{22}$, $X_{23}$, or X24 is CH, $CH_2$, nitrogen, oxygen, or sulfur.
  - $R_7$, $R_8$, and $R_9$ are independently selected from the group consisting of hydrogen and linear or branched alkyl $C_1$-$C_6$.

In an implementation of the fifth embodiment, step (a) the said base is selected between sodium hydroxide or lithium hydroxide and said solvent is polar protic, selected among alcohols, water, or combinations thereof. In another embodiment, in step (a) the solvent is a mixture of methanol and water.

In an implementation of the fifth embodiment, step (b) the coupling agent is selected as a combination between TCFH and NMI and the selected solvent is dichloromethane.

The Formula (Ia) or Formula (Ib) compound(s) of this invention have been prepared from the synthetic route described in General Scheme 1. However, those skilled in the art will readily notice that additional detailing and/or modifications to the arrangement of one or more steps can be accomplished without departing from the processes taught herein. Such variations can be, without limitation, combinations of solvents and catalysts, including stereoselective ones, protective groups, among others.

In the following General Scheme 1, the formation of the Formula II, III and IV intermediates is described, in addition to the formation of the Formula (I) compound (s) .

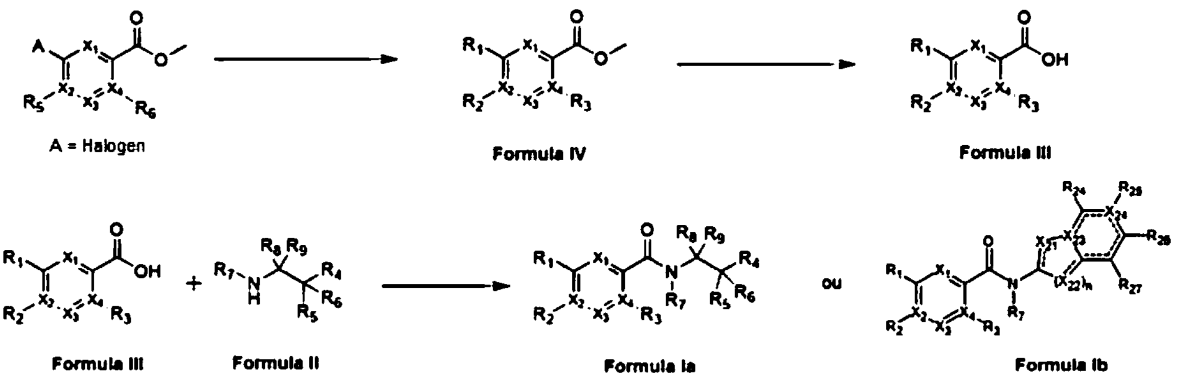

A = Halogen

Formula IV

Formula III

Formula III

Formula II

Formula Ia

ou

Formula Ib

GENERAL SCHEME 1

As illustrated in General Scheme 1, Formula I compounds can be prepared from amide coupling reactions between Formula III intermediates and Formula II intermediates in the presence of coupling agents in suitable solvents such as DCM or other aprotic solvents such as DMF. In turn, Formula III intermediates can be prepared by hydrolysis reaction from the corresponding esters (Formula IV) obtained following various methods. Formula II intermediates can be prepared from the corresponding reagents following different methods, some of which can be obtained commercially. The intermediates obtained by such methodologies are described below.

**FORMULA IV INTERMEDIATES:**

Following General Scheme 1, the intermediates of Formula IV are presented, however, such methods are not limiting.

**METHOD IV-A**

*Intermediates IV-1* to *IV-32:*

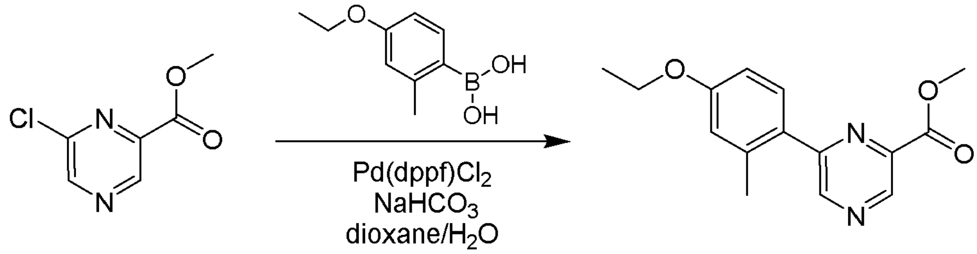


In a round-bottomed flask containing methyl 6-chloropyrazine-2-carboxylate (26.1 mmol), $NaHCO_3$ (39.1 mmol), and (4-ethoxy-2-methyl-phenyl)boronic acid (27.4 mmol) in dioxane (50.0 mL) and $H_2O$ (10.0 mL), $Pd(dppf)Cl_2$ (1.30 mmol) was added under $N_2$ atmosphere. Subsequently, the reaction mixture was heated to 60 °C and kept under agitation for 16 hours under $H_2$ atmosphere. After total consumption of the starting reagent (monitored by LC-MS and CCD), the reaction mixture was concentrated under reduced pressure for solvent removal. The residue was diluted with $H_2O$ (100 mL) and extracted with AcOEt (50.0 mL x 3). The organic phase was washed with saturated sodium chloride solution (*brine*) (50.0 mL), filtered and concentrated under reduced pressure. The residue obtained was purified by column chromatography (petroleum ether/AcOEt:10/10/1). 5.70 g (80.2% yield) of the product of interest were obtained in the form of a white solid.

**TABLE 1. FORMULA IV INTERMEDIATES OBTAINED FROM THE CORRESPONDING REAGENTS FOLLOWING THE IV-A METHOD.**

| Intermediate | Structure | Intermediate | Structure |
|---|---|---|---|
| IV-1 | 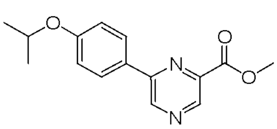 | IV-16 | 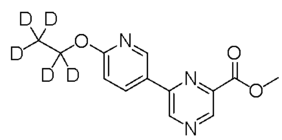 |
| IV-2 | 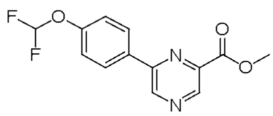 | IV-17 | 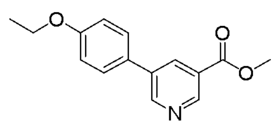 |
| IV-3 | 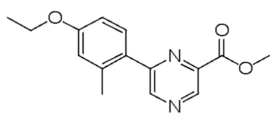 | IV-18 | 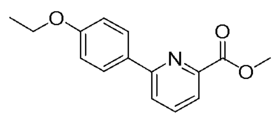 |

(continued)

| Intermediate | Structure | Intermediate | Structure |
|---|---|---|---|
| IV-4 | 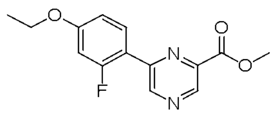 | IV-19 | 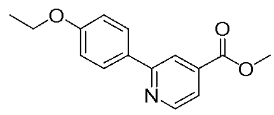 |
| IV-5 | 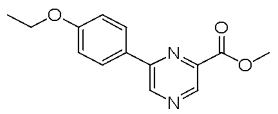 | IV-20 | 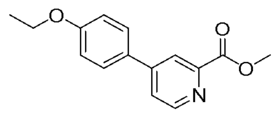 |
| IV-6 | 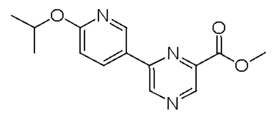 | IV-21 | 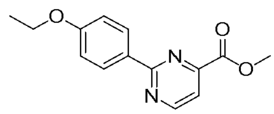 |
| IV-7 | 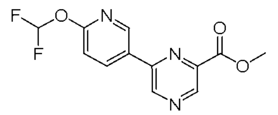 | IV-22 | 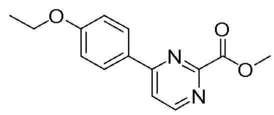 |
| IV-8 | 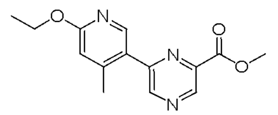 | IV-23 | 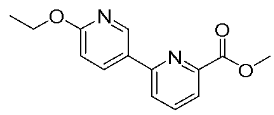 |
| IV-9 | 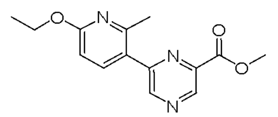 | IV-24 | 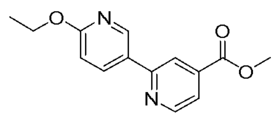 |
| IV-10 | 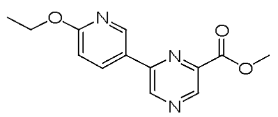 | IV-25 | 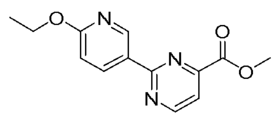 |
| IV-11 | 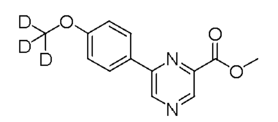 | IV-26 | 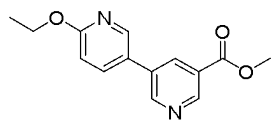 |
| IV-12 | 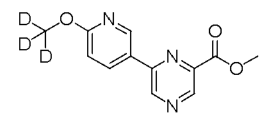 | IV-27 | 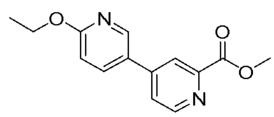 |
| IV-13 | 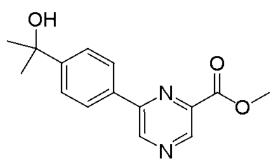 | IV-28 | 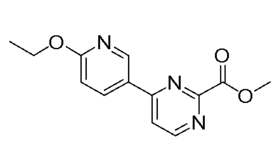 |
| IV-14 | 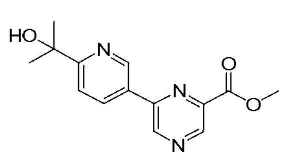 | IV-29 | 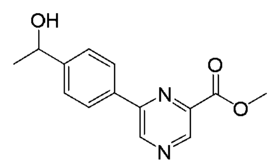 |

(continued)

| Intermediate | Structure | Intermediate | Structure |
|---|---|---|---|
| IV-15 | 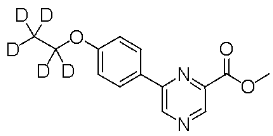 | IV-30 | 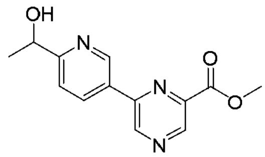 |

**METHOD IV-B**

Step IV-B-i:

*Precursors IV-31i and IV-32i:*

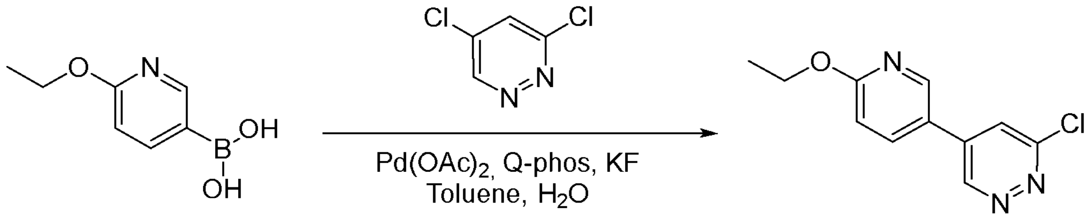


In a round-bottomed flask containing 3,5-dichloropyridazine (13.4 mmol) in toluene (20 mL) and $H_2O$ (5 mL), KF (33.6 mmol) and (6-ethoxypyridin-3-yl)boronic acid (14.8 mmol) were added at room temperature. Next, the mixture was purged with $N_2$ for 15 minutes and then Pd(OAc)$_2$ (0.671 mmol) and Q-phos (0.671 mmol) were added at room temperature under $N_2$ atmosphere. Subsequently, the mixture was again purged with $N_2$ for 5 minutes, heated to 70 °C and kept under agitation for 16 hours. After total consumption of the starting reagent (monitored by CCD and LC-MS), the mixture was cooled to room temperature and filtered with celite. The solid residue was washed with AcOEt (50 mL) and the filtrate was diluted with $H_2O$ (50 mL), followed by extraction with AcOEt (100 mL x 2). The organic phase was washed with saturated sodium chloride solution *(brine)* (100 mL), dried on anhydrous sodium sulfate and concentrated under reduced pressure. The residue obtained was purified by column chromatography (heptane/AcOEt:70/30). 1.10 g (35% yield) of the product of interest was obtained in the form of a white solid.

Step IV-B-ii:

*Intermediates IV-31 and IV-32:*

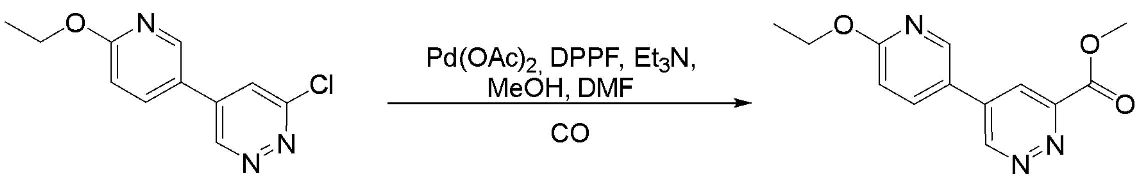


A solution of 3-chloro-5-(6-ethoxypyridine-3-yl)pyridazine (4.24 mmol) and $Et_3N$ (6.36 mmol) in MeOH (20 mL) and DMF (20 mL) was placed in a Parr reactor at room temperature. Next, the mixture was purged with $N_2$ and then DPPF (0.424 mmol) and Pd(OAc)$_2$ (0.212 mmol) were added under $N_2$ atmosphere. Subsequently, the mixture was kept under agitation in the Parr reactor under CO atmosphere (50 Psi) at 70 °C for 16 hours. After total consumption of the starting reagent (monitored by CCD and LC-MS), the mixture was cooled to room temperature and filtered with celite. The solid residue was washed with AcOEt (30 mL) and the filtrate was diluted with $H_2O$ (50 mL), followed by extraction with AcOEt (50 mL x 2). The organic phase was washed with $H_2O$ (50 mL x 2), saturated sodium chloride solution (*brine*) (100 mL), dried on anhydrous sodium sulfate, filtered and concentrated under reduced pressure. The residue obtained was purified by column chromatography (heptane/AcOEt:60/40). 700 mg (83% yield) of the product of interest were obtained in the

form of a white solid.

**TABLE 2. FORMULA IV INTERMEDIATES OBTAINED FROM THE CORRESPONDING REAGENTS FOLLOWING METHOD IV-B.**

| Intermediate | Structure |
|---|---|
| IV-31 | 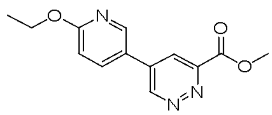 |
| IV-32 | 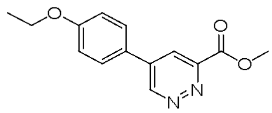 |

**FORMULA III INTERMEDIATES:**

Following General Scheme 1, the Formula III intermediates are presented, however, such methods are not limiting.

**METHOD III-A**

*Intermediates III-1 to III-30:*

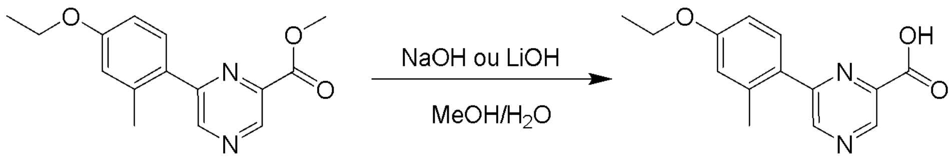


In a round-bottomed flask containing methyl 6-(4-ethoxy-2-methylphenyl)pyrazine-2-carboxylate (11.0 mmol) in MeOH (15.0 mL), NaOH (22.0 mmol) was added in H2O (5 mL). Subsequently, the reaction mixture was kept under agitation at 20 °C for 2 hours. After total consumption of the starting reagent (monitored by CCD), HCl (2 N) was added up to pH = 5. Subsequently, the mixture was filtered and washed with H2O (10.0 mL x 2). The solid residue was concentrated under reduced pressure. 2.80 g (98.4% yield) of the product of interest were obtained in the form of a white solid, which was sent to the next step without further purification.

**TABLE 3. FORMULA III INTERMEDIATES OBTAINED FROM THE CORRESPONDING REAGENTS FOLLOWING METHOD IIIA.**

| Intermed iate | Structure | Interme diate | Structure |
|---|---|---|---|
| III-1 | 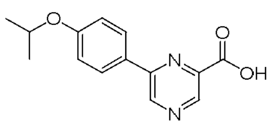 | III-16 | 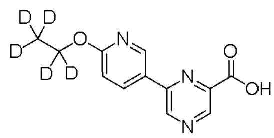 |
| III-2 | 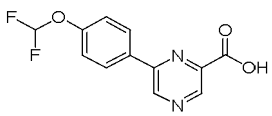 | III-17 | 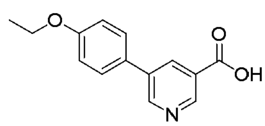 |
| III-3 | 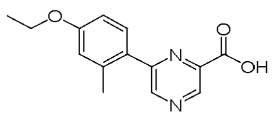 | III-18 | 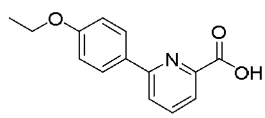 |

(continued)

| Intermed iate | Structure | Interme diate | Structure |
|---|---|---|---|
| III-4 | 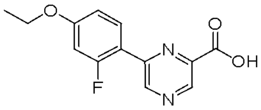 | III-19 | 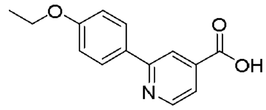 |
| III-5 | 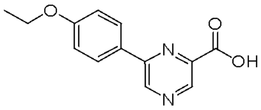 | III-20 | 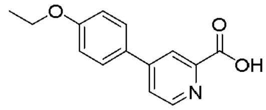 |
| III-6 | 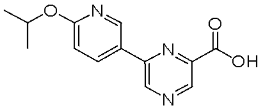 | III-21 | 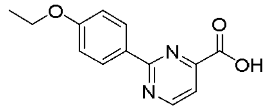 |
| III-7 | 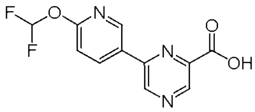 | III-22 | 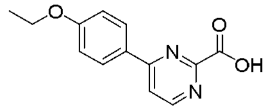 |
| III-8 | 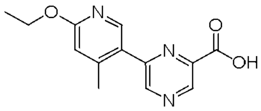 | III-23 | 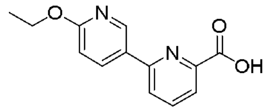 |
| III-9 | 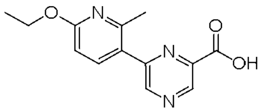 | III-24 | 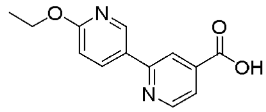 |
| III-10 | 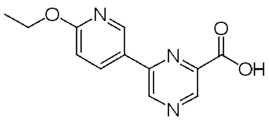 | III-25 | 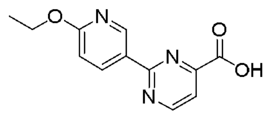 |
| III-11 | 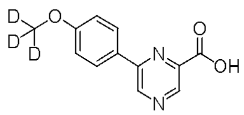 | III-26 | 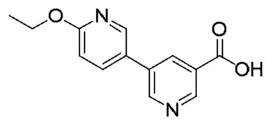 |
| III-12 | 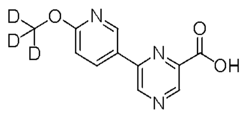 | III-27 | 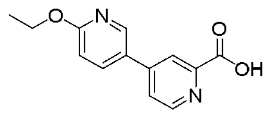 |
| III-13 | 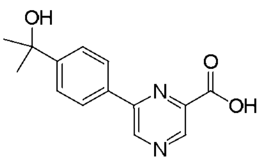 | III-28 | 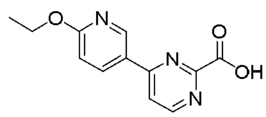 |
| III-14 | 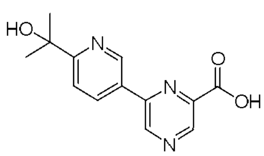 | III-29 | 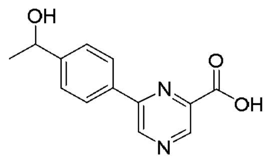 |

(continued)

| Intermed iate | Structure | Interme diate | Structure |
|---|---|---|---|
| III-15 | 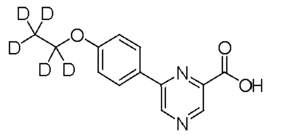 | III-30 | 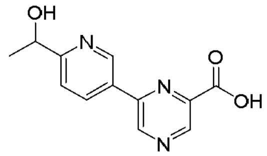 |

**METHOD IIIb**

*Intermediates III-31 to III-34:*

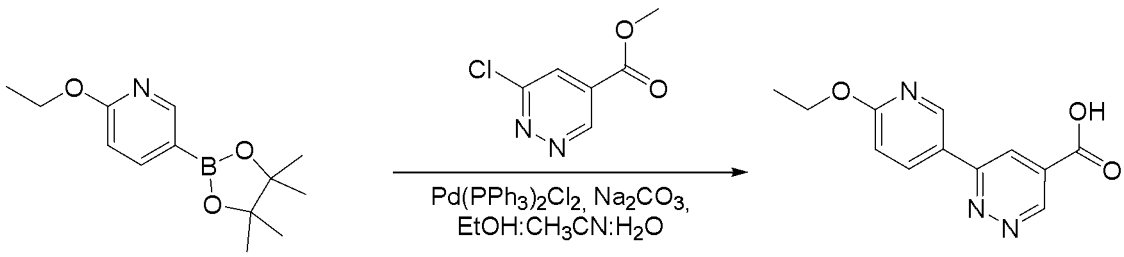


In a round-bottomed flask containing methyl 6-chloropyridazine-4-carboxylate (11.6 mmol) in EtOH (10 mL), MeCN (10 mL), and $H_2O$ (10 mL), $Na_2CO_3$ (11.6 mmol) and 2-ethoxy-5-(4,4,5,5-tetramethyl-1,3,2-dioxaborolan-2-yl) pyridine (12.7 mmol) were added at room temperature. Next, the mixture was purged with $N_2$ for 15 minutes and then $Pd(PPh_3)_2Cl_2$ (0.579 mmol) was added at room temperature under atmosphere of $N_2$. Subsequently, the mixture was again purged with $N_2$ for 5 minutes, heated to 100 °C and kept under agitation for 2 hours. After total consumption of the starting reagent (monitored by CCD and LC-MS), the mixture was cooled to room temperature and filtered with celite. The solid residue was washed with $H_2O$ (50 mL) and the filtrate was acidified by adding HCl (1.0 N) up to pH = 2-3, followed by extraction with AcOEt (50 mL x 3). The organic phase was washed with saturated sodium chloride solution *(brine)* (100 mL), dried on anhydrous sodium sulfate and concentrated under reduced pressure. The residue obtained was agitated with diethyl ether (30 mL) and dried under reduced pressure. 1.75 g (61% yield) of the product of interest were obtained in the form of a white solid.

**TABLE 4. FORMULA III INTERMEDIATES OBTAINED FROM THE CORRESPONDING REAGENTS FOLLOWING METHOD III-B.**

| Intermediate | Structure |
|---|---|
| III-31 | 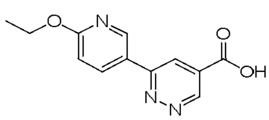 |
| III-32 | 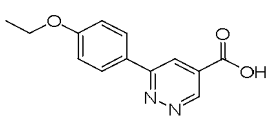 |
| III-33 | 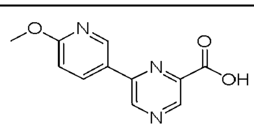 |
| III-34 | 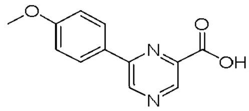 |

**FORMULA II INTERMEDIATES:**

Following General Scheme 1, the Formula II intermediates are presented, however, such methods are not exhaustive.

**METHOD II-A**

Step II-A-i:

*Precursors II-li - II-4i:*

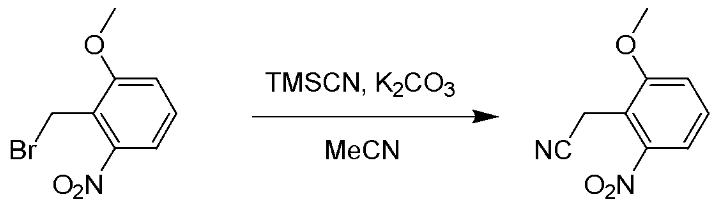


In a round-bottomed flask containing a solution of 2-(bromomethyl)-1-methoxy-3-nitrobenzene (1.0 eq.) in MeCN (9 mL), $K_2CO_3$ (1.20 eq.) and TMSCN (1.5 eq.) were added. The reaction mixture was heated to 60 °C and was kept under agitation for 4 hours. After full consumption of the starting reagent (monitored by CCD), the mixture was concentrated under reduced pressure to remove the solvent. The residue obtained was diluted with $H_2O$ (10 mL) and extracted with MTBE (5 mL x 3). The organic phase was concentrated under reduced pressure up to half the volume and the brown solid obtained was filtered and used in the next step without additional purification.

**TABLE 5. PRECURSORS OBTAINED FROM THE CORRESPONDING REAGENTS FOLLOWING *STEP II-A-i.***

| Precursor | Structure |
|---|---|
| *II-1i* | 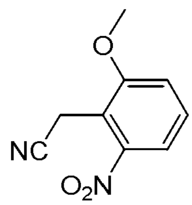 |
| *II-2i* | 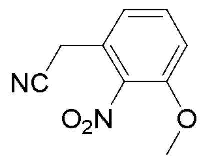 |
| *II-3i* | 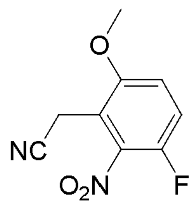 |
| *II-4i* | 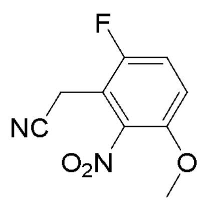 |

Step II-A-ii:

*Precursors II-lii - II-4ii:*

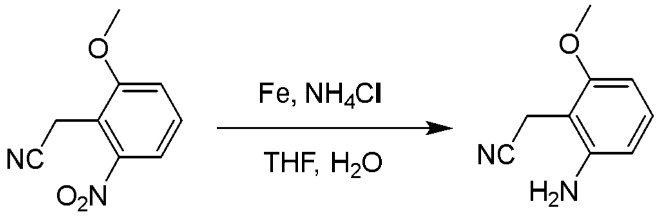


In a round-bottomed flask containing a solution of 2-(2-methoxy-6-nitrophenyl)acetonitrile (1.0 eq.) in THF (16 mL) and $H_2O$ (16 mL), $NH_4Cl$ (7.00 eq.) was added. Next, Fe (5.0 eq.) was added, the reaction mixture was heated to 35 °C and was kept under agitation for 16 hours. After total consumption of the starting reagent (monitored by CCD), the reaction mixture was filtered and diluted in AcOEt (30 mL). The residue obtained was extracted with AcOEt (10 mL x 3) and $H_2O$. The organic phase was concentrated under reduced pressure and purified by chromatography column (petroleum ether/AcOEt:50/10/1). The product obtained was used in the next step without further purification.

**TABLE 6. PRECURSORS OBTAINED FROM THE CORRESPONDING REAGENTS FOLLOWING *STEP II-A-ii.***

| Precursor | Structure |
|---|---|
| *II-1ii* | 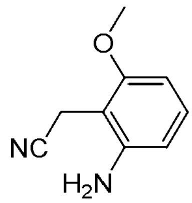 |
| *II-2ii* | 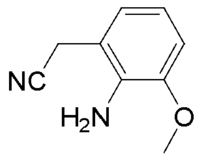 |
| *II-3ii* | 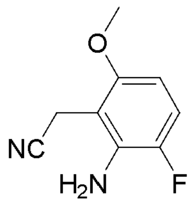 |
| *II-4ii* | 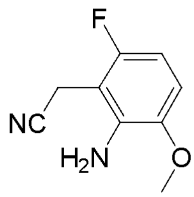 |

Step II-A-iii:

*Intermediates II-1* to *II-4:*

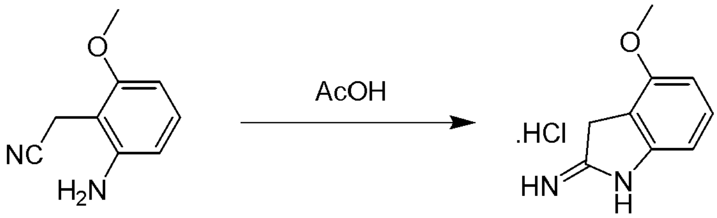


In a round-bottomed flask 2-(2-amino-6-methoxyphenyl)acetonitrile (1.0 eq.) was diluted in AcOH (9 mL). The reaction mixture was heated to 60 °C and was kept under agitation for 16 hours. After total consumption of the starting reagent (monitored by CCD), the reaction mixture was filtered and purified by chromatography column. The product was obtained as a white solid.

**TABLE 7. FORMULA II INTERMEDIATES OBTAINED FROM THE CORRESPONDING REAGENTS FOLLOWING *STEP II-A-iii.***

| Intermediate | Structure |
|---|---|
| II-1 | 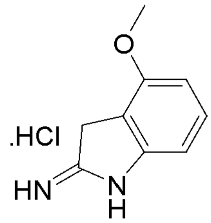 |
| II-2 | 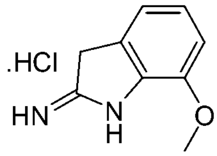 |
| II-3 | 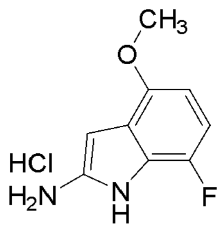 |
| II-4 | 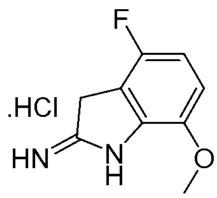 |

**METHOD II-B**

*Intermediates II-5* to *II-9:*

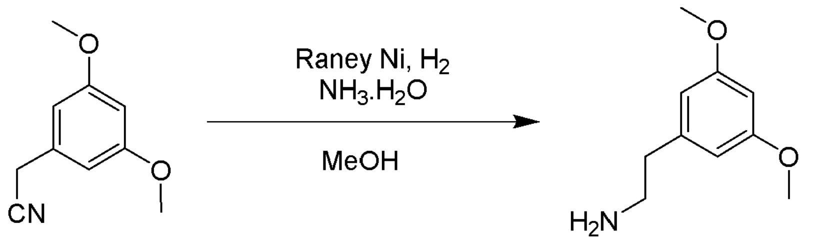


In a round-bottomed flask containing 2-(3,5-dimethoxyphenyl)acetonitrile (11.3 mmol) in MeOH (10.0 mL), $NH_3 \cdot H_2O$ (26.0 mmol) and Raney-Ni (23.3 mmol) were added under $N_2$ atmosphere. The mixture was de-gassed and purged with $H_2$ for 3 times. It was subsequently heated to 60 °C and maintained in agitation for 16 hours under an atmosphere of $H_2$ (50 Psi). After total consumption of the starting reagent (monitored by LC-MS), the mixture was filtered, washed with MeOH (20.0 mL x 3) and concentrated under reduced pressure. 2.00 g of the product of interest (97.7% yield) were obtained in the form of a colorless oil, which was sent to the next step without further purification.

**TABLE 8. FORMULA II INTERMEDIATES OBTAINED FROM THE CORRESPONDING REAGENTS FOLLOWING METHOD II-B.**

| Intermediate | Structure | Intermediate | Structure |
|---|---|---|---|
| II-5 | 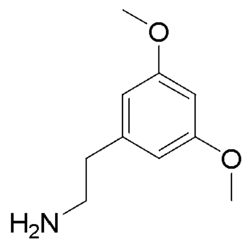 | II-8 | 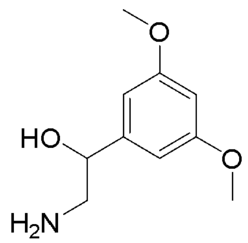 |
| II-6 | 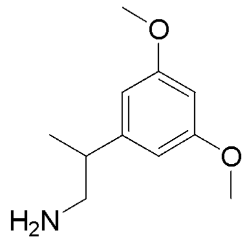 | II-9 | 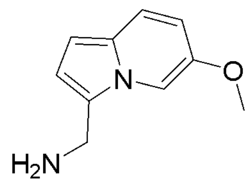 |
| II-7 | 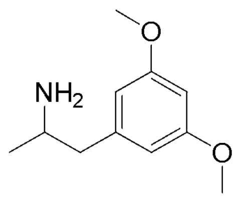 | | |

**METHOD II-C**

Step II-C-i:

*Precursor II-10i:*

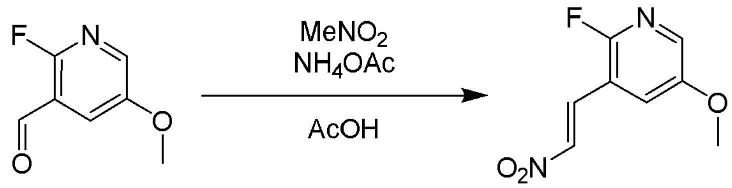


In a round-bottomed flask containing 2-fluoro-5-methoxycotinaldehyde (16.7 mmol) in AcOH (15.0 mL), $CH_3NO_2$ (207 mmol) and $NH_4OAc$ (8.38 mmol) were added. Subsequently, the reaction mixture was kept under agitation at 20 °C for 2 hours. After total consumption of the starting reagent (monitored by CCD), the mixture was diluted with $H_2O$ (30.0 mL) and extracted with DCM (15.0 mL x 3). The organic phase was washed with saturated sodium chloride solution (*brine*) (30.0 mL), dried on anhydrous sodium sulfate, filtered and concentrated under reduced pressure. The residue obtained was purified by column chromatography (petroleum ether/AcOEt:50/10/1). 1.70 g (51.1% yield) of the product of interest were obtained in the form of a yellow solid.

<u>Step II-C-ii:</u>

*Intermediate II-10:*

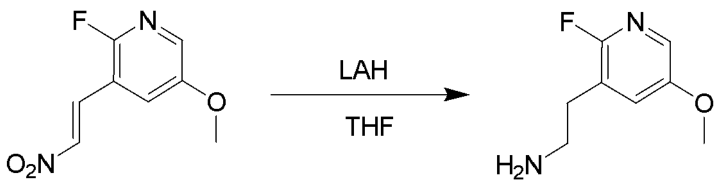


In a round-bottomed flask containing (*E*)-2-fluoro-5-methoxy-3-(2-nitrovinyl)pyridine (8.58 mmol) in THF (15.0 mL), $LiAlH_4$ (25.7 mmol) was added at 0 °C. Subsequently, the reaction mixture was kept under agitation at 20 °C for 2 hours. After total consumption of the starting reagent (monitored by LC-MS), $Na_2SO_4.10H_2O$ (1.50 g) was added to the reaction mixture at 0 °C. The mixture was then filtered and concentrated under reduced pressure. 800 mg (54.7% yield) of the product of interest (crude) was obtained in the form of a yellow solid, which was sent to the next step without further purification.

**METHOD II-D**

<u>Step II-D-i:</u>

*II-11i Precursors:*

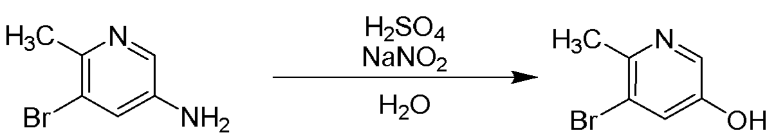


In a round-bottomed flask containing 5-bromo-6-methylpyridine-3-amine (106 mmol), $H_2SO_4$ (534 mmol) in $H_2O$ (190 mL), a solution of $NaNO_2$ (117 mmol) was added in $H_2O$ (10.0 mL) at 0 °C for 30 minutes. Subsequently, the reaction mixture was heated to 25 °C and kept under agitation for 16 hours. After full consumption of the starting reagent (monitored by CCD), $Na_2CO_3$ was added up to pH = 7. Next, the mixture was extracted with AcOEt (100 mL x 3). Subsequently, the organic phase was washed with saturated sodium chloride solution (*brine*) (100 mL), dried on anhydrous sodium sulfate, filtered, concentrated under reduced pressure, and purified by column chromatography (petroleum ether/AcOEt: 50/10/1). 7.60 g (37.6% yield) of the product of interest were obtained in the form of a yellow solid.

<u>Step II-D-ii:</u>

*Precursor II-11ii:*

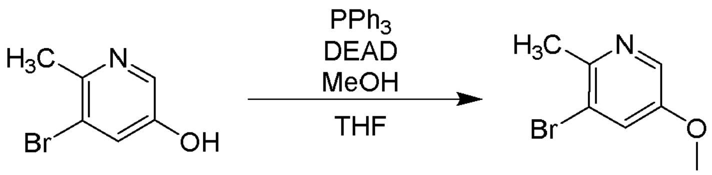


In a round-bottomed flask containing 5-bromo-6-methylpyridine-3-ol (34.5 mmol) in THF (65.0 mL), $PPh_3$ (51.8 mmol), MeOH (69.1 mmol), and DEAD (51.8 mmol) were added at 0 °C under an atmosphere of $N_2$. Subsequently, the reaction mixture was heated to 25 °C and kept under agitation for 16 hours. After total consumption of the starting reagent (monitored by CCD), the reaction mixture was diluted with $H_2O$ (50.0 mL) and extracted with AcOEt (20.0 mL x 3). Subsequently, the organic phase was concentrated under reduced pressure and purified by column chromatography (petroleum ether/AcOEt:50/10/1). 4.40 g (62.7% yield) of the product of interest were obtained in the form of a colorless oil.

Step II-D-iii:

*Precursors II-11iii to II-13iii:*

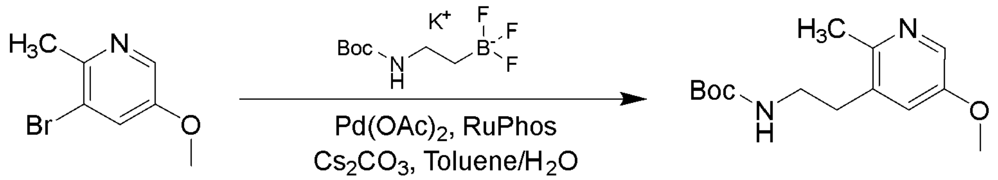


A round-bottomed flask containing the mixture of 3-bromo-5-methoxy-2-methylpyridine (9.90 mmol), (2-((tert-butoxycarbonyl)amino)ethyl)potassium trifluoroborate (10.8 mmol), RuPhos (989 µmol), $Pd(OAc)_2$ (494 µmol) and $Cs_2CO_3$ (19.8 mmol) in toluene (9.00 mL) and $H_2O$ (3.00 mL) was de-gassed and purged with $N_2$ 3 times. Subsequently, the reaction mixture was heated to 90 °C and kept in agitation for 16 hours under $N_2$ atmosphere. After total consumption of the starting reagent (monitored by CCD), the mixture was diluted with $H_2O$ (20.0 mL) and extracted with AcOEt (15.0 mL x 3). The organic phase was concentrated under reduced pressure. The residue obtained was purified by preparative HPLC ([$H_2O$ (HCl) /ACN] 5% → 35% B). 1.60 g (60.2% yield) of the product of interest were obtained in the form of a colorless oil.

**TABLE 9. PRECURSORS OBTAINED FROM THE CORRESPONDING REAGENTS FOLLOWING *STEP II-D-iii.***

| Precursor | Structure |
|---|---|
| *II-11iii* | 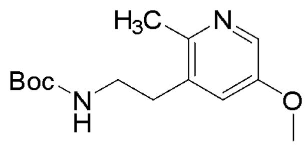 |
| *II-12iii* | 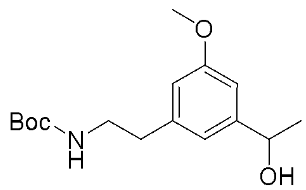 |
| *II-13iii* | 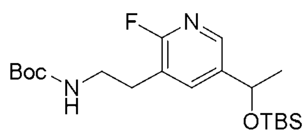 |

Step II-D-iii:

*Intermediates II-11a II-13:*

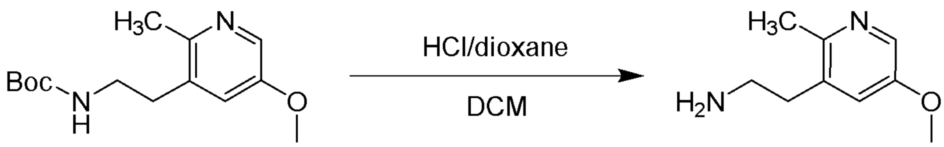


In a round-bottomed flask containing *tert*-butyl (6.01 mmol) carbamate in DCM (4.00 mL), HCl/dioxane (4 M, 4.00 mL) was added. Subsequently, the reaction mixture was kept under agitation at 25 °C for 30 minutes. After total consumption of the starting reagent (monitored by LC-MS), the mixture was concentrated under reduced pressure. 1.20 g (98.5% yield, HCl) of the product of interest were obtained in the form of a white solid, which was sent to the next step without further purification.

**TABLE 10. FORMULA II INTERMEDIATES OBTAINED FROM THE CORRESPONDING REAGENTS FOLLOWING METHOD II-D.**

| Intermediate | Structure |
|---|---|
| II-11 | 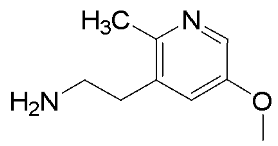 |
| II-12 | 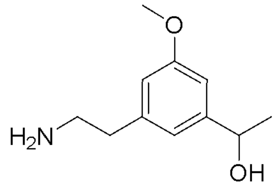 |
| II-13 | 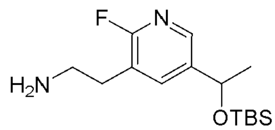 |

**METHOD II-E**

Step II-E-i:

*Precursors II-14i and II-15i:*

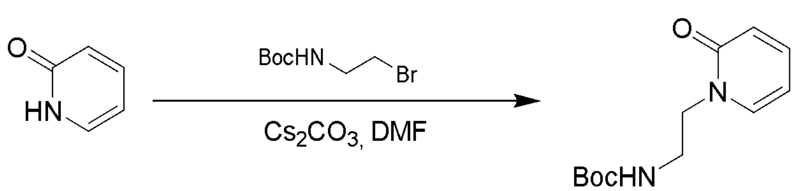


In a round-bottomed flask containing pyridin-2(1H)-one (18.9 mmol) in DMF (20.0 mL), (2-bromoethyl)carbamate of tert-butyl (20.8 mmol) and $Cs_2CO_3$ (28.3 mmol) were added. Subsequently, the reaction mixture was kept under agitation at 20 °C for 16 hours. After total consumption of the starting reagent (monitored by CCD), the mixture was diluted in $H_2O$ (20.0 mL) and extracted with AcOEt (20.0 mL x 3). The organic phase was washed with saturated sodium chloride

solution *(brine)* (50.0 mL), dried on anhydrous sodium sulfate, filtered and concentrated under reduced pressure. The residue obtained was purified by column chromatography (petroleum ether/AcOEt:10/10/1). 3.37 g (72.9% yield) of the product of interest were obtained in the form of a white solid.

**TABLE 11. PRECURSORS OBTAINED FROM THE CORRESPONDING REAGENTS FOLLOWING *STEP II-E-i.***

| Precursor | Structure |
|---|---|
| *II-14i* | 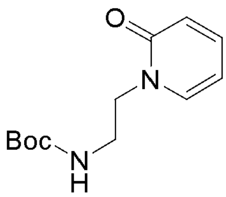 |
| *II-15i* | 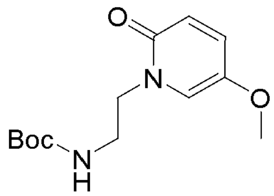 |

Step II-E-ii:

*Intermediates II-14 and II-15:*

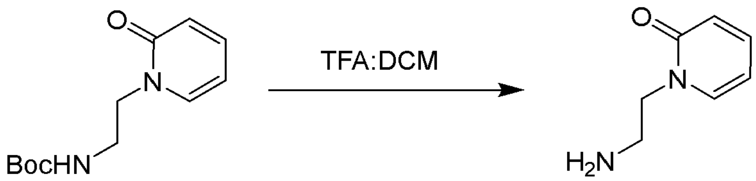


In a round-bottomed flask containing (2-(2-oxopyridin- 1(2 *H*-yl)-ethyl)carbamate (*tert*}-butyl (14.1 mmol), TFA (18.0 mL) and DCM (6.00 mL) were added. Subsequently, the reaction mixture was kept under agitation at 25 °C for 16 hours. After total consumption of the starting reagent (monitored by CCD), the mixture was concentrated under reduced pressure. 6.00 g (84.1% yield) of the product of interest (crude) were obtained in the form of a yellow oil, which was sent to the next step without further purification.

**TABLE 12. INTERMEDIATES OBTAINED FROM THE CORRESPONDING REAGENTS FOLLOWING *STEP II-E-ii.***

| Intermediate | Structure |
|---|---|
| II-14 | 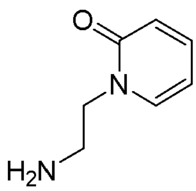 |
| II-15 | 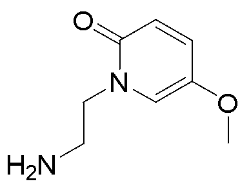 |

**METHOD II-F**

Step II-F-i:

*Precursor II-16i:*

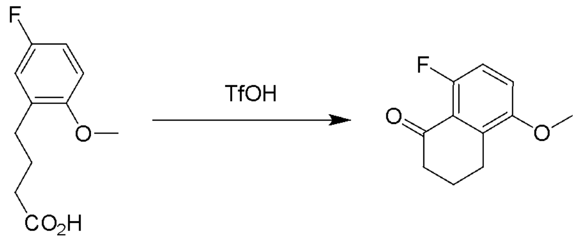


In a round-bottomed flask containing 4-(5-fluoro-2-methoxyphenyl)butanoic acid (42.4 mmol), TfOH (100 mL) was added. Subsequently, the reaction mixture was heated to 40 °C and kept under agitation for 2 hours. After total consumption of the starting reagent (monitored by CCD), the reaction mixture was diluted with $H_2O$ (100 mL) and extracted with DCM (100 mL x 3). The organic phase was washed with saturated sodium chloride solution (*brine*) (200 mL) and concentrated under reduced pressure. The residue obtained was purified by column chromatography (petroleum ether/AcOEt: 10/1 - 0/1). 6.43 g (77.7% yield) of the product of interest were obtained in the form of a white solid.

Step II-F-ii:

*Precursor II-16ii:*

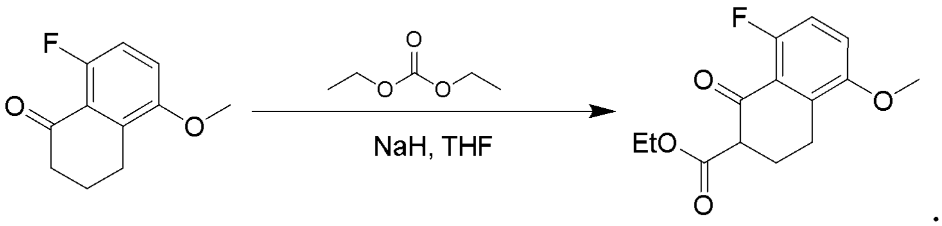


In a round-bottomed flask containing a suspension of NaH (43.2 mmol) in THF (30.0 mL), diethyl carbonate (32.4 mmol) and next 8-fluoro-5-methoxy-3,4-dihydronaphthalen-1(2H)-one (21.6 mmol) in THF (10.0 mL) dropwise was added. Subsequently, the reaction mixture was heated to 70 °C and kept under agitation for 2 hours. After total consumption of the starting reagent (monitored by CCD and HPLC), the reaction mixture was cooled to room temperature and citric acid (2 M) was added. Then, the mixture was diluted in $H_2O$ (40.0 mL) and extracted with AcOEt (40.0 mL x 3). The organic phase was washed with saturated sodium chloride solution (*brine*) (80.0 mL) and concentrated under reduced pressure. 6.13 g (91.5% yield) of the product of interest were obtained in the form of a brown solid, which was sent to the next step without further purification.

Step II-F-iii:

*Precursor II-16iii:*

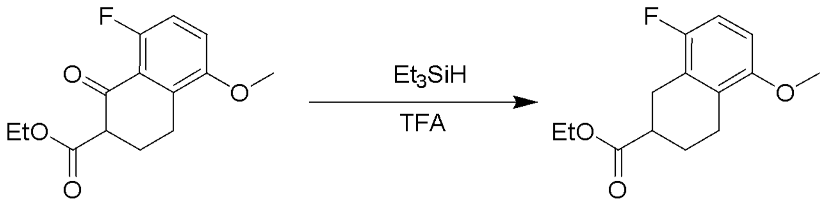


In a round-bottomed flask containing 8-fluoro-5-methoxy-1-oxo-1,2,3,4-ethyl tetrahydronaphthalene-2-carboxylate (19.8 mmol) in TFA (60.0 mL), $Et_3SiH$ (59.4 mmol) was added dropwise at 10-15 °C. Subsequently, the reaction mixture was kept at 10-15 °C under agitation for 30 minutes. After total consumption of the starting reagent (monitored by CCD), most of the TFA was removed, the resulting mixture was diluted in $H_2O$ (40.0 mL) and extracted with AcOEt (40.0 mL x 3). The organic phase was washed with saturated sodium chloride solution (*brine*) (80.0 mL), dried on anhydrous sodium sulfate and concentrated under reduced pressure. The residue obtained was purified by column chromatography (petroleum ether/AcOEt: 10/1 $\rightarrow$ 0/1). 4.36 g (84.6% yield) of the product of interest were obtained in the form of a yellow solid.

Step II-F-iv:

*Precursor II-16iv:*

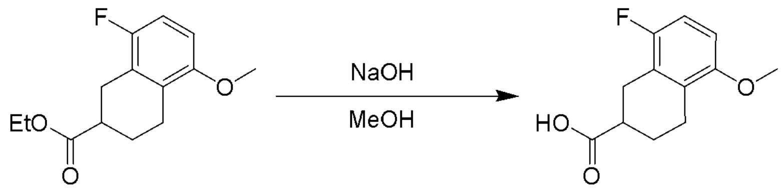


In a round-bottomed flask containing ethyl 8-fluoro-5-methoxy-1,2,3,4-tetrahydronaphthalene-2-carboxylate (17.2 mmol) in MeOH (45.0 mL), NaOH (1 M, 51.8 mL) was added dropwise. Subsequently, the reaction mixture was kept at 20 °C under agitation for 16 hours. After full consumption of the starting reagent (monitored by LC-MS), the reaction mixture was concentrated under reduced pressure to remove the solvent. Next, HCl (2 M) was added up to pH = 1-2 and the mixture was filtered. The residue was washed with $H_2O$ (60.0 mL) and dried under vacuum. 3.20 g (78.4% yield) of the product of interest were obtained in the form of a white solid, which was sent to the next step without further purification.

Step II-F-v:

*Precursor II-16v:*

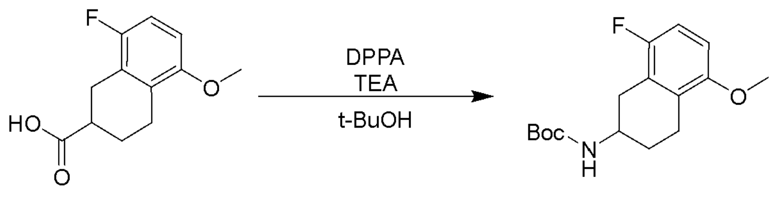


DPPA (11.6 mmol) was added to a round-bottomed flask containing 8-fluoro-5-methoxylic-1,2,3,4-tetrahydronaphthalene-2-carboxylic acid (9.69 mmol) and TEA (19.3 mmol) in t-BuOH (20.0 mL). Subsequently, the reaction mixture was kept at 90 °C under agitation for 2 hours. After total consumption of the starting reagent (monitored by CCD), the solvent was evaporated under reduced pressure. Next, the residue was diluted in $H_2O$ (20.0 mL) and extracted with AcOEt (20.0 mL x 3). The organic phase was washed with saturated sodium chloride solution (*brine*) (40.0 mL) and concentrated under reduced pressure. 3.13 g (71.1% yield) of the product of interest were obtained in the form of a yellow oil, which was sent to the next step without further purification.

Step II-F-vi:

*Intermediate II-16:*

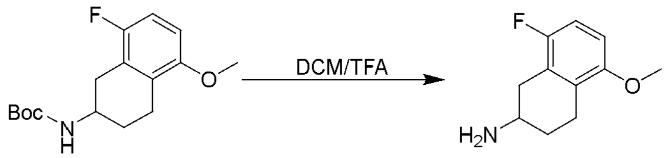


In a round-bottomed flask containing *tert*-butyl (8-fluoro-5-methoxy-1,2,3,4-tetrahydronaphthalene-2-yl)carbamate, TFA (7.00 mL) and DCM (21.0 mL) were added. Subsequently, the reaction mixture was kept under agitation at 20 °C for 2 hours. After total consumption of the starting reagent (monitored by CCD), the mixture was concentrated under reduced pressure, diluted in $H_2O$ (60.0 mL) and extracted with AcOEt (30.0 mL x 3). A solution of $Na_2CO_3$ up to pH = 10 was added to the aqueous phase, the mixture was diluted in $H_2O$ (60.0 mL) and extracted with AcOEt (60.0 mL x 3). The organic phase was concentrated under reduced pressure. 1.28 g (92.1% yield) of the product of interest (crude) were obtained in the form of a brown oil, which was sent to the next step without further purification.

**METHOD II-G**

Step II-G-i:

*Precursor II-17i:*

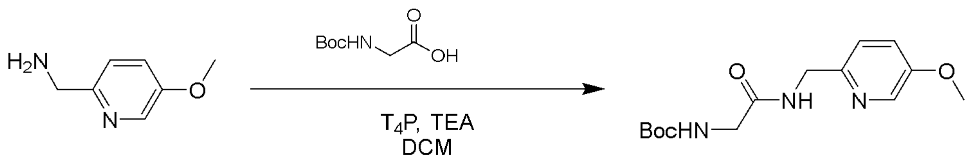


In a round-bottomed flask containing (5-methoxypyridine-2-yl)methenamine (15.5 mmol) in DCM (50 mL), (*tert*-butoxycarbonyl)glycine (15.5 mmol), $T_4P$ (23.3 mmol), and TEA (31.1 mmol) was added at 0 °C. Subsequently, the reaction mixture was heated to 20 °C and kept under agitation for 16 hours. After total consumption of the starting reagent (monitored by LC-MS), the mixture was diluted with $H_2O$ (50 mL) and extracted with DCM (150 mL). The organic phase was concentrated under reduced pressure. 4.00 g (87.0% yield) of the product of interest were obtained in the form of a yellow oil.

Step II-G-ii:

*Precursor II-17ii:*

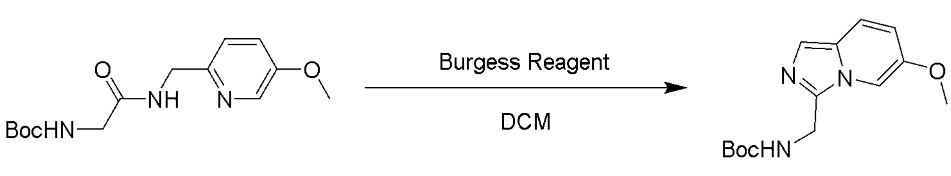


In a round-bottomed flask containing *tert*-butyl (13.5 mmol) in DCM (50 mL), Burgess's reagent (135 mmol) was added to a round-bottomed flask containing (2-(((5-methoxypyridine-2-yl)methyl)amino)-2-oxoethyl)carbamate. Subsequently, the reaction mixture was kept under agitation at 0-25 °C for 5 hours. After total consumption of the starting reagent (monitored by LC-MS), the mixture was diluted with $H_2O$ (100 mL) and extracted with DCM (300 mL). The organic phase was concentrated under reduced pressure and purified by preparative HPLC ([$H_2O(NH_4HCO_3)$ / ACN] 30% → 60% B).

1.10 g (29.2% yield) of the product of interest were obtained in the form of a white solid.

Step II-G-iii:

*Intermediate II-17:*

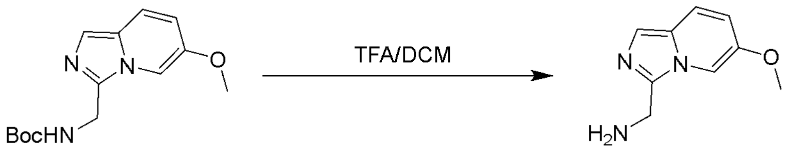


In a round-bottomed flask containing ((6-methoxyimidazo[1,5-a]pyridine-3-yl)methyl)carbamate of *tert*-butyl (3.61 mmol) in DCM (1 mL), TFA (5.36 mL) was added. Subsequently, the reaction mixture was kept under agitation at 25 °C for 2 hours. After total consumption of the starting reagent (monitored by LC-MS), the mixture was concentrated under reduced pressure. 1.00 g (95.2% yield) of the product of interest (crude) were obtained in the form of a white solid.

**METHOD II-H**

Step II-H-i:

*Precursor II-18i:*

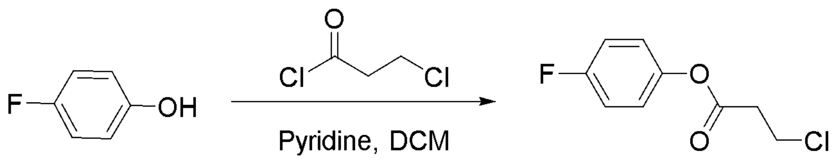


In a round-bottomed flask containing 4-fluorophenol (44.64 mmol) in DCM (50 mL), pyridine (6.0 mL) and 3-chloropropanoyl chloride (44.64 mmol) were added drip at 0 °C. Subsequently, the reaction mixture was heated to room temperature and kept under agitation for 1 hour. After total consumption of the starting reagent (monitored by CCD), the mixture was diluted with $H_2O$ and extracted with AcOEt. The organic phase was dried on anhydrous sodium sulfate, concentrated under reduced pressure and purified by column chromatography (hexane/AcOEt: 9/1 → 8/2). 2.0 g (22% yield) of the product of interest were obtained.

Step II-H-ii:

*Precursor II-18ii:*

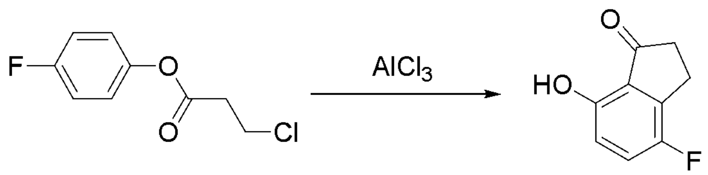


A round-bottomed flask containing 4-fluorophenyl 3-chloropropanoate (24.10 mmol) in $AlCl_3$ (72.27 mmol) was heated to 180 °C and kept under agitation for 3 hours. After total consumption of the starting reagent, the reaction mixture was diluted with $H_2O$ and extracted with AcOEt. The organic phase was dried on anhydrous sodium sulfate, concentrated under reduced pressure and purified by column chromatography (hexane/AcOEt: 10/0 → 80/20). 2.0 g (49.96% yield) of the product of interest were obtained.

Step II-H-iii:

*Precursor II-18iii:*

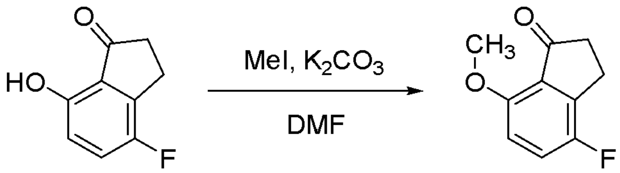


In a round-bottomed flask containing 4-fluoro-7-hydroxy-2,3-dihydro-1H-inden-1-one (12.05 mmol) in DMF (20 mL), $K_2CO_3$ (24.09 mmol) and methyl iodide (60.23 mmol) were added. Subsequently, the reaction mixture was kept under agitation at room temperature for 16 hours. After total consumption of the starting reagent, the reaction mixture was diluted with $H_2O$ and extracted with AcOEt. The organic phase was dried on anhydrous sodium sulfate, concentrated under reduced pressure and purified by column chromatography (hexane/AcOEt: 100/0 → 50/50). 1.0 g (46% yield) of the product of interest were obtained.

Step II-H-iv:

*Precursor II-18 iv:*

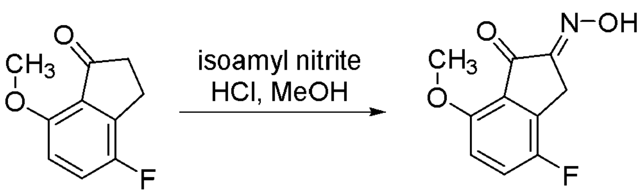


In a round-bottomed flask containing 4-fluoro-7-methoxy-2,3-dihydro-1H-inden-1-one (5.55 mmol) in MeOH (10 mL), isoamyl nitrite (1.2 mL) and concentrated HCl (0.75 mL) were added. Subsequently, the reaction mixture was heated to 40 °C and kept under agitation for 2 hours. Next, MeOH (5 mL) and isoamyl nitrite (0.5 mL) were added. The mixture was cooled to room temperature and kept under agitation for another 16 hours. After total consumption of the starting reagent (monitored by CCD and LC-MS), the reaction mixture was filtered and dried under reduced pressure. 500 mg (43% yield) of the product of interest were obtained.

Step II-H-v:

*Intermediate II-18vi:*

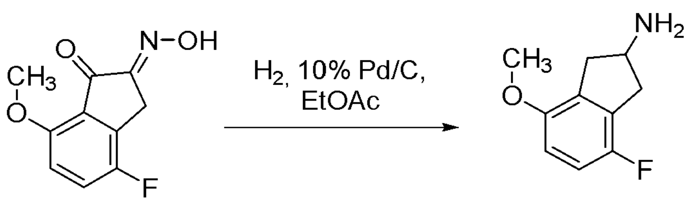


In a round-bottomed flask containing (E)-4-fluoro-2-(hydroxyimin)-7-methoxy-2,3-dihydro-1H-inden-1-one (7.18 mmol) in AcOH (7.5 mL), $H_2SO_4$ (0.5 mL) and 10%Pd/C (700 mg) were added. Subsequently, the reaction mixture was kept under agitation at room temperature for 16 hours under $H_2$ atmosphere. After total consumption of the starting reagent (monitored by CCD and LC-MS), the reaction mixture was filtered with celite and concentrated under reduced pressure. 700 mg (53.84% yield) of the product of interest were obtained.

**METHOD II-I**

Step II-I-iv:

*Intermediates II-19 and II-20i:*

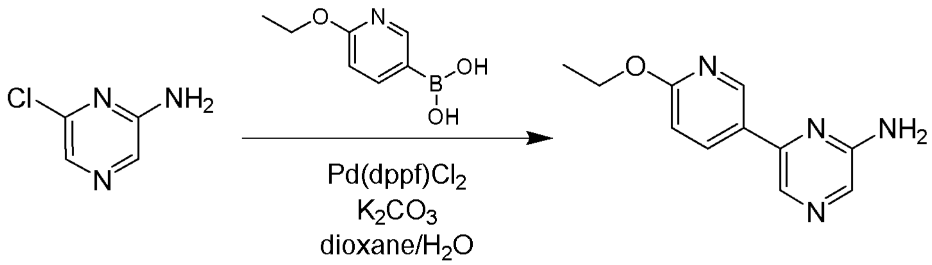


In a round-bottomed flask containing 6-chloropyrazine-2-amino (7.72 mmol) and (6-ethoxypyridine-3-yl)boronic acid in dioxane (6.00 mL), Pd(dppf)$Cl_2$ (463 μmol) was added at 25 °C and the mixture was kept in agitation for 30 minutes. Next, $K_2CO_3$ (25.7 mmol) in $H_2O$ (4.00 mL) was added. Subsequently, the reaction mixture was heated to 100 °C and maintained under agitation for 16 hours under an atmosphere of $N_2$. After total consumption of the starting reagent (monitored by CCD and LC-MS), the reaction mixture was diluted with AcOEt (300 mL) and extracted with saturated sodium chloride solution (*brine*) (100 mL x 3). The mixture was filtered, and the phases were separated. The aqueous phase was extracted with AcOEt (100 mL x 3). The organic phases were pooled, dried on anhydrous sodium sulfate and concentrated in a vacuum. The residue was purified by column chromatography (petroleum ether/AcOEt: 100/1 → 5/1). 1.30 g (77.8% yield) of the product of interest were obtained in the form of a yellow solid.

**TABLE 13. FORMULA II INTERMEDIATES OBTAINED FROM THE CORRESPONDING REAGENTS FOLLOWING METHOD II-I.**

| Intermediate | Structure |
|---|---|
| II-19 | 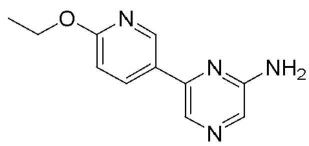 |
| II-20 | 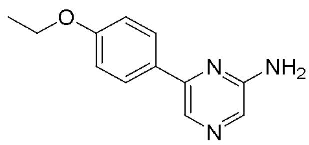 |

**METHOD** II-J

Step II-J-i:

*Precursors II-21i* to *II-24i:*

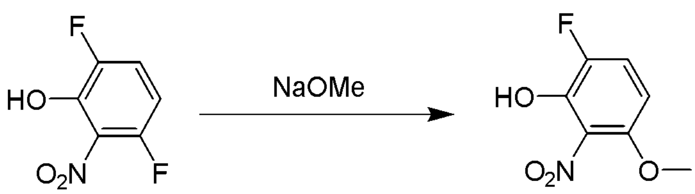

In a round-bottomed flask containing 3,6-difluoro-2-nitrophenol (5.14 mmol), NaOMe (25.7 mmol) was added. Subsequently, the reaction mixture was heated to 60 °C and kept under agitation for 16 hours. After total consumption of the starting reagent (monitored by LC-MS), the reaction mixture was diluted with $H_2O$ (10.0 mL) and extracted with AcOEt (7.00 mL x 2). The organic phase was concentrated under reduced pressure. 930 mg (96.6% yield) of the product of interest (crude) were obtained in the form of a yellow solid, which was sent to the next step without further purification.

**TABLE 14. PRECURSORS OBTAINED FROM THE CORRESPONDING REACTANTS FOLLOWING *STEP II-J-i.***

| Precursor | Structure |
|---|---|
| *II-21i* | 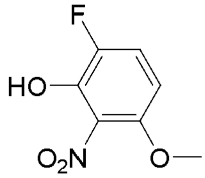 |
| *II-22i* | 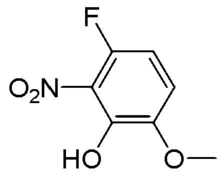 |
| *II-23i* | 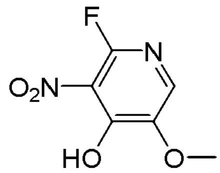 |
| *I-24i* | 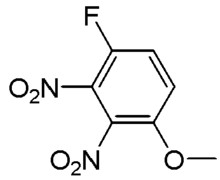 |

Step II-J-ii:

*Precursors II-21ii* to *II-23ii:*

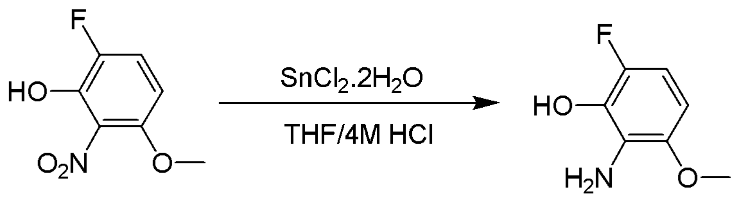


In a round-bottomed flask containing 6-fluoro-3-methoxy-2-nitrophenol (4.97 mmol) in THF (9.30 mL), $SnCl_2 \cdot 2H_2O$ (14.9 mmol) and HCl (4.00 M, 9.30 mL) were added. Subsequently, the reaction mixture was heated to 70 °C and kept under agitation for 2 hours. After total consumption of the starting reagent (monitored by LC-MS), the reaction mixture was diluted with $H_2O$ (10.0 mL) counting excess $NaHCO_3$ and AcOEt (10.0 mL). Next, the mixture was filtered and extracted with AcOEt (8.00 mL x 2). The organic phase was concentrated under reduced pressure. 710 mg (90.9% yield) of the product of interest (crude) were obtained in the form of a yellow solid, which was sent to the next step without further purification.

**TABLE 15. PRECURSORS OBTAINED FROM THE CORRESPONDING REACTANTS FOLLOWING *STEP II-J-ii.***

| **Precursor** | **Structure** |
|---|---|
| *II-21ii* | 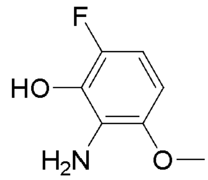 |
| *II-22ii* | 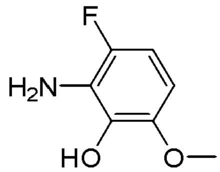 |
| *II-23ii* | 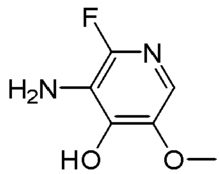 |
| *I-24ii* | 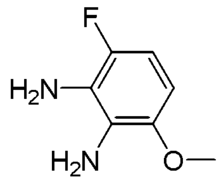 |

Step II-J-iii:

*Intermediates II-21iii to II-24iii:*

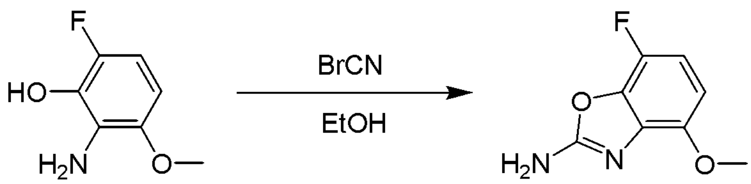


In a round-bottomed flask containing 2-amino-6-fluoro-3-methoxyphenol (3.63 mmol) in EtOH (5.70 mL), BrCN (8.69 mmol) was added. Subsequently, the reaction mixture was heated to 50 °C and kept under agitation for 2 hours. After total consumption of the starting reagent (monitored by LC-MS), a solution of $NaHCO_3$ (10.0%) was added up to pH = 7. The mixture was then filtered and concentrated under reduced pressure. 480 mg (72.6% yield) of the product of interest (crude) were obtained in the form of a black solid, which was sent to the next step without further purification.

**TABLE 16. INTERMEDIATES OBTAINED FROM THE CORRESPONDING REAGENTS FOLLOWING *STEP II-J-iii.***

| **Intermediate** | **Structure** |
|---|---|
| II-21 | 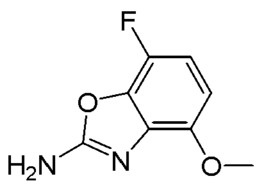 |

(continued)

| Intermediate | Structure |
|---|---|
| II-22 | 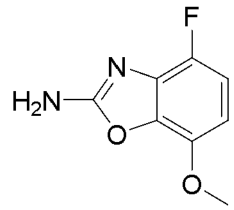 |
| II-23 | 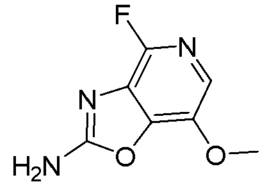 |
| I-24 | 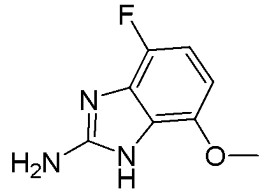 |

## EXAMPLES

The following examples, described in detail, serve to illustrate the embodiments of this invention without, however, having any limitation character to the scope of protection thereof.

### EXAMPLE 1: *N*-(3,5-DIMETHOXYPHENETHYL)-6-(4-ETHOXY-2-METILPHENYL)PYRAZINE-2-CARBOXAMIDE.

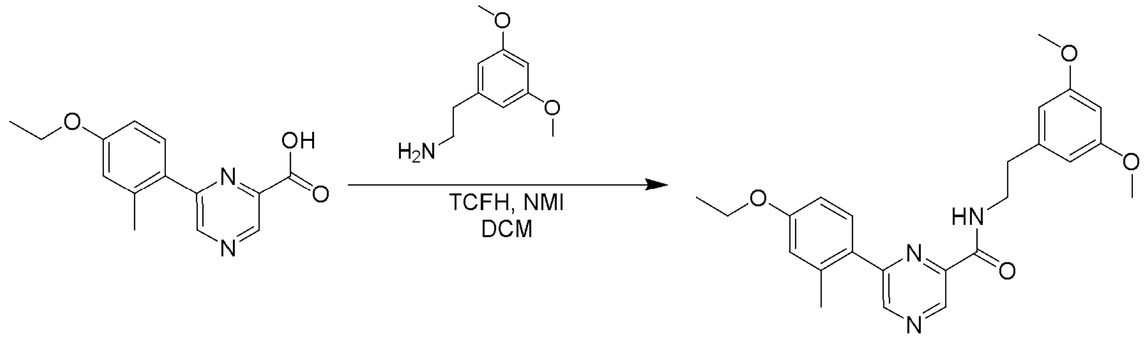


In a round-bottomed flask containing 6-(4-ethoxy-2-methylphenyl)pyrazine-2-carboxylic acid (1.16 mmol), 2-(3,5-dimethoxyphenyl)ethanamine (1.39 mmol) and NMI (2.44 mmol) in DCM (10.0 mL), TCFH (1.74 mmol) was added at 0 °C. Subsequently, the reaction mixture was kept under agitation at 20 °C for 16 hours. After total consumption of the starting reagent (monitored by LC-MS), the mixture was diluted with $H_2O$ (10 mL) and extracted with DCM (10.0 mL x 3). The organic phase was dried on anhydrous sodium sulfate, filtered and concentrated under reduced pressure. The residue obtained was placed under agitation with MeOH (5 mL) at 20 °C for 30 minutes. 400 mg (80.0% yield) of the product of interest were obtained in the form of a white solid. Compounds 1-25, 28-53, 61-64, 70-71, 74-78, 83-84, 88-90, 93, 96-104 and 107-109, as shown in Table 17, are obtained by the procedure described for Example 1, changing the corresponding intermediates II and III.

### EXAMPLE 2: (*S*)-*N*-(2-AMINO-2-(3,5-DIMETHOXYPHENYL)ETHYL)-6-(4-ETHOXYPHENYL)PYRAZINE-2-CARBOXAMIDE.

Step I-2-i:

*Precursor I-2i:*

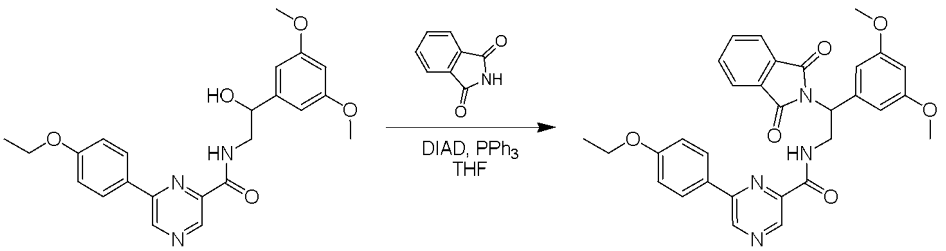


In a round-bottomed flask containing N-(2-(3,5-dimethoxyphenyl)-2-hydroxyethyl)-6-(4-ethoxyphenyl)pyrazine-2-carboxamide (1.65 mmol) in THF (10 mL) and $PPh_3$ (4.96 mmol) at 25 °C, DIAD (20.2 mmol) at 0 °C was added. Subsequently, the reaction mixture was kept under agitation at 25 °C for 12 hours. After total consumption of the starting reagent (monitored by LC-MS), the reaction mixture was concentrated under reduced pressure for solvent removal, diluted in $H_2O$ (30 mL) and extracted with AcOEt (50 mL x 3). The organic phase was dried on anhydrous sodium sulfate, filtered and concentrated under reduced pressure. The residue was purified by column chromatography (petroleum ether/AcOEt: 100/1 → 0/1). 630 mg (68.9% yield) of the product of interest were obtained in the form of a white solid.

Step I-2-ii:

*Formula I Compound:*

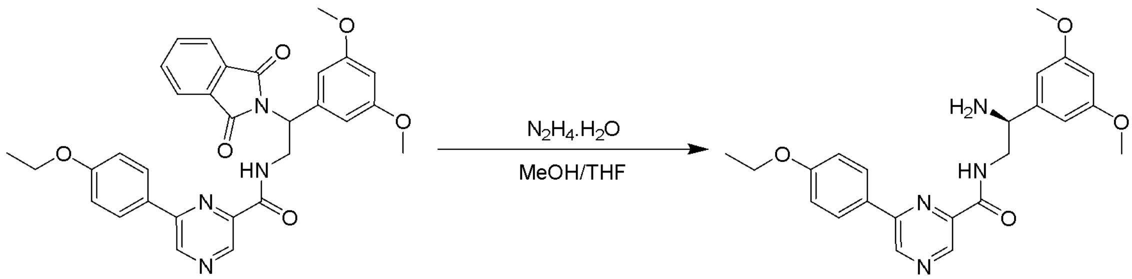


In a round-bottomed flask containing N-(2-(3,5-dimethoxyphenyl)-2-(1,3-dioxoisoindolin-2-yl)ethyl)-6-(4-ethoxyphenyl)pyrazine-2-carboxamide (361 μmol) in MeOH (12 mL) and THF (12 mL), $N_2H_4.H_2O$ (3.62 mmol) at 25 °C was added. Subsequently, the reaction mixture was heated to 65 °C and kept under agitation for 12 hours. After total consumption of the starting reagent (monitored by HPLC), the reaction mixture was concentrated under reduced pressure for solvent removal, diluted in $H_2O$ (30 mL) and extracted with DCM/MeOH 10:1 (50 mL x 8). The organic phase was dried on anhydrous sodium sulfate, filtered and concentrated under reduced pressure. The residue was purified by preparative HPLC ([$H_2O$(FA 0.2%) / ACN] 15% → 45% B). 186 mg of the isomer (*S*) and 189 mg of the isomer (*R*) were obtained, both in the form of a white solid.

Compounds 26 and 27, as shown in Table 17, are obtained by the procedure described for Example 1, changing the corresponding intermediates II and III.

**EXAMPLE 3: 6-(6-ETHOXYPYRIDINE-3-IL)-*N*-(4-FLUORO-7-METHOXY-2,3-DIHYDRO-1*H*-INDEN-2-IL)PYRAZINE-2-CARBOXAMIDE.**

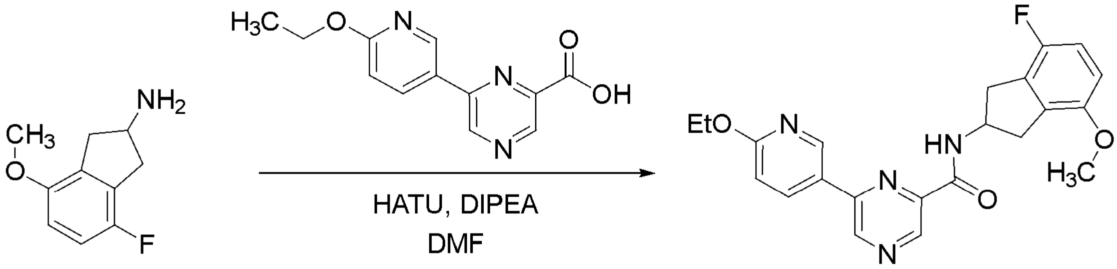


In a round-bottomed flask containing 6-(6-ethoxypyridine-3-yl)pyrazine-2-carboxylic acid (3.86 mmol) in DMF (7 mL) and EDC· HCl (5.8 mmol), HOBT (5.8 mmol) was added at 0 °C. Subsequently, the reaction mixture was kept under agitation at room temperature for 1 hour. Then, 4-fluoro-7-methoxy-2,3-dihydro-1H-inden-2-amine (3.87 mmol) was added and the reaction mixture was kept under agitation for another 16 hours. After total consumption of the starting reagent (monitored by CCD and LC-MS), the mixture was diluted in $H_2O$ (30 mL) and extracted with 10:1 AcOEt (50 mL x 8). The organic phase was dried on anhydrous sodium sulfate, filtered and concentrated under reduced pressure. The residue was purified by column chromatography. The racemic mixture obtained was separated by chiral HPLC. 140 mg (9%) and 150 mg (10%) of the separated isomers were obtained.

Compounds 56, 60, 68, 81-84, as shown in Table 17, are obtained by the procedure described for Example 1, changing the corresponding intermediates II and III.

**EXAMPLE 4: *N*-(2-(2-FLUORO-5-METHOXYPYRIDINE-3-IL)ETHYL)-6-(6-(1-HYDROXYETHYL)PYRIDINE-3-IL) PYRAZINE-2-CARBOXAMIDE.**

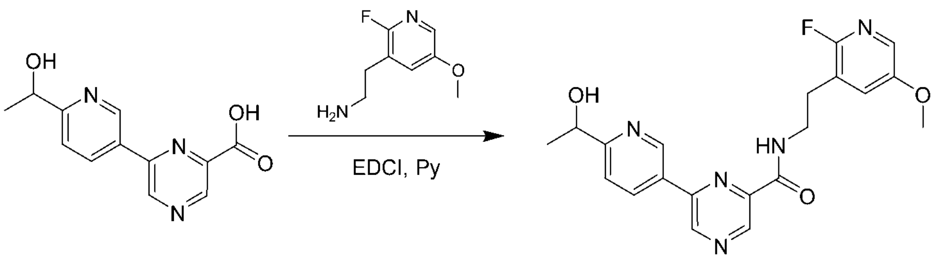


In a round-bottomed flask containing 6-(6-(1-hydroxyethyl)pyridine-3-yl)pyrazine-2-carboxylic acid (815 μmol) in pyridine (2.00 mL), EDCI (1.63 mmol) and 2-(2-fluoro-5-methoxypyridine-3-yl)etan-1-amine (1.22 mmol) were added. Subsequently, the reaction mixture was kept under agitation at 25 °C for 16 hours. After total consumption of the starting reagent (monitored by LC-MS), the mixture was filtered, and the residue was purified by preparative HPLC column chromatography ([$H_2O(NH_4HCO_3$ 10 mM) / ACN] 15% → 45% B) . 200 mg (61.0% yield) of the product of interest were obtained in the form of a yellow solid.

Compounds 57, 58 and 59, as shown in Table 17, are obtained by the procedure described for Example 1, changing the corresponding intermediates II and III.

**EXAMPLE 5: 6-(6-ETHOXYPYRIDINE-3-IL)-*N*-(4-FLUORO-7-METHOXYOXAZOLE[4,5-C]PYRIDINE-2-IL)PYRAZINE-2-CARBOXAMIDE.**

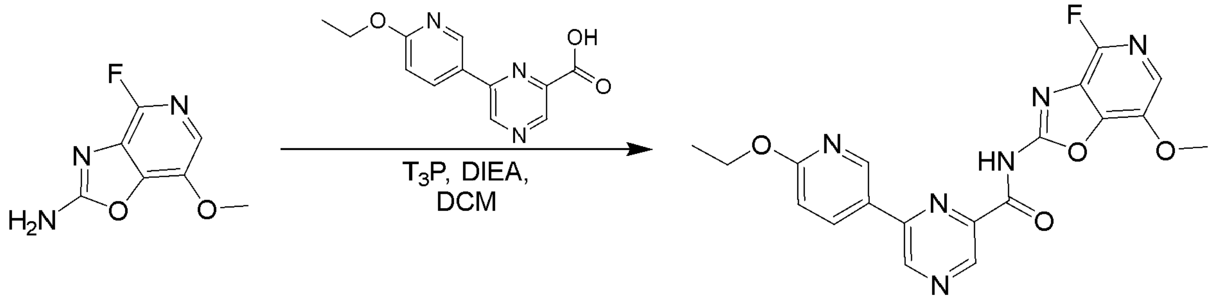


In a round-bottomed flask containing 6-(6-ethoxypyridine-3-yl)pyrazine-2-carboxylic acid (834 μmol) in DCM (1.40 mL), 4-fluoro-7-methoxy-oxazole[4,5-c]pyridine-2-amine (876 μmol), DIEA (2.50 mmol), and $T_3P$ (3.03 mmol) were added at 0 °C. Subsequently, the reaction mixture was maintained under agitation at 25 °C for 16 hours under $N_2$ atmosphere. After total consumption of the starting reagent (monitored by LC-MS), the mixture was diluted with $H_2O$ (5.00 mL) and extracted with DCM (2.00 mL x 2). The organic phase was concentrated under reduced pressure and the residue was placed under agitation with AcOEt (2.00 mL) at 25 °C for 10 minutes. 180 mg (51.0% yield) of the product of interest were obtained in the form of a yellow solid.

Compounds 62, 67, 69-71 and 74-75, as shown in Table 17, are obtained by the procedure described for Example 1, changing the corresponding intermediates II and III.

**EXAMPLE 6: (*S*)-6-(4-(ETOXY-$D_5$)PHENYL)-*N*-(2-(2-FLUORO-5-(METHOXY-$D_3$)PYRIDINE-3-IL)-2-HYDROXYETHYL)PYRAZINE-2-CARBOXAMIDE.**

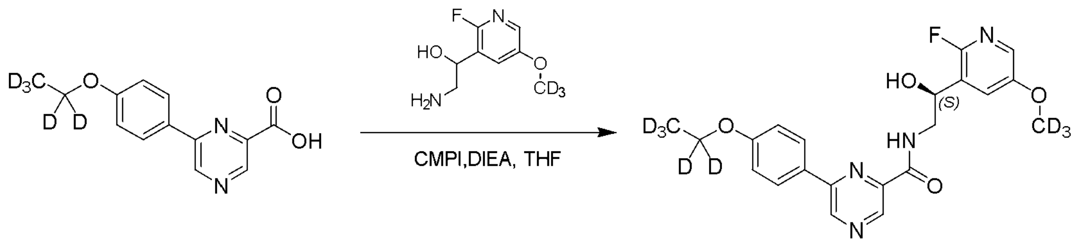


A round-bottomed flask containing 6-(4-(ethoxy-ds)phenyl)pyrazine-2-carboxylic acid (3.21 mmol), 2-amino-1-(2-fluoro-5-(methoxy-$d_3$)pyridine-3-yl)etan-1-ol (3.21 mmol), DIEA (4.81 mmol), CMPI (4.81 mmol) in THF (10 mL) was degasified and the mixture was kept under agitation at 25 °C for 12 hours. After total consumption of the starting reagent (monitored by LC-MS), the mixture was filtered and concentrated under reduced pressure. The residue was purified by preparative CCD (petroleum ether/AcOEt: 5/1). 450 mg (36.07% yield) of the product of interest was obtained in the form of a white solid.

Compounds 87, 88, 97 and 98, as shown in Table 17, are obtained by the procedure described for Example 1, changing the corresponding intermediates II and III

**EXAMPLE 7: (*S*)-N-(2-(3,5-DIMETHOXYPHENYL)-2-HYDROXYETHYL)-6-(4-(METHOXY-$D_3$)PHENYL)PYRAZINE-2-CARBOXAMIDE.**

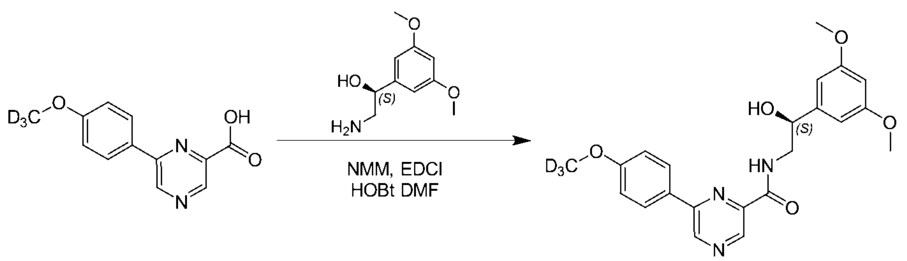

A round-bottomed flask containing 6-(4-(methoxy-$d_3$)phenyl)pyrazine-2-carboxylic acid (943 μmol) and (S)-2-amino-1-(3,5-dimethoxyphenyl)ethan-1-ol (1.13 mmol) in DMF (2.20 mL), was added NMM (1.88 mmol), EDCI (1.13 mmol), and HOBt (1.13 mmol) at 25 °C. Subsequently, the mixture was kept under agitation at 25 °C for 2 hours. After total consumption of the starting reagent (monitored by LC-MS), the mixture was purified by preparative HPLC (neutral condition). 304 mg (78.2% yield) of the product of interest were obtained in the form of a white solid.

Compounds 54, 55, 93, 100-101 and 111-112, as shown in Table 17, are obtained by the procedure described for Example 1, changing the corresponding intermediates II and III.

The General Formula I compound(s) of the present invention were obtained from the amide coupling between the Formula III intermediates and commercial amines or with the Formula II intermediates described above, synthesized according to the various methodologies described above and outlined in General Scheme 1, but not limited to these.

**TABLE 17. COMPOUNDS OBTAINED FROM SCHEME 1.**

| Compound Nº | Structure | $^1$H-NMR / MS (m/z) $[M+H]^+$ |
|---|---|---|
| 1 | 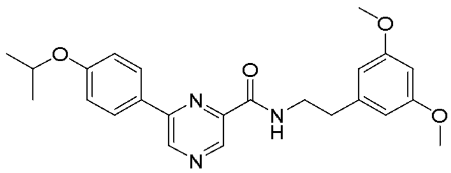 *N*-(3,5-dimethoxyphenethyl)-6-(4-isopropoxyphenyl)pyrazine-2-carboxamide | $^1$H-NMR, 400 MHz, DMSO-$d_6$ $\delta$ = 9.40 (s, 1H), 9.05 - 8.95 (m, 2H), 8.34 - 8.27 (m, 2H), 7.12 - 7.04 (m, 2H), 6.45 (d, J = 2.3 Hz, 2H), 6.36 (t, J = 2.3 Hz, 1H), 4.77 (quin, J = 6.0 Hz, 1H), 3.71 (s, 6H), 3.63 - 3.54 (m, 2H), 2.85 (t, J = 7.4 Hz, 2H), 1.32 (d, J = 6.1 Hz, 6H) . $[M+H]^+$: 422.5. |
| 2 | 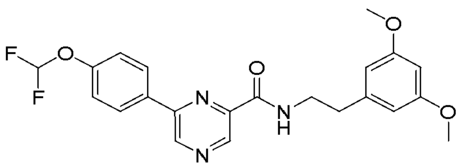 6-(4-(difluoromethoxy)phenyl)-*N*-(3,5-dimethoxyphenethyl) pyrazine-2-carboxamide | $^1$H-NMR, 400 MHz, DMSO-$d_6$ $\delta$ = 9.48 ( s, 1H), 9.11 (s, 1H), 9.09 - 9.04 (m, 1H), 8.48 - 8.42 (m, 2H), 7.62 - 7.22 (m, 3H), 6.45 (d, J = 2.3 Hz, 2H), 6.36 (t, J = 2.3 Hz, 1H), 3.71 (s, 6H), 3.64 - 3.53 (m, 2H), 2.85 (t, J = 7.4 Hz, 2H). $[M+H]^+$: 430.4. |
| 3 | 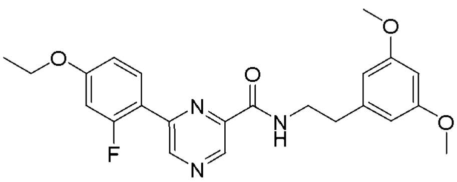 *N*-(3,5-dimethoxyphenethyl)-6-(4-ethoxy-2-fluorophenyl)pyrazine-2-carboxamide | $^1$H-NMR, 400 MHz, DMSO-$d_6$ $\delta$ = 9.18 (d, J = 2.3 Hz, 1H), 9.07 (s, 1H), 8.98 (t, J = 6.0 Hz, 1H), 8.25 (t, J = 9.1 Hz, 1H), 7.10 - 6.94 (m, 2H), 6.44 (d, J = 2.3 Hz, 2H), 6.35 (t, J = 2.3 Hz, 1H), 4.17 (q, J = 7.0 Hz, 2H), 3.70 (s, 6H), 3.64 - 3.52 (m, 2H), 2.84 (t, J = 7.4 Hz, 2H), 1.38 (t, J = 7.0 Hz, 3H). $[M+H]^+$: 426.5. |
| 4 | 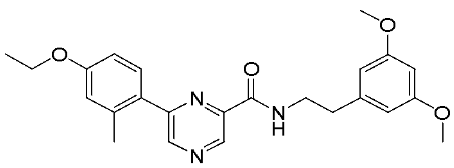 *N*-(3,5-dimethoxyphenethyl)-6-(4-ethoxy-2-methylphenyl)pyrazine-2-carboxamide | $^1$H-NMR, 400 MHz, DMSO-$d_6$ $\delta$ = 9.08 (s, 1H), 8.98 (s, 1H), 8.73 (br t, J = 5.8 Hz, 1H), 7.54 (d, J = 8.3 Hz, 1H), 6.98 - 6.88 (m, 2H), 6.40 (d, J = 2.1 Hz, 2H), 6.34 (t, J = 2.3 Hz, 1H), 4.11 (q, J = 6.9 Hz, 2H), 3.69 (s, 6H), 3.57 (q, J = 6.5 Hz, 2H), 2.81 (t, J = 7.3 Hz, 2H), 2.36 (s, 3H), 1.36 (t, J = 6.9 Hz, 3H). $[M+H]^+$: 422.4. |

(continued)

| Compound No | Structure | $^1$H-NMR / MS (m/z) [M+H]$^+$ |
|---|---|---|
| 5 | 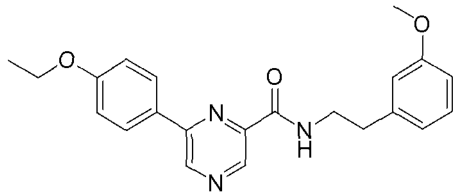<br>6-(4-ethoxyphenyl)-*N*-(3-methoxyphenethyl)pyrazine-2-carboxamide | $^1$H-NMR, 400 MHz, DMSO-$d_6$ $\delta$ = 9.41 (s, 1H), 9.08 - 8.96 (m, 2H), 8.33 (d, J = 8.8 Hz, 2H), 7.24 (t, J = 7.9 Hz, 1H), 7.10 (d, J = 8.8 Hz, 2H), 6.89 - 6.76 (m, 3H), 4.15 (q, J = 6.9 Hz, 2H), 3.72 (s, 3H), 3.63 - 3.53 (m, 2H), 2.89 (t, J = 7.4 Hz, 2H), 1.38 (t, J = 6.9 Hz, 3H).<br>[M+H]$^+$: 378.4. |
| 6 | 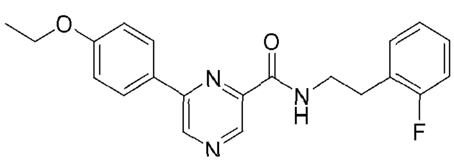<br>6-(4-ethoxyphenyl)-*N*-(2-fluorophenyl)pyrazine-2-carboxamide | $^1$H-NMR, 400 MHz, DMSO-$d_6$ $\delta$ = 9.41 (s, 1H), 9.13 (br t, J = 5.8 Hz, 1H), 9.01 (s, 1H), 8.34 (d, J = 8.8 Hz, 2H), 7.42 - 7.26 (m, 2H), 7.22 - 7.04 (m, 4H), 4.15 (q, J = 6.9 Hz, 2H), 3.65 - 3.50 (m, 2H), 2.96 (br t, J = 7.3 Hz, 2H), 1.38 (t, J = 7.0 Hz, 3H).<br>[M+H]$^+$: 366.4. |
| 7 | 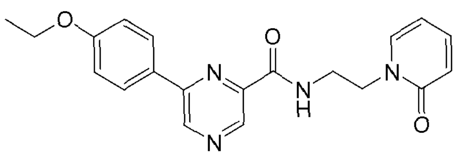<br>6-(4-ethoxyphenyl)-*N*-(2-(2-oxopyridinel(2*H*)-yl)ethyl)pyrazine-2-carboxamide | $^1$H-NMR, 400 MHz, DMSO-$d_6$ $\delta$ = 1.37 (t, J=6.94 Hz, 3 H) 3.66 (q, J=5.80 Hz, 2 H) 4.08 - 4.21 (m, 4 H) 6.15 (td, J=6.63, 1.13 Hz, 1 H) 6.41 (d, J=9.13 Hz, 1 H) 7.09 (d, J=8.88 Hz, 2 H) 7.40 (ddd, J=8.97, 6.72, 2.06 Hz, 1 H) 7.57 (dd, J=6.69, 1.81 Hz, 1 H) 8.36 (d, J=8.88 Hz, 2 H) 8.97 (s, 1 H) 9.18 (s, 1 H) 9.40 (s, 1 H).<br>[M+H]$^+$: 364.0. |
| 8 | 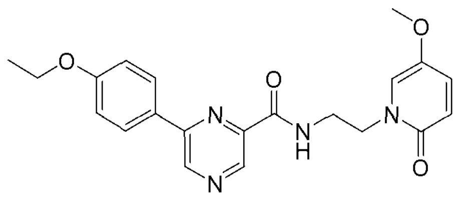<br>6-(4-ethoxyphenyl)-*N*-(2-(5-methoxy-2-oxopyridine-1(2*H*)-yl) ethyl)pyrazine-2-carboxamide | $^1$H-NMR, 400 MHz, DMSO-$d_6$ $\delta$ = 1.37 (t, J=7.00 Hz, 3 H) 3.48 (s, 3 H) 3.68 (q, J=5.75 Hz, 2 H) 4.09 - 4.19 (m, 4 H) 6.39 (d, J=9.63 Hz, 1 H) 7.06 - 7.13 (m, 2 H) 7.21 - 7.31 (m, 2 H) 8.33 - 8.40 (m, 2 H) 8.98 (s, 1 H) 9.19 (t, J=5.63 Hz, 1 H) 9.41 (s, 1 H).<br>[M+H]$^+$: 394.0. |
| 9 | 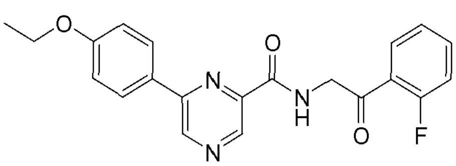<br>6-(4-ethoxyphenyl)-*N*-(2-(2-fluorophenyl)-2-oxoethyl)pyrazine-2-carboxamide | $^1$H-NMR, 400 MHz, DMSO-$d_6$ $\delta$ = 1.37 (t, J=6.94 Hz, 3 H) 4.14 (q, J=6.96 Hz, 2 H) 4.77 (dd, J=5.69, 2.56 Hz, 2 H) 7.08 - 7.16 (m, 2 H) 7.35 - 7.48 (m, 2 H) 7.68 - 7.81 (m, 1 H) 7.93 (td, J=7.57, 1.75 Hz, 1 H) 8.31 - 8.41 (m, 2 H) 9.05 (s, 1 H) 9.34 (t, J=5.69 Hz, 1 H) 9.46 (s, 1 H).<br>[M+H]$^+$: 379.0. |

(continued)

| Compound No | Structure | $^1$H-NMR / MS (m/z) [M+H]$^+$ |
|---|---|---|
| 10 | 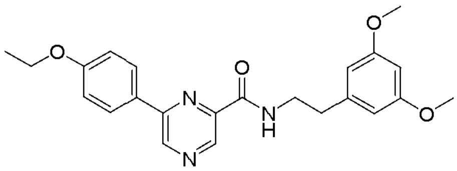 *N*-(3,5-dimethoxyphenethyl)-6-(4-ethoxyphenyl)pyrazine-2-carboxamide | $^1$H-NMR, 400 MHz, DMSO-$d_6$ $\delta$ = 10.24 (s, 1H), 9.51 (s, 1H), 9.11 (s, 1H), 8.45 (d, J = 8.6 Hz, 2H), 7.65 (d, J = 8.6 Hz, 2H), 7.32 - 7.08 (m, 3H), 4.15 (s, 2H), 3.26 (br t, J = 6.0 Hz, 2H), 2.99 (br t, J = 5.7 Hz, 2H). [M+H]$^+$: 408.1. |
| 11 | 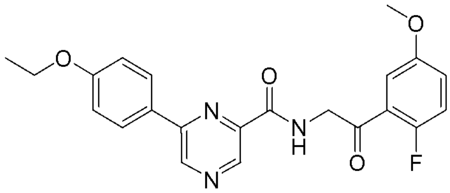 6-(4-ethoxyphenyl)-*N*-(2-(2-fluoro-5-methoxyphenyl)-2-oxoethyl)pyrazine-2-carboxamide | $^1$H-NMR, 400 MHz, DMSO-$d_6$ $\delta$ = 1.37 (t, J=7.00 Hz, 3 H) 3.81 (s, 3 H) 4.14 (q, J=6.88 Hz, 2 H) 4.75 (dd, J=5.69, 2.69 Hz, 2 H) 7.12 (d, J=8.88 Hz, 2 H) 7.23 - 7.43 (m, 3 H) 8.35 (d, J=8.88 Hz, 2 H) 9.05 (s, 1 H) 9.33 (t, J=5.69 Hz, 1 H) 9.46 (s, 1 H). [M+H]$^+$: 409.0. |
| 12 | 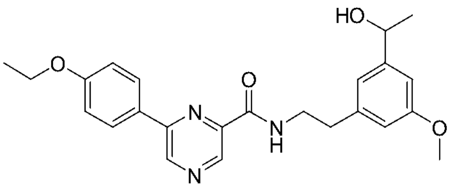 6-(4-ethoxyphenyl)-*N*-(3-(1-hydroxyethyl)-5-methoxyphenethyl)pyrazine-2-carboxamide | $^1$H-NMR, 400 MHz, DMSO-$d_6$ $\delta$ = 9.40 (s, 1H), 9.07 - 8.99 (m, 2H), 8.32 (d, J = 8.8 Hz, 2H), 7.09 (d, J = 8.9 Hz, 2H), 6.82 (s, 1H), 6.76 (s, 1H), 6.69 (s, 1H), 5.10 (d, J = 4.3 Hz, 1H), 4.64 (dd, J = 4.4, 6.3 Hz, 1H), 4.14 (q, J = 6.9 Hz, 2H), 3.72 (s, 3H), 3.62 - 3.53 (m, 2H), 2.87 (t, J = 7.6 Hz, 2H), 1.38 (t, J = 6.9 Hz, 3H), 1.26 (d, J = 6.4 Hz, 3H). [M+Na]$^+$: 444.2. |
| 13 | 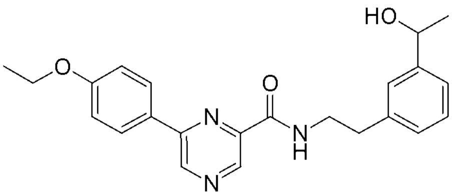 6-(4-ethoxyphenyl)-*N*-(3-(1-hydroxyethyl)phenethyl)pyrazine-2-carboxamide | $^1$H-NMR, 400 MHz, Methanol-$d_4$ $\delta$ = 9.21 (s, 1H), 9.03 (s, 1H), 8.19 - 8.07 (m, 2H), 7.38 - 7.13 (m, 4H), 7.10 - 7.03 (m, 2H), 4.83 - 4.73 (m, 1H), 4.19 - 4.07 (m, 2H), 3.72 (t, J = 7.3 Hz, 2H), 2.98 (t, J = 7.3 Hz, 2H), 1.49 - 1.41 (m, 3H), 1.37 (d, J = 6.5 Hz, 3H). [M+Na]$^+$: 414.5. |
| 14 | 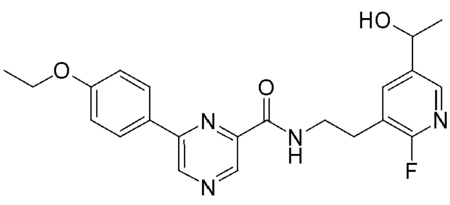 6-(4-ethoxyphenyl)-*N*-(2-(2-fluoro-5-(1-hydroxyethyl)pyridine-3-yl)ethyl)pyrazine-2-carboxamide | $^1$H-NMR, 400 MHz, DMSO-$d_6$ $\delta$ = 9.39 (s, 1H), 9.14 (br t, J = 6.1 Hz, 1H), 8.98 (s, 1H), 8.36 - 8.29 (m, 2H), 8.01 (s, 1H), 7.78 (dd, J = 2.3, 9.8 Hz, 1H), 7.13 - 7.04 (m, 2H), 5.30 (d, J = 4.4 Hz, 1H), 4.79 - 4.66 (m, 1H), 4.14 (q, J = 7.0 Hz, 2H), 3.61 (q, J = 6.9 Hz, 2H), 2.94 (t, J = 7.1 Hz, 2H), 1.37 (t, J = 6.9 Hz, 3H), 1.22 (d, J = 6.4 Hz, 3H). [M+H]$^+$: 411.3. |

(continued)

| Compound No | Structure | $^1$H-NMR / MS (m/z) [M+H]$^+$ |
|---|---|---|
| 15 | 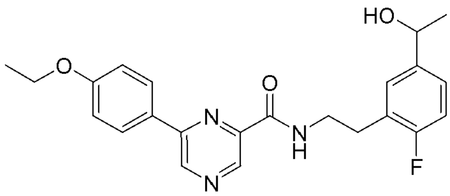 6-(4-ethoxyphenyl)-*N*-(2-fluoro-5-(1-hydroxyethyl)phenethyl) pyrazine-2-carboxamide | $^1$H-NMR, 400 MHz, DMSO-$d_6$ $\delta$ = 9.41 (s, 1H), 9.13 (t, J = 6.0 Hz, 1H), 9.01 (s, 1H), 8.39 - 8.29 (m, 2H), 7.30 (dd, J = 2.0, 7.5 Hz, 1H), 7.22 (ddd, J = 2.2, 5.4, 8.1 Hz, 1H), 7.14 - 7.06 (m, 3H), 5.14 (d, J = 4.0 Hz, 1H), 4.67 (br dd, J = 4.1, 6.2 Hz, 1H), 4.15 (q, J = 7.0 Hz, 2H), 3.64 - 3.53 (m, 2H), 2.94 (t, J = 7.4 Hz, 2H), 1.38 (t, J = 7.0 Hz, 3H), 1.23 (d, J = 6.4 Hz, 3H). [M+Na]$^+$: 432.2. |
| 16 | 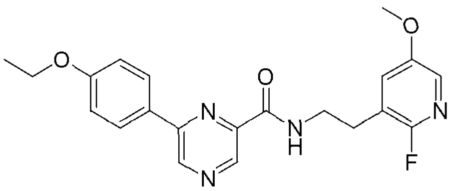 6-(4-ethoxyphenyl)-*N*-(2-(2-fluoro-5-methoxypyridine-3-yl) ethyl)pyrazine-2-carboxamide | $^1$H-NMR, 400 MHz, DMSO-$d_6$ $\delta$ = 9.40 (s, 1H), 9.12 (br t, J = 6.2 Hz, 1H), 8.99 (s, 1H), 8.35 - 8.29 (m, 2H), 7.76 - 7.72 (m, 1H), 7.49 (dd, J = 3.1, 8.1 Hz, 1H), 7.13 - 7.05 (m, 2H), 4.14 (q, J = 6.9 Hz, 2H), 3.76 (s, 3H), 3.63 (q, J = 6.7 Hz, 2H), 2.92 (t, J = 6.9 Hz, 2H), 1.37 (t, J = 6.9 Hz, 3H). [M+H]$^+$: 397.5. |
| 17 | 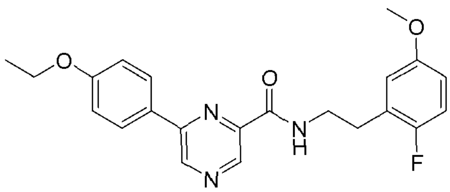 6-(4-ethoxyphenyl)-*N*-(2-fluoro-5-methoxyphenethyl)pyrazine-2-carboxamide | $^1$H-NMR, 400 MHz, DMSO-$d_6$ $\delta$ = 1.37 (t, J=7.00 Hz, 3 H) 2.91 (br t, J=7.32 Hz, 2 H) 3.54 - 3.63 (m, 2 H) 3.68 (s, 3 H) 4.14 (q, J=6.88 Hz, 2 H) 6.80 (dt, J=8.91, 3.61 Hz, 1 H) 6.85 - 6.91 (m, 1 H) 7.02 - 7.13 (m, 3 H) 8.29 - 8.35 (m, 2 H) 9.00 (s, 1 H) 9.08 (br t, J=5.82 Hz, 1 H) 9.40 (s, 1 H). [M+H]$^+$: 396.4. |
| 18 | 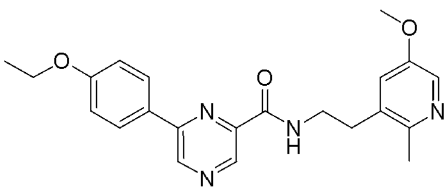 6-(4-ethoxyphenyl)-*N*-(2-(5-methoxy-2-methylpyridine-3-yl) ethyl)pyrazine-2-carboxamide | $^1$H-NMR, 400 MHz, DMSO-$d_6$ $\delta$ = 1.37 (t, J=6.94 Hz, 3 H) 2.46 (s, 3 H) 2.91 (t, J=7.38 Hz, 2 H) 3.52 - 3.64 (m, 2 H) 3.74 (s, 3 H) 4.14 (q, J=6.96 Hz, 2 H) 7.09 (d, J=8.88 Hz, 2 H) 7.18 (d, J=2.88 Hz, 1 H) 8.02 (d, J=2.88 Hz, 1 H) 8.33 (d, J=8.88 Hz, 2 H) 9.01 (s, 1 H) 9.12 (br t, J=5.94 Hz, 1 H) 9.40 (s, 1 H). [M+H]$^+$: 393.4. |

(continued)

| Compound No | Structure | $^1$H-NMR / MS (m/z) [M+H]$^+$ |
|---|---|---|
| 19 | 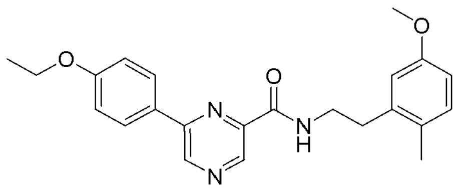 6-(4-ethoxyphenyl)-*N*-(5-methoxy-2-methylphenetyl)pyrazine-2-carboxamide | $^1$H-NMR, 400 MHz, DMSO-$d_6$ $\delta$ = 1.38 (t, J=7.00 Hz, 3 H) 2.28 (s, 3 H) 2.82 - 2.90 (m, 2 H) 3.49 - 3.58 (m, 2 H) 3.68 (s, 3 H) 4.14 (q, J=6.96 Hz, 2 H) 6.70 (dd, J=8.25, 2.75 Hz, 1 H) 6.77 (d, J=2.75 Hz, 1 H) 7.04 - 7.13 (m, 3 H) 8.31 - 8.36 (m, 2 H) 9.03 (s, 1 H) 9.10 (t, J=6.13 Hz, 1 H) 9.41 (s, 1 H). [M+H]$^+$: 392.1. |
| 20 | 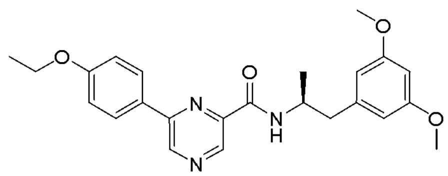 (*S*)-*N*-(1-(3,5-dimethoxyphenyl)propan-2-yl)-6-(4-ethoxyphenyl)pyrazine-2-carboxamide | $^1$H-NMR, 400 MHz, DMSO-$d_6$ $\delta$ = 9.38 (s, 1H), 8.97 (s, 1H), 8.66 (br d, J = 8.6 Hz, 1H), 8.33 - 8.31 (m, 1H), 8.31 - 8.29 (m, 1H), 7.12 - 7.10 (m, 1H), 7.10 - 7.08 (m, 1H), 6.45 (d, J = 2.3 Hz, 2H), 6.30 (t, J = 2.3 Hz, 1H), 4.34 - 4.25 (m, 1H), 4.14 (q, J = 7.0 Hz, 2H), 3.65 (s, 6H), 2.95 (dd, J = 8.0, 13.4 Hz, 1H), 2.78 (dd, J = 6.2, 13.3 Hz, 1H), 1.38 (t, J = 6.9 Hz, 3H), 1.24 (d, J = 6.6 Hz, 3H). [M+H]$^+$: 422.4. |
| 21 | 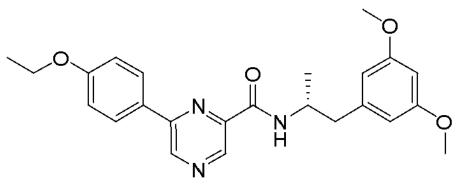 (*R*)-*N*-(1-(3,5-dimethoxyphenyl)propan-2-yl)-6-(4-ethoxyphenyl)pyrazine-2-carboxamide | $^1$H-NMR, 400 MHz, DMSO-$d_6$ $\delta$ = 9.38 (s, 1H), 8.97 (s, 1H), 8.66 (d, J = 8.6 Hz, 1H), 8.33 - 8.31 (m, 1H), 8.30 (s, 1H), 7.11 (s, 1H), 7.08 (s, 1H), 6.45 (d, J = 2.1 Hz, 2H), 6.30 (t, J = 2.2 Hz, 1H), 4.35 - 4.24 (m, 1H) , 4.14 (q, J = 7.0 Hz, 2H), 3.65 (s, 6H), 2.95 (dd, J = 8.1, 13.3 Hz, 1H), 2.84 - 2.72 (m, 1H), 1.38 (t, J = 6.9 Hz, 3H), 1.25 (d, J = 6.5 Hz, 3H). [M+H]$^+$: 422.4. |
| 22 | 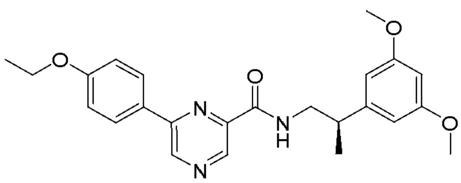 (*R*)-*N*-(2-(3,5-dimethoxyphenyl)propyl)-6-(4-ethoxyphenyl) pyrazine-2-carboxamide | $^1$H-NMR, 400 MHz, DMSO-$d_6$ $\delta$ = 9.39 (s, 1H), 9.00 (s, 1H), 8.77 (t, J = 6.0 Hz, 1H), 8.24 (d, J = 8.8 Hz, 2H), 7.08 (d, J = 8.8 Hz, 2H), 6.47 (d, J = 2.1 Hz, 2H), 6.37 (t, J = 2.1 Hz, 1H), 4.14 (q, J = 7.0 Hz, 2H), 3.71 (s, 6H), 3.61 - 3.51 (m, 1H), 3.50 - 3.41 (m, 1H), 3.08 (q, J = 7.2 Hz, 1H), 1.37 (t, J = 7.0 Hz, 3H), 1.23 (d, J = 6.9 Hz, 3H). [M+H]$^+$: 422.2. |

(continued)

| Compound No | Structure | $^1$H-NMR / MS (m/z) $[M+H]^+$ |
|---|---|---|
| 23 | 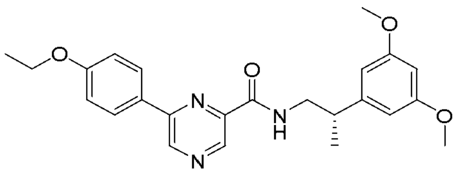 (*S*)-*N*-(2-(3,5-dimethoxyphenyl)propyl)-6-(4-ethoxyphenyl)pyrazine-2-carboxamide | $^1$H-NMR, 400 MHz, DMSO-$d_6$ $\delta$ = 9.39 (s, 1H), 9.00 (s, 1H), 8.78 (br t, J = 6.0 Hz, 1H), 8.25 (d, J = 8.8 Hz, 2H), 7.08 (d, J = 8.8 Hz, 2H), 6.47 (d, J = 2.1 Hz, 2H), 6.37 (t, J = 2.1 Hz, 1H), 4.14 (q, J = 7.0 Hz, 2H), 3.71 (s, 6H), 3.62 - 3.51 (m, 1H), 3.51 - 3.41 (m, 1H), 3.08 (q, J = 7.2 Hz, 1H), 1.38 (t, J = 6.9 Hz, 3H), 1.23 (d, J = 6.9 Hz, 3H). $[M+H]^+$: 422.2. |
| 24 | 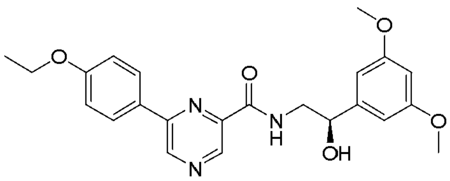 (*R*)-*N*-(2-(3,5-dimethoxyphenyl)-2-hydroxyethyl)-6-(4-ethoxyphenyl)pyrazine-2-carboxamide | $^1$H-NMR, 400 MHz, DMSO-$d_6$ $\delta$ = 9.43 (s, 1H), 9.04 (s, 1H), 8.81 (br t, J = 5.8 Hz, 1H), 8.29 (d, J = 8.9 Hz, 2H), 7.11 (d, J = 8.9 Hz, 2H), 6.58 (d, J = 2.1 Hz, 2H), 6.40 (t, J = 2.2 Hz, 1H), 5.64 (br s, 1H), 4.80 (dd, J = 4.4, 7.6 Hz, 1H), 4.15 (q, J = 6.9 Hz, 2H), 3.72 (s, 6H), 3.63 - 3.55 (m, 1H), 3.49 - 3.42 (m, 1H), 1.39 (t, J = 7.0 Hz, 3H). $[M+H]^+$: 424.1. |
| 25 | 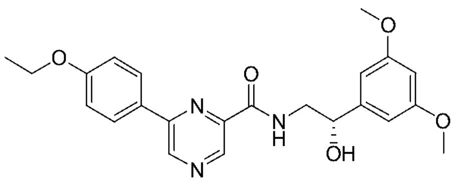 (*S*)-*N*-(2-(3,5-dimethoxyphenyl)-2-hydroxyethyl)-6-(4-ethoxyphenyl)pyrazine-2-carboxamide | $^1$H-NMR, 400 MHz, DMSO-$d_6$ $\delta$ = 9.43 (s, 1H), 9.04 (s, 1H), 8.81 (br t, J = 5.8 Hz, 1H), 8.29 (d, J = 8.8 Hz, 2H), 7.11 (d, J = 9.0 Hz, 2H), 6.58 (d, J = 2.3 Hz, 2H), 6.41 (t, J = 2.3 Hz, 1H), 5.64 (d, J = 4.3 Hz, 1H), 4.84 - 4.76 (m, 1H), 4.15 (q, J = 6.9 Hz, 2H), 3.72 (s, 6H), 3.62 - 3.55 (m, 1H), 3.50 - 3.43 (m, 1H), 1.39 (t, J = 6.9 Hz, 3H). $[M+H]^+$: 424.1. |
| 26 | 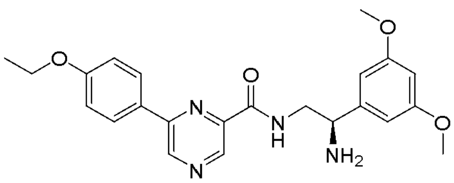 (*R*)-*N*-(2-amino-2-(3,5-dimethoxyphenyl)ethyl)-6-(4-ethoxyphenyl)pyrazine-2-carboxamide | $^1$H-NMR, 400 MHz, Chloroform-d $\delta$ = 9.25 (s, 1H), 9.13 (s, 1H), 8.19 (br t, J = 5.1 Hz, 1H), 8.00 - 7.91 (m, 2H), 7.09 - 7.00 (m, 2H), 6.61 (d, J = 2.3 Hz, 2H), 6.44 (t, J = 2.3 Hz, 1H), 4.22 (br t, J = 5.7 Hz, 1H), 4.14 (q, J = 7.0 Hz, 2H), 3.78 (s, 6H), 3.76 - 3.59 (m, 2H), 1.49 (t, J = 7.0 Hz, 3H). $[M+H]^+$: 423.2. |

(continued)

| Compound No | Structure | $^1$H-NMR / MS (m/z) [M+H]$^+$ |
|---|---|---|
| 27 | 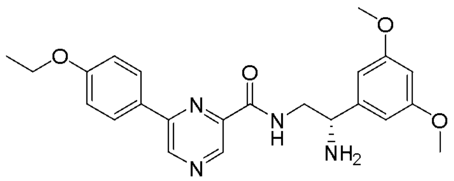<br>(*S*)-*N*-(2-amino-2-(3,5-dimethoxyphenyl)ethyl)-6-(4-ethoxy-phenyl)pyrazine-2-carboxamide | $^1$H-NMR, 400 MHz, Chloroform-d $\delta$ = 9.26 (s, 1H), 9.14 (s, 1H), 8.20 (br t, J = 6.0 Hz, 1H), 7.95 (d, J = 8.8 Hz, 2H), 7.06 (d, J = 8.8 Hz, 2H), 6.62 (d, J = 2.1 Hz, 2H), 6.45 (t, J = 2.1 Hz, 1H), 4.23 (t, J = 6.4 Hz, 1H), 4.16 (q, J = 6.9 Hz, 2H), 3.80 (s, 6H), 3.77 - 3.62 (m, 2H), 1.52 - 1.49 (m, 3H).<br>[M+H]$^+$: 423.2. |
| 28 | 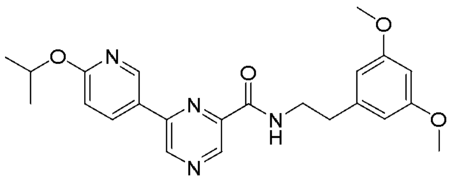<br>*N*-(3,5-dimethoxyphenethyl)-6-(6-isopropoxypyridin-3-yl)pyra-zine-2-carboxamide | $^1$H-NMR, 400 MHz, DMSO-$d_6$ $\delta$ = 9.47 (s, 1H), 9.23 (d, J = 2.1 Hz, 1H), 9.13 (t, J = 5.9 Hz, 1H), 9.08 (s, 1H), 8.67 (dd, J = 2.5, 8.8 Hz, 1H), 6.94 (d, J = 8.5 Hz, 1H), 6.44 (d, J = 2.3 Hz, 2H), 6.35 (t, J = 2.3 Hz, 1H), 5.36 (td, J = 6.1, 12.4 Hz, 1H), 3.71 (s, 6H), 3.63 - 3.52 (m, 2H), 2.85 (s, 2H), 1.36 (d, J = 6.3 Hz, 6H).<br>[M+H]$^+$: 423.5. |
| 29 | 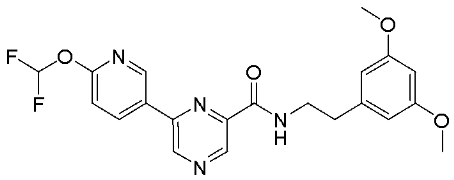<br>6-(6-(difluoromethoxy)pyridine-3-yl)-*N*-(3,5-dimethoxyphe-nethyl)pyrazine-2-carboxamide | $^1$H-NMR, 400 MHz, DMSO-$d_6$ $\delta$ = 9.56 (s, 1H), 9.35 - 9.27 (m, 1H), 9.24 - 9.07 (m, 2H), 8.92 (br d, J = 8.8 Hz, 1H), 8.06 - 7.59 (m, 1H), 7.38 - 7.24 (m, 1H), 6.52 - 6.31 (m, 3H), 3.70 (s, 6H), 3.62 - 3.55 (m, 2H), 2.90 - 2.80 (m, 2H).<br>[M+H]$^+$: 431,4. |
| 30 | 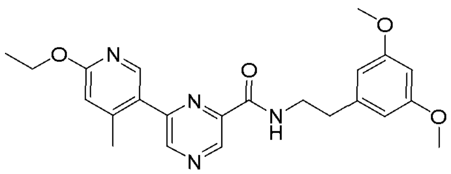<br>*N*-(3,5-dimethoxyphenethyl)-6-(6-ethoxy-4-methylpyridine-3-yl)pyrazine-2-carboxamide | $^1$H-NMR, 400 MHz, DMSO-$d_6$ $\delta$ = 9.14 (s, 1H), 9.07 (s, 1H), 8.80 (t, J = 5.9 Hz, 1H), 8.40 (s, 1H), 6.84 (s, 1H), 6.40 (d, J = 2.3 Hz, 2H), 6.36 - 6.29 (m, 1H), 4.38 (d, J = 7.0 Hz, 2H), 3.69 (s, 6H), 3.57 (q, J = 6.7 Hz, 2H), 2.81 (t, J = 7.2 Hz, 2H), 2.38 (s, 3H), 1.35 (t, J = 7.0 Hz, 3H).<br>[M+H]$^+$: 423.4. |
| 31 | 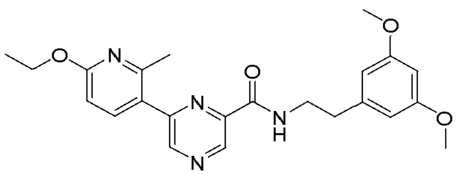<br>*N*-(3,5-dimethoxyphenethyl)-6-(6-ethoxy-2-methylpyridine-3-yl)pyrazine-2-carboxamide | $^1$H-NMR, 400 MHz, DMSO-$d_6$ $\delta$ = 9.11 (s, 1H), 9.06 (s, 1H), 8.80 (s, 1H), 7.98 (d, J = 8.4 Hz, 1H), 6.80 (d, J = 8.5 Hz, 1H), 6.41 (d, J = 2.3 Hz, 2H), 6.34 (s, 1H), 4.39 (q, J = 7.0 Hz, 2H), 3.69 (s, 6H), 3.57 (q, J = 6.8 Hz, 2H), 2.81 (t, J = 7.3 Hz, 2H), 2.53 (br s, 3H), 1.36 (t, J = 7.1 Hz, 3H).<br>[M+H]$^+$: 423.5. |

(continued)

| Compound №  | Structure | $^1$H-NMR / MS (m/z) $[M+H]^+$ |
|---|---|---|
| 32 | 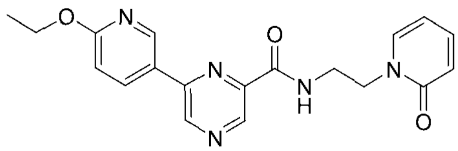 6-(6-ethoxypyridine-3-yl)-*N*-(2-(2-oxopyridine-1(2H)-yl)ethyl) pyrazine-2-carboxamide | $^1$H-NMR, 400 MHz, DMSO-$d_6$ $\delta$ = 1.37 (t, J=7.00 Hz, 3 H) 3.66 (q, J=5.88 Hz, 2 H) 4.14 (t, J=5.94 Hz, 2 H) 4.41 (q, J=7.09 Hz, 2 H) 6.14 (td, J=6.66, 1.31 Hz, 1 H) 6.40 (d, J=8.63 Hz, 1 H) 6.99 (d, J=9.13 Hz, 1 H) 7.39 (ddd, J=8.97, 6.72, 2.06 Hz, 1 H) 7.56 (dd, J=6.75, 1.75 Hz, 1 H) 8.71 (dd, J=8.75, 2.50 Hz, 1 H) 9.03 (s, 1 H) 9.22 (d, J=2.00 Hz, 1 H) 9.26 (br t, J=5.63 Hz, 1 H) 9.47 (s, 1 H).<br>$[M+H]^+$: 365,0. |
| 33 | 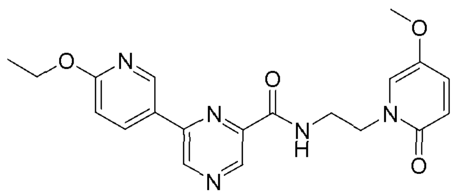 b-(b-ethoxypyridine-3-yl)-*N*-(2-(5-methoxy-2-oxopyridine-1(2*H*)-yl)ethyl)pyrazine-2-carboxamide | $^1$H-NMR, 400 MHz, DMSO-$d_6$ $\delta$ = 1.37 (t, J=7.07 Hz, 3 H) 3.47 (s, 3 H) 3.67 (br d, J=5.88 Hz, 2 H) 4.13 (t, J=5.82 Hz, 2 H) 4.41 (q, J=7.09 Hz, 2 H) 6.38 (d, J=9.63 Hz, 1 H) 6.98 (d, J=8.75 Hz, 1 H) 7.21 - 7.29 (m, 2 H) 8.73 (dd, J=8.76, 2.50 Hz, 1 H) 9.04 (s, 1 H) 9.24 (br d, J=2.00 Hz, 2 H) 9.48 (s, 1 H).<br>$[M+H]^+$: 395.0. |
| 34 | 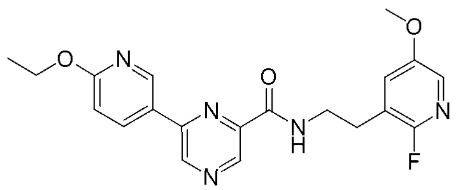 6-(6-ethoxypyridine-3-yl)-*N*-(2-(2-fluoro-5-methoxypyridine-3-yl)ethyl)pyrazine-2-carboxamide | $^1$H-NMR, 400 MHz, DMSO-$d_6$ $\delta$ = 9.47 (s, 1H), 9.25 - 9.17 (m, 2H), 9.05 (s, 1H), 8.68 (dd, J = 2.6, 8.7 Hz, 1H), 7.76 - 7.70 (m, 1H), 7.49 (dd, J = 3.0, 8.0 Hz, 1H), 6.98 (d, J = 8.8 Hz, 1H), 4.41 (q, J = 7.0 Hz, 2H), 3.76 (s, 3H), 3.62 (q, J = 6.7 Hz, 2H), 2.92 (t, J = 6.9 Hz, 2H), 1.37 (t, J = 7.1 Hz, 3H).<br>$[M+H]^+$: 398.4. |
| 35 | 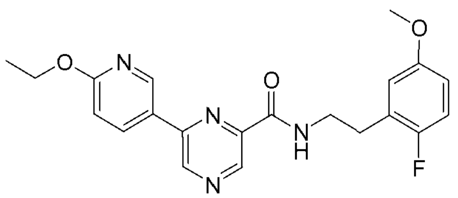 6-(6-ethoxypyridine-3-yl)-*N*-(2-fluoro-5-methoxyphenethyl) pyrazine-2-carboxamide | $^1$H-NMR, 400 MHz, DMSO-$d_6$ $\delta$ = 1.36 (t, J=7.00 Hz, 3 H) 2.91 (t, J=7.32 Hz, 2 H) 3.53 - 3.61 (m, 2 H) 3.68 (s, 3 H) 4.41 (q, J=7.00 Hz, 2 H) 6.79 (dt, J=8.88, 3.63 Hz, 1 H) 6.88 (dd, J=6.07, 3.19 Hz, 1 H) 6.98 (d, J=8.76 Hz, 1 H) 7.07 (t, J=9.26 Hz, 1 H) 8.68 (dd, J=8.69, 2.56 Hz, 1 H) 9.06 (s, 1 H) 9.14 - 9.23 (m, 2 H) 9.46 (s, 1 H).<br>$[M+H]^+$: 397.4. |

(continued)

| Compound №. | Structure | $^1$H-NMR / MS (m/z) [M+H]$^+$ |
|---|---|---|
| 36 | 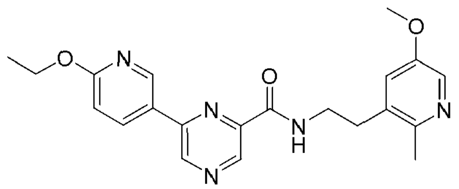 6-(6-ethoxypyridine-3-yl)-*N*-(2-(5-methoxy-2-methylpyridine-3-yl)ethyl)pyrazine-2-carboxamide | $^1$H-NMR, 400 MHz, DMSO-$d_6$ $\delta$ = 1.37 (t, J=7.07 Hz, 3 H) 2.65 (s, 3 H) 2.96 - 3.11 (m, 2 H) 3.66 (q, J=6.67 Hz, 2 H) 3.86 (s, 3 H) 4.36 - 4.47 (m, 2 H) 7.00 (d, J=8.76 Hz, 1 H) 7.97 (d, J=2.00 Hz, 1 H) 8.39 (d, J=2.63 Hz, 1 H) 8.66 (dd, J=8.69, 2.56 Hz, 1 H) 9.03 (s, 1 H) 9.15 - 9.25 (m, 2 H) 9.48 (s, 1 H). [M+H]$^+$: 394,4. |
| 37 | 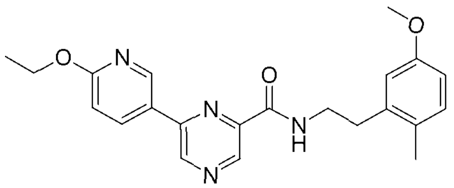 6-(6-ethoxypyridine-3-yl)-*N*-(5-methoxy-2-methylphenethyl) pyrazine-2-carboxamide | $^1$H-NMR, 400 MHz, DMSO-$d_6$ $\delta$ = 1.37 (t, J=7.07 Hz, 3 H) 2.27 (s, 3 H) 2.82 - 2.89 (m, 2 H) 3.48 - 3.57 (m, 2 H) 3.68 (s, 3 H) 4.41 (q, J=7.09 Hz, 2 H) 6.69 (dd, J=8.19, 2.69 Hz, 1 H) 6.76 (d, J=2.63 Hz, 1 H) 6.99 (d, J=8.76 Hz, 1 H) 7.07 (d, J=8.38 Hz, 1 H) 8.69 (dd, J=8.76, 2.50 Hz, 1 H) 9.08 (s, 1 H) 9.17 - 9.25 (m, 2 H) 9.47 (s, 1 H). [M+H]$^+$: 393.4. |
| 38 | 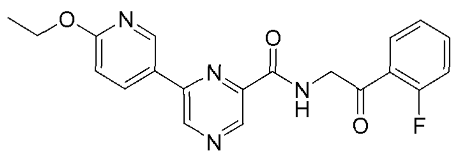 6-(6-ethoxypyridine-3-yl)-*N*-(2-(2-fluorophenyl)-2-oxoethyl) pyrazine-2-carboxamide | $^1$H-NMR, 400 MHz, DMSO-$d_6$ $\delta$ = 1.37 (t, J=7.00 Hz, 3 H) 4.42 (q, J=7.09 Hz, 2 H) 4.76 (dd, J=5.75, 2.63 Hz, 2 H) 7.01 (d, J=8.76 Hz, 1 H) 7.36 - 7.47 (m, 2 H) 7.69 - 7.78 (m, 1 H) 7.93 (td, J=7.57, 1.75 Hz, 1 H) 8.71 (dd, J=8.75, 2.50 Hz, 1 H) 9.10 (s, 1 H) 9.23 (d, J=2.38 Hz, 1 H) 9.43 (t, J=5.75 Hz, 1 H) 9.52 (s, 1 H). [M+H]$^+$: 380.0. |
| 39 | 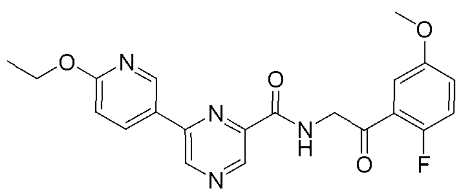 6-(6-ethoxypyridine-3-yl)-*N*-(2-(2-fluoro-5-methoxyphenyl)-2-oxoethyl)pyrazine-2-carboxamide | $^1$H-NMR, 400 MHz, DMSO-$d_6$ $\delta$ = 1.37 (t, J=7.07 Hz, 3 H) 3.47 (s, 3 H) 3.67 (br d, J=5.88 Hz, 2 H) 4.13 (t, J=5.82 Hz, 2 H) 4.41 (q, J=7.09 Hz, 2 H) 6.38 (d, J=9.63 Hz, 1 H) 6.98 (d, J=8.75 Hz, 1 H) 7.21 - 7.29 (m, 2 H) 8.73 (dd, J=8.76, 2.50 Hz, 1 H) 9.04 (s, 1 H) 9.24 (br d, J=2.00 Hz, 2 H) 9.48 (s, 1 H). [M+H]$^+$: 395.0. |

(continued)

| Compound No | Structure | $^1$H-NMR / MS (m/z) [M+H]$^+$ |
|---|---|---|
| 40 | 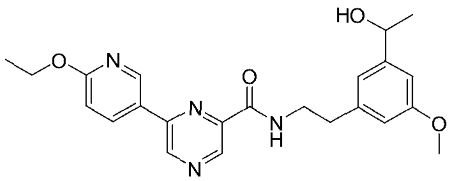 6-(6-ethoxypyridine-3-yl)-*N*-(3-(1-hydroxyethyl)-5-methoxyphenethyl)pyrazine-2-carboxamide | $^1$H-NMR, 400 MHz, DMSO-$d_6$ $\delta$ = 9.47 (s, 1H), 9.22 (d, J = 2.4 Hz, 1H), 9.15 (br t, J = 6.0 Hz, 1H), 9.07 (s, 1H), 8.68 (dd, J = 2.5, 8.8 Hz, 1H), 6.98 (d, J = 8.8 Hz, 1H), 6.82 (s, 1H), 6.76 (s, 1H), 6.68 (s, 1H), 5.09 (d, J = 4.3 Hz, 1H), 4.69 - 4.59 (m, 1H), 4.41 (q, J = 7.0 Hz, 2H), 3.71 (s, 3H), 3.62 - 3.50 (m, 2H), 2.86 (br t, J = 7.6 Hz, 2H), 1.37 (t, J = 7.1 Hz, 3H), 1.25 (d, J = 6.5 Hz, 3H) . [M+H]$^+$: 423.4. |
| 41 | 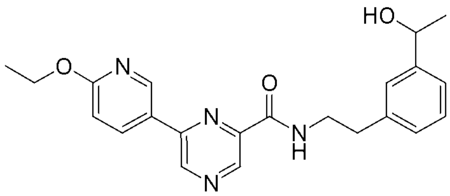 6-(6-ethoxypyridine-3-yl)-*N*-(3-(1-hydroxyethyl)phenethyl)pyrazine-2-carboxamide | $^1$H-NMR, 400 MHz, Methanol-$d_4$ $\delta$ = 9.27 (s, 1H), 9.11 (s, 1H), 8.95 (d, J = 2.5 Hz, 1H), 8.49 (dd, J = 2.6, 8.8 Hz, 1H), 7.37 - 7.10 (m, 4H), 6.93 (d, J = 8.8 Hz, 1H), 4.79 (q, J = 6.5 Hz, 1H), 4.44 (q, J = 7.0 Hz, 2H), 3.71 (t, J = 7.3 Hz, 2H), 2.98 (t, J = 7.3 Hz, 2H), 1.43 (t, J = 7.1 Hz, 3H), 1.37 (d, J = 6.5 Hz, 3H). [M+H]$^+$: 393.4. |
| 42 | 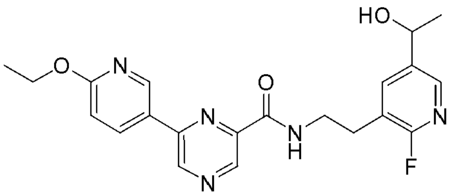 6-(6-ethoxypyridine-3-yl)-*N*-(2-(2-fluoro-5-(1-hydroxyethyl)pyridin-3-yl)ethyl)pyrazine-2-carboxamide | $^1$H-NMR, 400 MHz, DMSO-$d_6$ $\delta$ = 9.47 (s, 1H), 9.29 - 9.15 (m, 2H), 9.06 (s, 1H), 8.70 (dd, J = 2.5, 8.8 Hz, 1H), 7.29 (dd, J = 2.1, 7.4 Hz, 1H), 7.21 (ddd, J = 2.2, 5.4, 8.1 Hz, 1H), 7.08 (dd, J = 8.6, 9.8 Hz, 1H), 6.98 (d, J = 8.8 Hz, 1H), 5.12 (d, J = 4.1 Hz, 1H), 4.73 - 4.57 (m, 1H), 4.41 (q, J = 7.0 Hz, 2H), 3.64 - 3.48 (m, 2H), 2.93 (br t, J = 7.5 Hz, 2H), 1.37 (t, J = 7.1 Hz, 3H), 1.22 (d, J = 6.5 Hz, 3H). [M+H]$^+$: 411.4. |
| 43 | 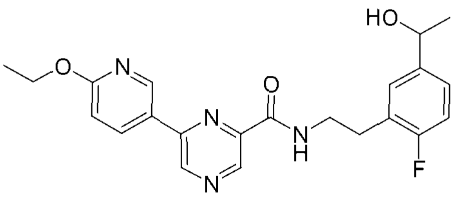 6-(6-ethoxypyridine-3-yl)-*N*-(2-fluoro-5-(1-hydroxyethyl)phenethyl)pyrazine-2-carboxamide | $^1$H-NMR, 400 MHz, DMSO-$d_6$ $\delta$ = 9.47 (s, 1H), 9.29 - 9.15 (m, 2H), 9.06 (s, 1H), 8.70 (dd, J = 2.5, 8.8 Hz, 1H), 7.29 (dd, J = 2.1, 7.4 Hz, 1H), 7.21 (ddd, J = 2.2, 5.4, 8.1 Hz, 1H), 7.08 (dd, J = 8.6, 9.8 Hz, 1H), 6.98 (d, J = 8.8 Hz, 1H), 5.12 (d, J = 4.1 Hz, 1H), 4.73 - 4.57 (m, 1H), 4.41 (q, J = 7.0 Hz, 2H), 3.64 - 3.48 (m, 2H), 2.93 (br t, J = 7.5 Hz, 2H), 1.37 (t, J = 7.1 Hz, 3H), 1.22 (d, J = 6.5 Hz, 3H). [M+H]$^+$: 411.4. |

(continued)

| Compound No | Structure | $^1$H-NMR / MS (m/z) $[M+H]^+$ |
|---|---|---|
| 44 | 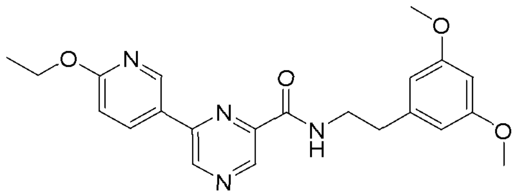<br>*N*-(3,5-dimethoxyphenethyl)-6-(6-ethoxypyridine-3-yl)pyrazine-2-carboxamide | $^1$H-NMR, 400 MHz, DMSO-$d_6$ $\delta$ = 9.48 (s, 1H), 9.22 (d, J = 2.4 Hz, 1H), 9.12 (t, J = 6.1 Hz, 1H), 9.08 (s, 1H), 8.68 (dd, J = 2.5, 8.8 Hz, 1H), 6.99 (d, J = 8.8 Hz, 1H), 6.44 (d, J = 2.3 Hz, 2H), 6.35 (t, J = 2.3 Hz, 1H), 4.42 (q, J = 7.0 Hz, 2H), 3.71 (s, 6H), 3.63 - 3.52 (m, 2H), 2.85 (t, J = 7.4 Hz, 2H), 1.38 (t, J = 7.1 Hz, 3H).<br>$[M+H]^+$: 409.4. |
| 45 | 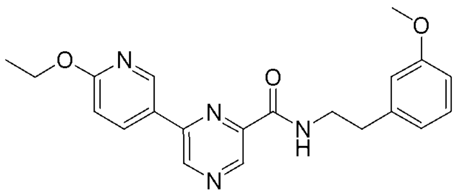<br>6-(6-ethoxypyridine-3-yl)-*N*-(3-methoxyphenethyl)pyrazine-2-carboxamide | $^1$H-NMR, 400 MHz, DMSO-$d_6$ $\delta$ = 9.48 (s, 1H), 9.23 (d, J = 2.3 Hz, 1H), 9.16 (br t, J = 5.9 Hz, 1H), 9.08 (s, 1H), 8.69 (dd, J = 2.5, 8.8 Hz, 1H), 7.23 (t, J = 7.8 Hz, 1H), 7.00 (d, J = 8.8 Hz, 1H), 6.90 - 6.75 (m, 3H), 4.42 (q, J = 7.0 Hz, 2H), 3.72 (s, 3H), 3.63 - 3.52 (m, 2H), 2.89 (t, J = 7.6 Hz, 2H), 1.38 (t, J = 7.0 Hz, 3H).<br>$[M+H]^+$: 379.4. |
| 46 | 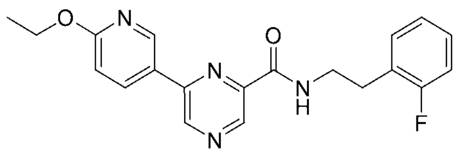<br>6-(6-ethoxypyridine-3-yl)-*N*-(2-fluorophenethyl)pyrazine-2-carboxamide | $^1$H-NMR, 400 MHz, DMSO-$d_6$ $\delta$ = 9.53 (s, 1H), 9.33 - 9.25 (m, 2H), 9.12 (s, 1H), 8.76 (dd, J = 2.5, 8.8 Hz, 1H), 7.44 - 7.29 (m, 2H), 7.26 - 7.16 (m, 2H), 7.05 (d, J = 8.8 Hz, 1H), 4.47 (q, J = 7.0 Hz, 2H), 3.72 - 3.57 (m, 2H), 3.01 (t, J = 7.4 Hz, 2H), 1.43 (t, J = 7.1 Hz, 3H).<br>$[M+H]^+$: 367.4. |
| 47 | 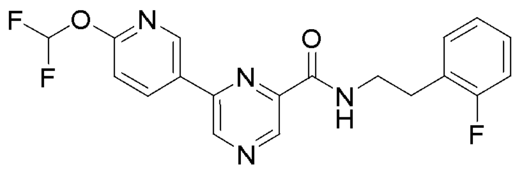<br>6-(6-(difluoromethoxy)pyridine-3-yl)-*N*-(2-fluorophenethyl)pyrazine-2-carboxamide | $^1$H-NMR, 400 MHz, DMSO-$d_6$ $\delta$ = 9.56 (s, 1H), 9.33 (d, J = 2.3 Hz, 1H), 9.28 (br t, J = 5.8 Hz, 1H), 9.14 (s, 1H), 8.92 (dd, J = 2.4, 8.7 Hz, 1H), 8.03 - 7.63 (m, 1H), 7.38 - 7.31 (m, 2H), 7.31 - 7.24 (m, 1H), 7.21 - 7.11 (m, 2H), 3.64 - 3.55 (m, 2H), 2.96 (t, J = 7.4 Hz, 2H).<br>$[M+H]^+$: 389.1. |
| 48 | 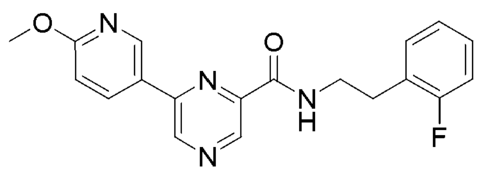<br>*N*-(2-fluorophenethyl)-6-(6-methoxypyridine-3-yl)pyrazine-2-carboxamide | $^1$H-NMR, 400 MHz, DMSO-$d_6$ $\delta$ = 9.48 (s, 1H), 9.24 (d, J = 2.4 Hz, 1H), 9.23 - 9.18 (m, 1H), 9.07 (s, 1H), 8.70 (dd, J = 2.5, 8.7 Hz, 1H), 7.38 - 7.32 (m, 1H), 7.32 - 7.24 (m, 1H), 7.20 - 7.11 (m, 2H), 7.03 (d, J = 8.8 Hz, 1H), 3.97 (s, 3H), 3.63 - 3.56 (m, 2H), 2.96 (t, J = 7.4 Hz, 2H).<br>$[M+H]^+$: 353.1 |

(continued)

| Compound No | Structure | $^1$H-NMR / MS (m/z) [M+H]$^+$ |
|---|---|---|
| 49 | 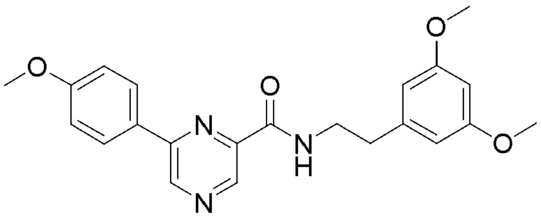 *N*-(3,5-dimethoxyphenethyl)-6-(4-methoxyphenyl)pyrazine-2-carboxamide | $^1$H-NMR, 400 MHz, DMSO-$d_6$ $\delta$ = 9.42 (s, 1H), 9.03 (s, 1H), 9.02 - 8.98 (m, 1H), 8.36 - 8.30 (m, 2H), 7.15 - 7.09 (m, 2H), 6.45 (d, J = 2.3 Hz, 2H), 6.36 (t, J = 2.2 Hz, 1H), 3.87 (s, 3H), 3.71 (s, 6H), 3.63 - 3.56 (m, 2H), 2.85 (t, J = 7.4 Hz, 2H). [M+H]$^+$: 394.1. |
| 50 | 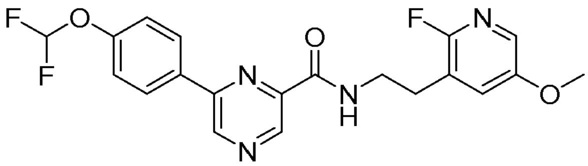 6-(4-(difluoromethoxy)phenyl)-*N*-(2-(2-fluoro-5-methoxypyridine-3-yl)ethyl)pyrazine-2-carboxamide | $^1$H-NMR, 400 MHz, DMSO-$d_6$ $\delta$ = 9.47 (s, 1H), 9.17 (br t, J = 5.9 Hz, 1H), 9.07 (s, 1H), 8.45 (d, J = 8.8 Hz, 2H), 7.74 (br s, 1H), 7.59 (s, 1H), 7.49 (dd, J = 2.8, 8.1 Hz, 1H), 7.41 (s, 1H), 7.36 (d, J = 8.8 Hz, 2H), 7.23 (s, 1H), 3.75 (s, 3H), 3.63 (q, J = 6.6 Hz, 2H), 2.92 (br t, J = 6.9 Hz, 2H). [M+H]$^+$: 419.1. |
| 51 | 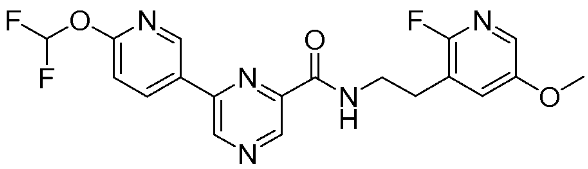 6-(6-(difluoromethoxy)pyridine-3-yl)-*N*-(2-(2-fluoro-5-methoxypyridine-3-yl)ethyl)pyrazine-2-carboxamide | $^1$H-NMR, 400 MHz, DMSO-$d_6$ $\delta$ = 9.55 (s, 1H), 9.30 (d, J = 2.3 Hz, 1H), 9.26 (br t, J = 6.1 Hz, 1H), 9.12 (s, 1H), 8.90 (dd, J = 2.5, 8.6 Hz, 1H), 8.04 - 7.62 (m, 2H), 7.49 (dd, J = 3.0, 8.0 Hz, 1H), 7.31 (d, J = 8.6 Hz, 1H), 3.75 (s, 3H), 3.63 (q, J = 6.8 Hz, 2H), 2.92 (t, J = 7.0 Hz, 2H). [M+H]$^+$: 420.1. |
| 52 | 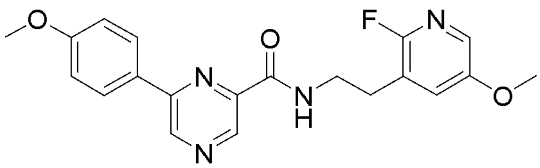 *N*-(2-(2-fluoro-5-methoxypyridine-3-yl)ethyl)-6-(4-methoxyphenyl)pyrazine-2-carboxamide | $^1$H-NMR, 400 MHz, DMSO-$d_6$ $\delta$ = 9.40 (s, 1H), 9.11 (t, J = 6.0 Hz, 1H), 8.99 (s, 1H), 8.33 (d, J = 8.9 Hz, 2H), 7.78 - 7.69 (m, 1H), 7.49 (dd, J = 3.0, 8.0 Hz, 1H), 7.14 - 7.04 (m, 2H), 3.86 (s, 3H), 3.76 (s, 3H), 3.63 (q, J = 6.8 Hz, 2H), 2.92 (t, J = 7.0 Hz, 2H). [M+H]$^+$: 383.1. |
| 53 | 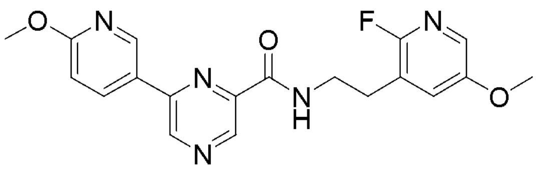 *N*-(2-(2-fluoro-5-methoxypyridine-3-yl)ethyl)-6-(6-methoxypyridine-3-yl)pyrazine-2-carboxamide | $^1$H-NMR, 400 MHz, DMSO-$d_6$ $\delta$ = 9.47 (s, 1H), 9.25 - 9.17 (m, 2H), 9.05 (s, 1H), 8.68 (dd, J = 2.5, 8.8 Hz, 1H), 7.78 - 7.66 (m, 1H), 7.49 (dd, J = 2.9, 8.1 Hz, 1H), 7.01 (d, J = 8.8 Hz, 1H), 3.95 (s, 3H), 3.75 (s, 3H), 3.62 (q, J = 6.8 Hz, 2H), 2.92 (t, J = 7.0 Hz, 2H). [M+H]$^+$: 384.1 |

(continued)

| Compound Nº | Structure | $^1$H-NMR / MS (m/z) [M+H]$^+$ |
|---|---|---|
| 54 | 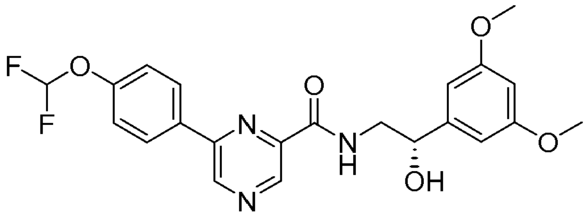 (*S*)-6-(4-(difluoromethoxy)phenyl)-*N*-(2-(3,5-dimethoxyphenyl)-2-hydroxyethyl)pyrazine-2-carboxamide | $^1$H-NMR, 400 MHz, DMSO-$d_6$ $\delta$ = 9.49 (s, 1H), 9.11 (s, 1H), 8.87 (t, J = 5.88 Hz, 1H), 8.41 (d, J = 8.76 Hz, 2H), 7.37 (d, J = 8.76 Hz, 3H), 6.57 (d, J = 2.25 Hz, 2H), 6.40 (t, J = 2.19 Hz, 1H), 5.63 (br s, 1H), 4.80 (br dd, J = 7.19, 4.57 Hz, 1H), 3.71 (s, 6H), 3.62 - 3.53 (m, 1H), 3.46 (ddd, J = 13.35, 7.54, 5.88 Hz, 1H). [M+H]$^+$: 446, 1. |
| 55 | 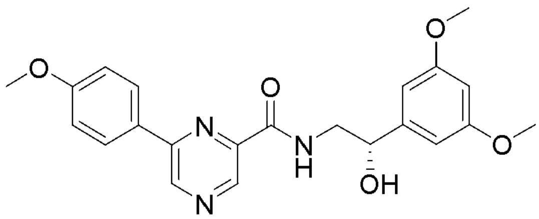 (*S*)-N-(2-(3,5-dimethoxyphenyl)-2-hydroxyethyl)-6-(4-methoxyphenyl)pyrazine-2-carboxamide | $^1$H-NMR, 400 MHz, DMSO-$d_6$ $\delta$ = 9.42 (s, 1H), 9.03 (s, 1H), 8.81 (t, J = 5.82 Hz, 1H), 8.29 (d, J = 9.01 Hz, 2H), 7.12 (d, J = 8.88 Hz, 2H), 6.58 (d, J = 2.25 Hz, 2H), 6.40 (t, J = 2.25 Hz, 1H), 5.79 - 5.48 (m, 1H), 4.80 (dd, J = 7.63, 4.50 Hz, 1H), 3.86 (s, 3H), 3.71 (s, 6H), 3.53 - 3.62 (m, 1H), 3.46 (ddd, J = 13.32, 7.57, 5.88 Hz, 1H). [M+H]$^+$: 410.2. |
| 56 | 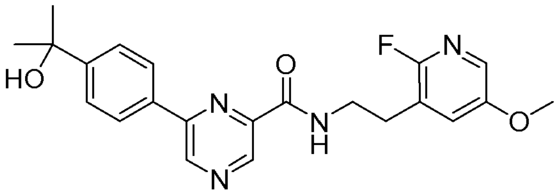 *N*-(2-(2-fluoro-5-methoxypyridine-3-yl)ethyl)-6-(4-(2-hydroxypropane-2-yl)phenyl)pyrazine-2-carboxamide | $^1$H-NMR, 400 MHz, DMSO-$d_6$ $\delta$ = 9.43 (s, 1H), 9.10 (br t, J = 6.0 Hz, 1H), 9.06 (s, 1H), 8.27 (d J = 8.5 Hz, 2H), 7.75 (t, J = 2.4 Hz, 1H), 7.65 (d, J = 8.5 Hz, 2H), 7.51 (dd, J = 3.0, 8.0 Hz, 1H), 5.16 (s, 1H), 3.77 (s, 3H), 3.65 (q, J = 6.7 Hz, 2H), 2.94 (t, J = 6.9 Hz, 2H), 1.49 (s, 6H). [M+H]$^+$: 411.1. |
| 57 | 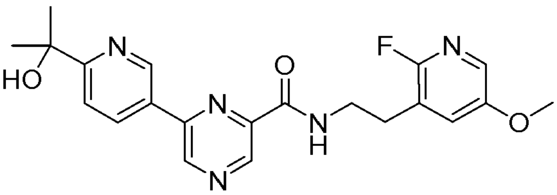 *N*-(2-(2-fluoro-5-methoxypyridine-3-yl)ethyl)-6-(6-(2-hydroxypropane-2-yl)pyridine-3-yl)pyrazine-2-carboxamide | $^1$H-NMR, 400 MHz, DMSO-$d_6$ $\delta$ = 9.52 (s, 1H), 9.48 (d, J = 1.6 Hz, 1H), 9.24 (t, J = 6.0 Hz, 1H), 9.12 (s, 1H), 8.71 (dd, J = 2.4 Hz, 1H), 7.84 (d, J = 8.4 Hz, 1H), 7.75 - 7.73 (m, 1H), 7.52 (dd, J = 3.2 Hz, 1H), 5.38 (s, 1H), 3.76 (s, 3H), 3.64 (q, J = 6.8 Hz, 2H), 2.95 (t, J = 7.2 Hz, 2H), 1.50 (s, 6H). [M+H]$^+$: 412.2. |

(continued)

| Compound Nº | Structure | $^1$H-NMR / MS (m/z) [M+H]$^+$ |
|---|---|---|
| 58 | 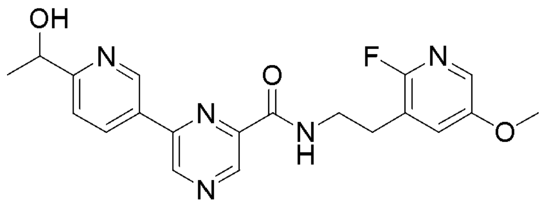<br>*N*-(2-(2-fluoro-5-methoxypyridine-3-yl)ethyl)-6-(6-(1-hydroxyethyl)pyridine-3-yl)pyrazine-2-carboxamide | $^1$H-NMR, 400 MHz, DMSO-$d_6$ $\delta$ = 9.53 (s, 1H), 9.46 (d, J = 1.7 Hz, 1H), 9.22 (t, J = 6.1 Hz, 1H), 9.12 (s, 1H), 8.72 (dd, J = 2.3, 8.3 Hz, 1H), 7.76 - 7.72 (m, 1H), 7.69 (d, J = 8.2 Hz, 1H), 7.50 (dd, J = 3.0, 8.0 Hz, 1H), 5.53 (br d, J = 3.1 Hz, 1H), 4.83 (br d, J = 4.5 Hz, 1H), 3.76 (s, 3H), 3.64 (q, J = 6.8 Hz, 2H), 2.93 (t, J = 7.0 Hz, 2H), 2.51 (td, J = 1.8, 3.6 Hz, 27H), 1.43 (d, J = 6.6 Hz, 3H).<br>[M+H]$^+$: 398.1. |
| 59 | 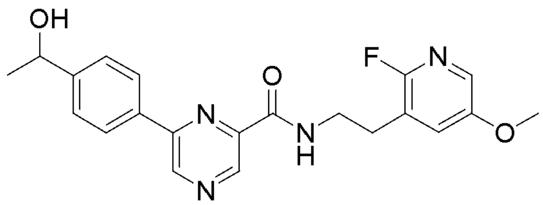<br>*N*-(2-(2-fluoro-5-methoxypyridine-3-yl)ethyl)-6-(4-(1-hydroxyethyl)phenyl)pyrazine-2-carboxamide | $^1$H-NMR, 400 MHz, DMSO-$d_6$ $\delta$ = 9.45 (s, 1H), 9.13 (t, J = 6.1 Hz, 1H), 9.06 (s, 1H), 8.31 (d, J = 8.3 Hz, 2H), 7.78 - 7.70 (m, 1H), 7.59 - 7.46 (m, 3H), 5.31 (br s, 1H), 4.83 (br d, J = 6.2 Hz, 1H), 3.76 (s, 3H), 3.64 (q, J = 6.8 Hz, 2H), 2.93 (t, J = 7.0 Hz, 2H), 1.38 (d, J = 6.4 Hz, 3H).<br>[M+H]$^+$: 397.2. |
| 60 | 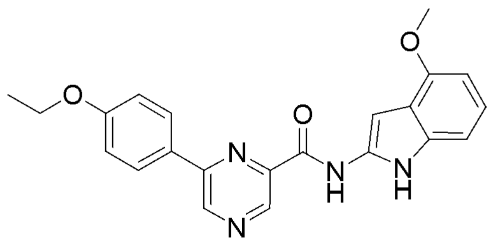<br>6-(4-ethoxyphenyl)-*N*-(4-methoxy-1*H*-indole-2-yl)pyrazine-2-carboxamide | $^1$H-NMR, 400 MHz, DMSO-$d_6$ $\delta$ = 11.16 (s, 2H), 9.52 (d, J = 11.2 Hz, 1H), 9.18 (s, 1H), 8.49 (m, 2H), 7.16 (d, J = 9.2 Hz, 2H), 7.09 (d, J = 8.0 Hz, 1H), 6.99 (d, J = 7.6 Hz, 1H), 6.65 (d, J = 1.6 Hz, 1H), 6.54 (d, J = 7.6 Hz, 1H), 4.19 (q, J = 6.8 Hz, 2H), 3.88 (s, 3 H), 1.41 (t, J = 6.8 Hz, 3H).<br>[M+H]$^+$: 389.1. |
| 61 | 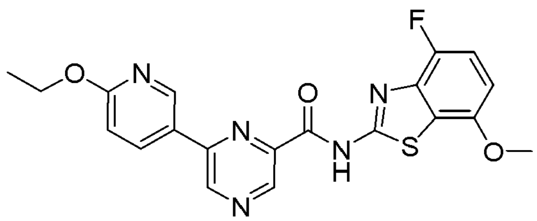<br>6-(6-ethoxypyridine-3-yl)-*N*-(4-fluoro-7-methoxybenzo[d]thiazole-2-yl)pyrazine-2-carboxamide | $^1$H-NMR, 400 MHz, DMSO-$d_6$ $\delta$ = 13.49 (s, 1H), 9.58 (s, 1H), 9.36 (d, J = 2.4 Hz, 1H), 9.26 (s, 1H), 8.98 (dd, J = 2.4, 8.8 Hz, 1H), 7.31 (dd, J = 8.9, 10.4 Hz, 1H), 7.01 (d, J = 8.6 Hz, 1H), 6.94 (dd, J = 2.9, 8.8 Hz, 1H), 4.43 (q, J = 6.9 Hz, 2H), 3.97 (s, 3H), 1.38 (t, J = 7.0 Hz, 3H).<br>[M+H]$^+$: 426.1. |
| 62 | 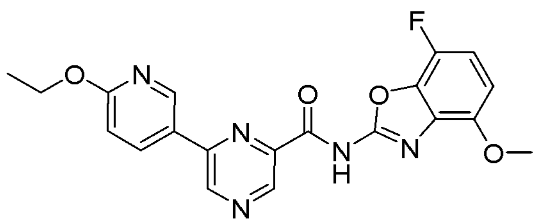<br>6-(6-ethoxypyridine-3-yl)-*N*-(7-fluoro-4-methoxybenzo[d]oxazole-2-yl)pyrazine-2-carboxamide | $^1$H-NMR, 400 MHz, DMSO-$d_6$ $\delta$ = 12.46 (br s, 1H), 9.58 (s, 1H), 9.33 (d, J = 2.3 Hz, 1H), 9.22 (s, 1H), 8.89 (dd, J = 2.5, 8.8 Hz, 1H), 7.23 (t, J = 9.5 Hz, 1H), 7.01 (d, J = 8.8 Hz, 1H), 6.92 (dd, J = 3.3, 9.2 Hz, 1H), 4.42 (q, J = 7.0 Hz, 2H), 3.97 (s, 3H), 1.37 (t, J = 7.0 Hz, 3H).<br>[M+H]$^+$: 426.1. |

(continued)

| Compound №| Structure | $^1$H-NMR / MS (m/z) [M+H]$^+$ |
|---|---|---|
| 63 | 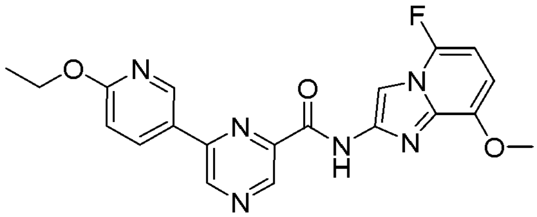<br>6-(6-ethoxypyridine-3-yl)-*N*-(5-fluoro-8-methoxyimidazo[1,2-a] pyridine-2-yl)pyrazine-2-carboxamide | $^1$H-NMR, 400 MHz, DMSO-$d_6$ $\delta$ = 11.78 (s, 1H), 9.53 (s, 1H), 9.31 (d, J = 2.4 Hz, 1H), 9.22 (s, 1H), 8.93 (dd, J = 2.5, 8.8 Hz, 1H), 8.20 (s, 1H), 7.01 (d, J = 8.8 Hz, 1H), 6.84 - 6.73 (m, 2H), 4.43 (q, J = 7.0 Hz, 2H), 3.95 (s, 3H), 1.39 (t, J = 7.0 Hz, 3H).<br>[M+H]$^+$: 409.1. |
| 64 | 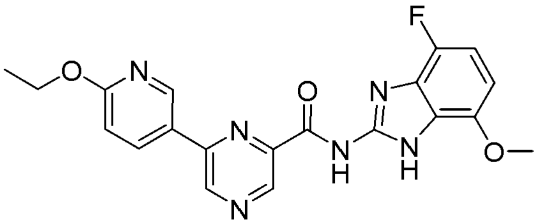<br>6-(6-ethoxypyridine-3-yl)-*N*-(4-fluoro-7-methoxy-1*H*-benzo[d] imidazole-2-yl)pyrazine-2-carboxamide | $^1$H-NMR, 400 MHz, DMSO-$d_6$ $\delta$ = 12.78 - 12.29 (m, 1H), 12.22 - 12.13 (m, 1H), 9.59 (s, 1H), 9.35 (d, J = 2.2 Hz, 1H), 9.25 (s, 1H), 8.94 (dd, J = 2.2, 8.8 Hz, 1H), 7.06 - 6.90 (m, 2H), 6.77 - 6.60 (m, 1H), 4.43 (q, J = 7.1 Hz, 2H), 3.94 (s, 3H), 1.38 (t, J = 7.0 Hz, 3H).<br>[M+H]$^+$: 409.1. |
| 65 | 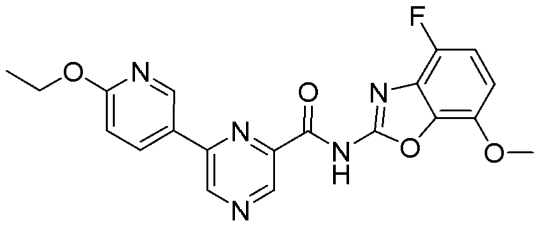<br>6-(6-ethoxypyridine-3-yl)-*N*-(4-fluoro-7-methoxybenzo[d]oxazole-2-yl)pyrazine-2-carboxamide | $^1$H-NMR, 400 MHz, DMSO-$d_6$ $\delta$ = 12.50 (s, 1H), 9.58 (s, 1H), 9.33 (d, J = 2.3 Hz, 1H), 9.22 (s, 1H), 8.89 (dd, J = 2.5, 8.7 Hz, 1H), 7.19 (t, J = 9.4 Hz, 1H), 7.04 - 6.95 (m, 2H), 4.42 (q, J = 7.1 Hz, 2H), 3.97 (s, 3H), 1.37 (t, J = 7.0 Hz, 3H).<br>[M+H]$^+$: 410.1. |
| 66 | 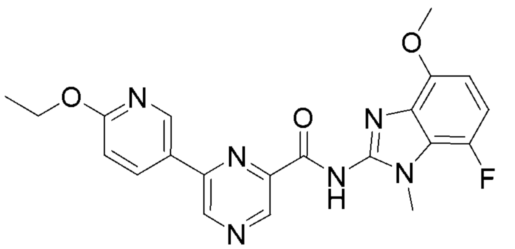<br>6-(6-ethoxypyridine-3-yl)-*N*-(7-fluoro-4-methoxy-1-methyl-1*H*-benzo[d]imidazole-2-yl)pyrazine-2-carboxamide | $^1$H-NMR, 400 MHz, DMSO-$d_6$ $\delta$ = 1.66 - 11.34 (m, 1H), 9.66 - 9.49 (m, 1H), 9.37 - 9.15 (m, 2H), 8.92 - 8.74 (m, 1H), 7.11 - 6.89 (m, 2H), 6.76 - 6.61 (m, 1H), 4.47 - 4.37 (m, 2H), 3.92 (s, 3H), 3.86 - 3.78 (m, 3H), 1.37 (t, J = 7.0 Hz, 3H).<br>[M+H]$^+$: 423.2. |
| 67 | 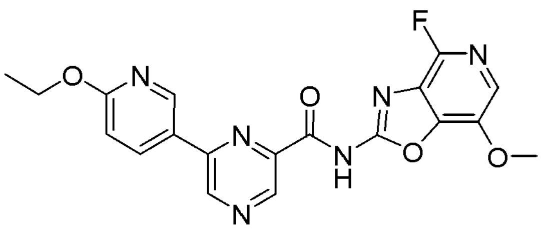<br>6-(6-ethoxypyridine-3-yl)-*N*-(4-fluoro-7-methoxyoxazole[4,5-c] pyridine-2-yl)pyrazine-2-carboxamide | $^1$H-NMR, 400 MHz, DMSO-$d_6$ $\delta$ = 9.56 (s, 1H), 9.31 (s, 2H), 9.22 (s, 1H), 8.87 (d, J = 8.8 Hz, 1H), 7.79 (s, 1H), 7.02 (d, J = 8.4 Hz, 1H), 4.45 (q, J = 6.8 Hz, 2H), 4.06 (s, 3H), 1.39 (t, J = 7.2 Hz, 3H).<br>[M+H]$^+$: 411.1. |

(continued)

| Compound No | Structure | $^1$H-NMR / MS (m/z) [M+H]$^+$ |
|---|---|---|
| 68 | 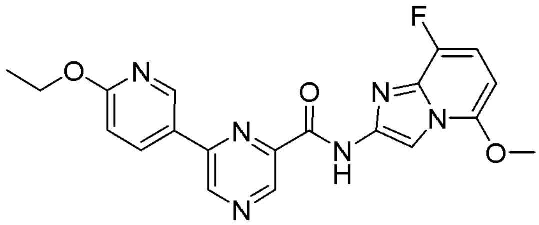 6-(6-ethoxypyridine-3-yl)-*N*-(8-fluoro-5-methoxyimidazo[1,2-a] pyridine-2-yl)pyrazine-2-carboxamide | $^1$H-NMR, 400 MHz, DMSO-$d_6$ $\delta$ = 11.71 (s, 1H), 9.52 (s, 1H), 9.30 (d, J = 2.5 Hz, 1H), 9.21 (s, 1H), 8.92 (dd, J = 8.7, 2.6 Hz, 1H), 8.20 (d, J = 3.0 Hz, 1H), 7.27 (dd, J = 11.0, 8.4 Hz, 1H), 7.00 (d, J = 8.7 Hz, 1H), 6.32 (dd, J = 8.5, 3.0 Hz, 1H), 4.43 (q, J = 7.0 Hz, 2H), 4.13 (s, 3H), 1.38 (t, J = 7.1 Hz, 3H). [M+H]$^+$: 409.4. |
| 69 | 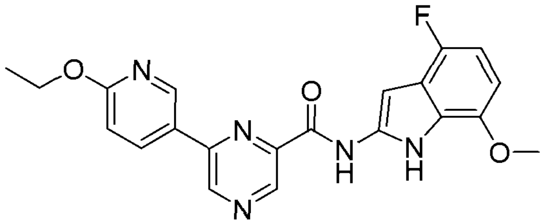 6-(6-ethoxypyridine-3-yl)-*N*-(4-fluoro-7-methoxy-1*H*-indole-2-yl)pyrazine-2-carboxamide | $^1$H-NMR, 400 MHz, DMSO-$d_6$ $\delta$ = 11.42 (s, 1H), 10.96 (s, 1H), 9.58 (s, 1H), 8.81 (t, J = 2.0 Hz, 1H), 9.24 (s, 1H), 8.76 (dd, J = 6.0, 2.8 Hz, 1H), 7.06 (dd, J = 8.4, 0.4 Hz, 1H), 6.80 (s, 1H), 6.73 - 6.69 (m, 1H), 6.59 - 6.56 (m, 1H), 4.47 (q, J = 6.8 Hz, 2H), 3.93 (s, 3H), 1.41 (t, J = 7.2 Hz, 3H). [M+H]$^+$: 408.3. |
| 70 | 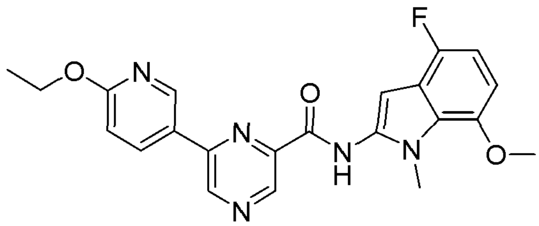 6-(6-ethoxypyridine-3-yl)-*N*-(4-fluoro-7-methoxy-1-methyl-1*H*-indole-2-yl)pyrazine-2-carboxamide | $^1$H-NMR, 400 MHz, DMSO-$d_6$ $\delta$ = 9.55 (s, 1H), 9.28 (d, J = 2.4 Hz, 1H), 9.20 (s, 1H), 8.81 (dd, J = 6.0, 2.4 Hz, 1H), 7.01 (d, J = 8.8 Hz, 1H), 6.76 (t, J = 8.8 Hz, 1H), 6.64 - 6.61 (m, 1H), 6.53 (s, 1H), 4.45 (q, J = 6.8 Hz, 2H), 3.90 (d, J = 0.8 Hz, 6H), 1.39 (t, J = 6.8 Hz, 3H). [M+H]$^+$: 422.3. |
| 71 | 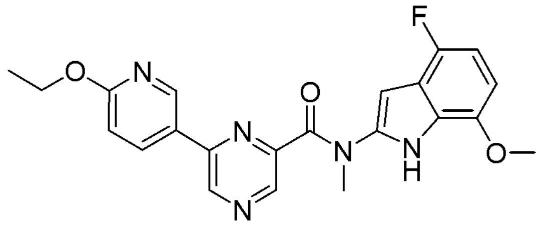 6-(6-ethoxypyridine-3-yl)-*N*-(4-fluoro-7-methoxy-1*H*-indole-2-yl)-*N*-methylpyrazine-2-carboxamide | $^1$H-NMR, 400 MHz, DMSO-$d_6$ $\delta$ = 12.08 (s, 1H), 9.13 (s, 1H), 8.81 (s, 1H), 8.61 (s, 1H), 7.82 (d, J = 8.0 Hz, 1H), 6.63 - 6.54 (m, 3H), 6.12 (s, 1H), 4.35 (q, J = 7.2 Hz, 2H), 3.90 (s, 3H), 3.46 (s, 3H), 1.33 (t, J = 6.8 Hz, 3H). [M+H]$^+$: 422.3. |
| 72 | 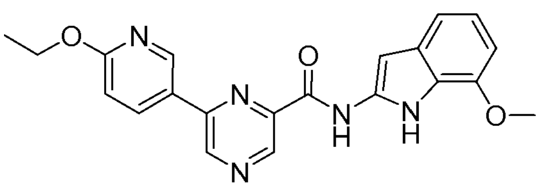 6-(6-ethoxypyridine-3-yl)-*N*-(7-methoxy-1*H*-indole-2-yl)pyrazine-2-carboxamide | $^1$H-NMR, 400 MHz, DMSO-$d_6$ $\delta$ = 1.41 (3H, t, J = 7.00 Hz), 3.94 (3H, s), 4.42 - 4.48 (2H, m), 6.03 - 6.29 (1H, m), 6.66 (1H, d, J = 8.00 Hz), 6.73 (1H, t, J = 7.75 Hz), 7.00 (1H, d, J = 8.76 Hz), 7.50 (2H, br s), 8.45 (1H, m), 8.84 (1H, s), 9.01 (1H, d, J = 2.38 Hz), 9.47 (1H, s), 11.19 (1H, br s) . [M+H]$^+$: 390.1. |

(continued)

| Compound Nº | Structure | $^1$H-NMR / MS (m/z) [M+H]$^+$ |
|---|---|---|
| 73 | 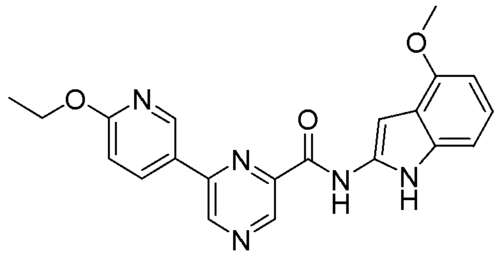 6-(6-ethoxypyridine-3-yl)-*N*-(4-methoxy-1*H*-indole-2-yl)pyrazine-2-carboxamide | $^1$H-NMR, 400 MHz, DMSO-$d_6$ $\delta$ = 1.35 (3H, t, J = 7.03 Hz) 3.01 (3H, s) 4.37 (2H, m) 6.38 (1H, d, J = 7.82 Hz) 6.81 - 6.93 (3H, m) 7.80 (2H, s) 8.24 (1H, m) 8.63 (1H, s) 8.79 (1H, d, J = 2.32 Hz) 9.22 (1H, s) . [M+H]$^+$: 390.1. |
| 74 | 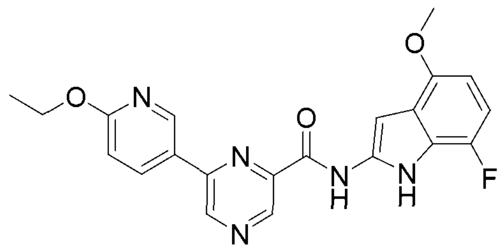 6-(6-ethoxypyridine-3-yl)-*N*-(7-fluoro-4-methoxy-1*H*-indole-2-yl)pyrazine-2-carboxamide | $^1$H-NMR, 400 MHz, DMSO-$d_6$ $\delta$ = 11.47 (s, 1H), 10.97 (s, 1H), 9.57 (s, 1H), 9.30 (d, J = 2.4 Hz, 1H), 9.24 (s, 1H), 8.76 (dd, J = 6.4, 2.4 Hz, 1H), 7.06 (d, J = 8.8 Hz, 1H), 6.84 - 6.79 (m, 2H), 6.45 (dd, J = 3.2 Hz, 1H), 4.46 (q, J = 6.8 Hz, 2H), 3.86 (s, 3H), 1.40 (t, J = 6.8 Hz, 3H). [M+H]$^+$: 408.1. |
| 75 | 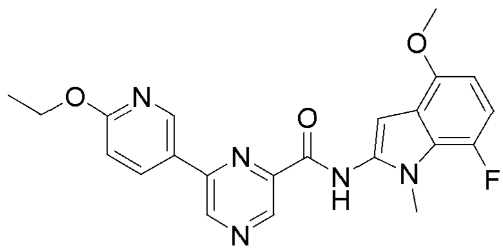 6-(6-ethoxypyridine-3-yl)-*N*-(7-fluoro-4-methoxy-1-methyl-1*H*-indole-2-yl)pyrazine-2-carboxamide | $^1$H-NMR, 400 MHz, DMSO-$d_6$ $\delta$ = 10.94 (s, 1H), 9.58 (s, 1H), 9.31 (d, J = 2.4 Hz, 1H), 9.20 (s, 1H), 8.85 (dd, J = 6.4, 2.4 Hz, 1H), 7.02 (d, J = 8.8 Hz, 1H), 6.90 - 6.84 (m, 1H), 6.53 (d, J = 2.0 Hz, 1H), 6.47 (dd, J = 5.6, 2.8 Hz, 1H), 4.43 (t, J = 7.2 Hz, 2H), 3.86 (s, 3H), 3.80 (s, 3H), 1.39 (t, J = 6.8 Hz, 3H). [M+H]$^+$: 422.1. |
| 76 | 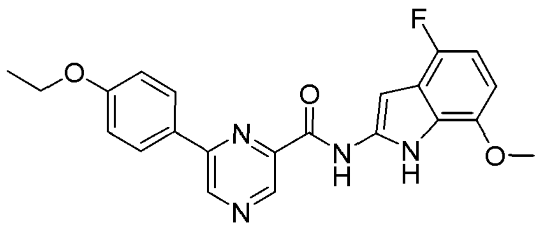 6-(4-ethoxyphenyl)-*N*-(4-fluoro-7-methoxy-1*H*-indole-2-yl)pyrazine-2-carboxamide | $^1$H-NMR, 400 MHz, DMSO-$d_6$ $\delta$ = 11.30 - 11.51 (m, 1H), 9.21 (s, 1H), 8.63 (s, 1H), 7.95 - 7.99 (m, 2H), 7.44 (br s, 2H), 6.98 - 7.02 (m, 2H), 6.53 - 6.57 (m, 1H), 6.43 - 6.49 (m, 1H), 4.05 - 4.10 (m, 2H), 3.86 (s, 3H), 1.33 (t, J = 6.94 Hz, 3H). [M+H]$^+$: 407.2. |
| 77 | 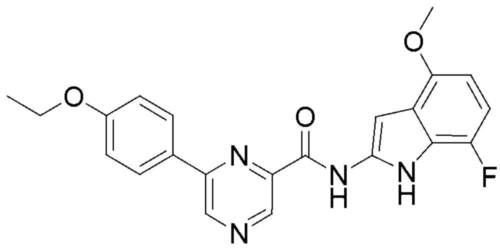 6-(4-ethoxyphenyl)-*N*-(7-fluoro-4-methoxy-1*H*-indole-2-yl)pyrazine-2-carboxamide | $^1$H-NMR, 400 MHz, DMSO-$d_6$ $\delta$ = 11.61 (br s, 1H), 9.16 (s, 1H), 8.56 (s, 1H), 7.92 (d, J = 8.88 Hz, 2H), 7.58 (s, 2H), 7.00 (d, J = 8.88 Hz, 2H), 6.68 - 6.78 (m, 1H), 6.27 (dd, J = 8.88, 3.50 Hz, 1H), 4.07 (q, J = 6.92 Hz, 2H), 2.96 (s, 3H), 1.33 (t, J = 7.00 Hz, 3H). [M+H]$^+$: 407.1. |

(continued)

| Compound № | Structure | $^1$H-NMR / MS (m/z) [M+H]$^+$ |
|---|---|---|
| 78 | 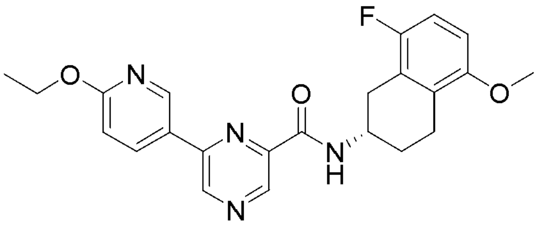 (*S*)-6-(6-ethoxypyridine-3-yl)-*N*-(8-fluoro-5-methoxy-1,2,3,4-tetrahydronaphthalene-2-yl)pyrazine-2-carboxamide | $^1$H-NMR, 400 MHz, DMSO-$d_6$ $\delta$ = 1.36 (br t, J=6.94 Hz, 3 H) 1.85 (qd, J=11.63, 5.00 Hz, 1 H) 2.08 (br d, J=10.01 Hz, 1 H) 2.53 - 2.66 (m, 1 H) 2.78 - 2.97 (m, 2 H) 3.03 (br dd, J=16.38, 3.38 Hz, 1 H) 3.77 (s, 3 H) 4.22 (br s, 1 H) 4.40 (q, J=6.96 Hz, 2 H) 6.78 (br dd, J=8.38, 3.88 Hz, 1 H) 6.91 - 7.01 (m, 2 H) 8.68 (br d, J=8.75 Hz, 1 H) 8.93 (br d, J=8.00 Hz, 1 H) 9.10 (s, 1 H) 9.22 (s, 1 H) 9.46 (s, 1 H). [M+H]$^+$: 423.2. |
| 79 | 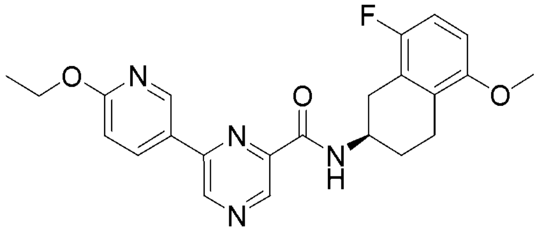 (*R*)-6-(6-ethoxypyridine-3-yl)-*N*-(8-fluoro-5-methoxy-1,2,3,4-tetrahydronaphthalene-2-yl)pyrazine-2-carboxamide | $^1$H-NMR, 400 MHz, DMSO-$d_6$ $\delta$ = 1.36 (br t, J=6.94 Hz, 3 H) 1.85 (qd, J=11.63, 5.00 Hz, 1 H) 2.08 (br d, J=10.01 Hz, 1 H) 2.53 - 2.66 (m, 1 H) 2.78 - 2.97 (m, 2 H) 3.03 (br dd, J=16.38, 3.38 Hz, 1 H) 3.77 (s, 3 H) 4.22 (br s, 1 H) 4.40 (q, J=6.96 Hz, 2 H) 6.78 (br dd, J=8.38, 3.88 Hz, 1 H) 6.91 - 7.01 (m, 2 H) 8.68 (br d, J=8.75 Hz, 1 H) 8.93 (br d, J=8.00 Hz, 1 H) 9.10 (s, 1 H) 9.22 (s, 1 H) 9.46 (s, 1 H). [M+H]$^+$: 423.2. |
| 80 | 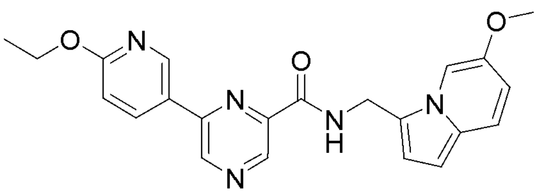 6-(6-ethoxypyridine-3-yl)-*N*-((6-methoxyindolizine-3-yl)methyl) pyrazine-2-carboxamide | $^1$H-NMR, 400 MHz, DMSO-$d_6$ $\delta$ = 9.54 (t, J = 6.1 Hz, 1H), 9.46 (s, 1H), 9.21 (d, J = 2.4 Hz, 1H), 9.11 (s, 1H), 8.68 (dd, J = 2.5, 8.8 Hz, 1H), 8.02 (d, J = 7.0 Hz, 1H), 6.96 (d, J = 8.6 Hz, 1H), 6.70 (d, J = 3.9 Hz, 1H), 6.56 (t, J = 7.1 Hz, 1H), 6.40 (d, J = 3.9 Hz, 1H), 6.16 (d, J = 7.4 Hz, 1H), 4.84 (d, J = 6.1 Hz, 2H), 4.39 (q, J = 7.1 Hz, 2H), 3.86 (s, 3H), 1.35 (t, J = 7.0 Hz, 3H). [M+H]$^+$: 404.2 |
| 81 | 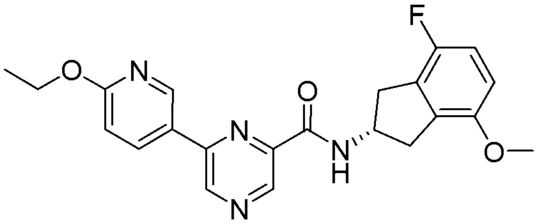 (*S*)-6-(6-ethoxypyridine-3-yl)-*N*-(4-fluoro-7-methoxy-2,3-dihydro-1*H*-inden-2-yl)pyrazine-2-carboxamide | $^1$H-NMR, 400 MHz, DMSO-$d_6$ $\delta$ = 9.47 (s, 1H), 9.28 - 9.21 (m, 2H), 9.09 (s, 1H), 8.70 (dd, J = 8.7, 2.5 Hz, 1H), 7.03 - 6.93 (m, 2H), 6.81 (dd, J = 8.9, 3.7 Hz, 1H), 4.92 (q, J = 7.7 Hz, 1H), 4.40 (q, J = 7.0 Hz, 2H), 3.77 (s, 3H), 3.30 - 3.21 (m, 2H), 3.10 (dd, J = 16.5, 6.9 Hz, 1H), 3.02 (dd, J = 16.8, 6.9 Hz, 1H), 1.35 (t, J = 7.1 Hz, 3H). [M+H]$^+$: 409.4. |

(continued)

| Compound No | Structure | $^1$H-NMR / MS (m/z) [M+H]$^+$ |
|---|---|---|
| 82 | 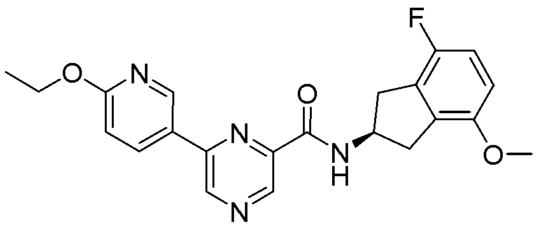 (*R*)-6-(6-ethoxypyridine-3-yl)-*N*-(4-fluoro-7-methoxy-2,3-dihydro-1*H*-inden-2-yl)pyrazine-2-carboxamide | $^1$H-NMR, 400 MHz, DMSO-$d_6$ $\delta$ = 9.47 (s, 1H), 9.28 - 9.21 (m, 2H), 9.09 (s, 1H), 8.70 (dd, J = 8.7, 2.6 Hz, 1H), 7.03 - 6.93 (m, 2H), 6.81 (dd, J = 8.9, 3.7 Hz, 1H), 4.92 (q, J = 7.6 Hz, 1H), 4.40 (q, J = 7.0 Hz, 2H), 3.77 (s, 3H), 3.30 - 3.21 (m, 2H), 3.10 (dd, J = 16.5, 6.9 Hz, 1H), 3.02 (dd, J = 16.5, 6.9 Hz, 1H),1.35 (t, J = 7.1 Hz, 3H).<br>[M+H]$^+$: 409.4. |
| 83 | 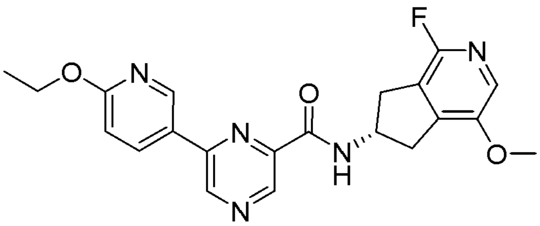 (*S*)-6-(6-ethoxypyridine-3-yl)-*N*-(1-fluoro-4-methoxy-6,7-dihydro-5*H*-cyclopenta[c]pyridine-6-yl)pyrazine-2-carboxamide | $^1$H-NMR, 400 MHz, DMSO-$d_6$ $\delta$ = 9.47 (s, 1H), 9.31 (d, J = 8.0 Hz, 1H), 9.24 (d, J = 2.0 Hz, 1H), 9.10 (s, 1H), 8.71 (dd, J = 2.8, 6.0 Hz, 1H), 7.73 (d, J = 1.6 Hz, 1H), 6.98 (d, J = 8.8 Hz, 1H), 5.04 - 4.95 (m, 1H), 4.43 (q, J = 6.8 Hz, 2H), 3.89 (s, 3H), 3.40 - 3.35 (m, 2H), 3.13 - 3.06 (m, 2H), 1.38 (t, J = 7.2 Hz, 3H).<br>[M+H]$^+$: 410.1. |
| 84 | 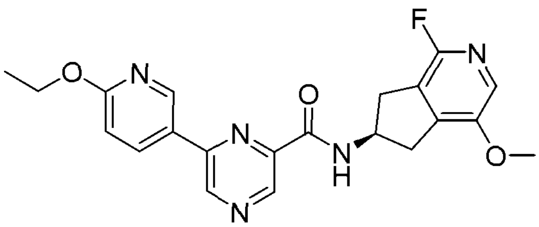 (*R*)-6-(6-ethoxypyridine-3-yl)-*N*-(1-fluoro-4-methoxy-6,7-dihydro-5*H*-cyclopenta[c]pyridine-6-yl)pyrazine-2-carboxamide | $^1$H-NMR, 400 MHz, DMSO-$d_6$ $\delta$ = 9.47 (s, 1H), 9.31 (d, J = 8.0 Hz, 1H), 9.24 (d, J = 2.0 Hz, 1H), 9.10 (s, 1H), 8.70 (dd, J = 2.4 Hz, 1H), 7.72 (s, 1H), 5.05 - 4.99 (m, 1H), 4.42 (q, J = 6.8 Hz, 2H), 3.88 (s, 3H), 3.13 - 3.05 (m, 2H), 1.37 (t, J = 7.2 Hz, 3H).<br>[M+H]$^+$: 410.1. |
| 85 | 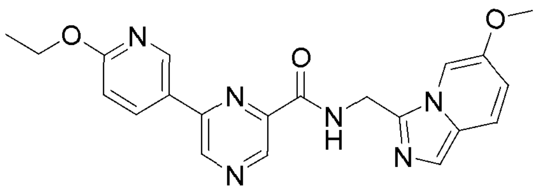 6-(6-ethoxypyridine-3-yl)-*N*-((6-methoxyimidazo[1,5-a]pyridine-3-yl)methyl)pyrazine-2-carboxamide | $^1$H-NMR, 400 MHz, DMSO-$d_6$ $\delta$ = 9.79 (br t, J = 6.1 Hz, 1H), 9.48 (s, 1H), 9.23 (d, J = 2.4 Hz, 1H), 9.12 (s, 1H), 8.73 (dd, J = 2.5, 8.8 Hz, 1H), 8.06 (s, 1H), 7.49 (d, J = 9.8 Hz, 1H), 7.30 (s, 1H), 6.96 (d, J = 8.6 Hz, 1H), 6.59 (dd, J = 1.9, 9.8 Hz, 1H), 4.95 (d, J = 6.1 Hz, 2H), 4.40 (q, J = 7.0 Hz, 2H), 3.78 (s, 3H), 1.35 (t, J = 7.1 Hz, 3H).<br>[M+H]$^+$: 405.2. |

(continued)

| Compound No | Structure | $^1$H-NMR / MS (m/z) [M+H]$^+$ |
|---|---|---|
| 86 | 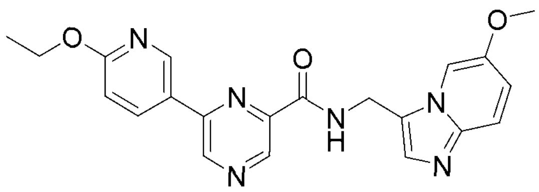 6-(6-ethoxypyridine-3-yl)-*N*-((6-methoxyimidazo[1,2-a]pyridine-3-yl)methyl)pyrazine-2-carboxamide | $^1$H-NMR, 400 MHz, DMSO-$d_6$ $\delta$ = 9.67 (br t, J = 5.8 Hz, 1H), 9.47 (s, 1H), 9.21 (d, J = 1.6 Hz, 1H), 9.11 (s, 1H), 8.69 (dd, J = 2.2, 8.7 Hz, 1H), 8.32 (s, 1H), 7.56 - 7.45 (m, 2H), 7.03 (dd, J = 1.8, 9.6 Hz, 1H), 6.97 (d, J = 8.8 Hz, 1H), 4.89 (br d, J = 6.0 Hz, 2H), 4.40 (q, J = 7.1 Hz, 2H), 3.83 (s, 3H), 1.35 (t, J = 7.0 Hz, 3H) . [M+H]$^+$: 405.1. |
| 87 | 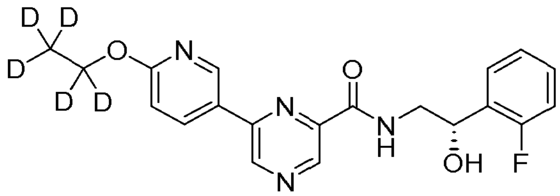 (*S*)-6-(6-(ethoxy-$d_5$) pyridine-3-yl)-*N*-(2-(2-fluorophenyl)-2-hydroxyethyl)pyrazine-2-carboxamide | $^1$H-NMR, 400 MHz, DMSO-$d_6$ $\delta$ = 9.46 (s, 1H), 9.19 - 9.03 (m, 3H), 8.67(d, 1H), 7.57 - 7.24 (t, J = 7.9 Hz, 2H), 7.10 (d, J = 8.8 Hz, 3H), 5.70 (m, 1H), 5.15 (q, 1H), 3.72 (s, 3H), 3.63 - 3.53 (m, 1H), 3.49 (t, J = 7.4 Hz, 4H). [M+H]$^+$: 387.2. |
| 88 | 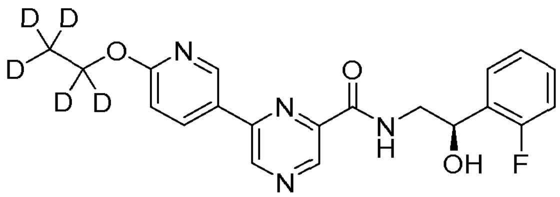 (*R*)-6-(6-(ethoxy-$d_5$) pyridine-3-yl)-*N*-(2-(2-fluorophenyl)-2-hydroxyethyl)pyrazine-2-carboxamide | $^1$H-NMR, 400 MHz, DMSO-$d_6$ $\delta$ = 9.47 (s, 1H), 9.19 - 9.03 (m, 3H), 8.67(d, 1H), 7.57 - 7.24 (t, J = 7.9 Hz, 2H), 7.10 (d, J = 8.8 Hz, 3H), 5.70 (m, 1H), 5.15 (q, 1H), 3.72 (s, 3H), 3.63 - 3.53 (m, 1H), 3.49 (t, J = 7.4 Hz, 4H). [M+H]$^+$: 387.2. |
| 89 | 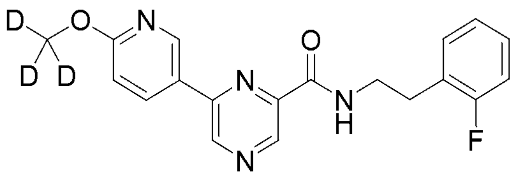 *N*-(2-fluorophenethyl)-6-(6-(methoxy-$d_3$)pyridine-3-yl)pyrazine-2-carboxamide | $^1$H-NMR, 400 MHz, DMSO-$d_6$ $\delta$ = 9.48 (s, 1H), 9.24 (s, 1H), 9.24 - 9.19 (m, 1H), 9.07 (s, 1H), 8.73 - 8.67 (m, 1H), 7.39 - 7.32 (m, 1H), 7.32 - 7.24 (m, 1H), 7.21 - 7.12 (m, 2H), 7.05 - 7.00 (m, 1H), 3.63 - 3.56 (m, 2H), 2.96 (t, J = 7.4 Hz, 2H). [M+H]$^+$: 356.1. |
| 90 | 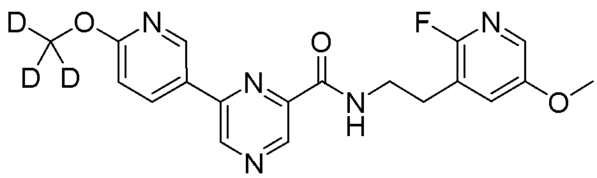 *N*-(2-(2-fluoro-5-methoxypyridine-3-yl)ethyl)-6-(6-(methoxy-$d_3$)pyridine-3-yl)pyrazine-2-carboxamide | $^1$H-NMR, 400 MHz, DMSO-$d_6$ $\delta$ = 9.47 (s, 1H), 9.23 - 9.18 (m, 2H), 9.05 (s, 1H), 8.68 (dd, J = 2.5, 8.8 Hz, 1H), 7.76 - 7.69 (m, 1H), 7.49 (dd, J = 3.0, 8.0 Hz, 1H), 7.06 - 6.96 (m, 1H), 3.75 (s, 3H), 3.62 (q, J = 6.8 Hz, 2H), 2.92 (t, J = 6.9 Hz, 2H). [M+H]$^+$: 387.2. |

(continued)

| Compound No | Structure | $^1$H-NMR / MS (m/z) [M+H]$^+$ |
|---|---|---|
| 91 | 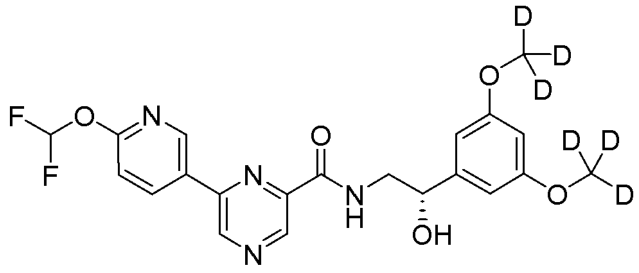 (*S*)-*N*-(2-(3,5-bis(methoxy-$d_3$)phenyl)-2-hydroxyethyl)-6-(6-(difluoromethoxy)pyridine-3-yl)pyrazine-2-carboxamide | $^1$H-NMR, 400 MHz, DMSO-$d_6$ $\delta$ = 9.56 (s, 1H), 9.27 (d, J = 2.1 Hz, 1H), 9.15 (s, 1H), 9.01 (t, J = 5.9 Hz, 1H), 8.87 (dd, J = 2.4, 8.7 Hz, 1H), 7.83 (t, J = 72.5 Hz, 1H), 7.32 (d, J = 8.6 Hz, 1H), 6.56 (d, J = 2.3 Hz, 2H), 6.38 (t, J = 2.3 Hz, 1H), 5.77 - 5.45 (m, 1H), 4.79 (dd, J = 4.5, 7.7 Hz, 1H), 3.61 - 3.40 (m, 2H). [M+H]$^+$: 453.2. |
| 92 | 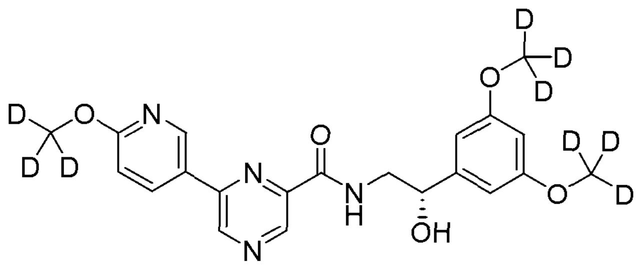 (*S*)-N-(2-(3,5-bis(methoxy-$d_3$)phenyl)-2-hydroxyethyl)-6-(6-(methoxy-$d_3$)pyridine-3-yl)pyrazine-2-carboxamide | $^1$H-NMR, 400 MHz, DMSO-$d_6$ $\delta$ = 99.48 (s, 1H), 9.19 (d, J = 2.3 Hz, 1H), 9.09 (s, 1H), 8.94 (t, J = 5.9 Hz, 1H), 8.65 (dd, J = 2.5, 8.7 Hz, 1H), 7.02 (d, J = 8.7 Hz, 1H), 6.56 (d, J = 2.3 Hz, 2H), 6.38 (t, J = 2.3 Hz, 1H), 5.61 (d, J = 4.5 Hz, 1H), 4.87 - 4.73 (m, 1H), 3.64 - 3.39 (m, 2H) . [M+H]$^+$: 420.2. |
| 93 | 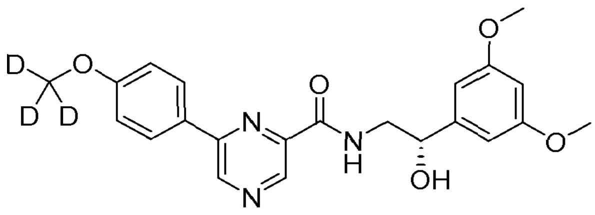 (*S*)-*N*-(2-(3,5-dimethoxyphenyl)-2-hydroxyethyl)-6-(4-(methoxy-$d_3$)phenyl)pyrazine-2-carboxamide | $^1$H-NMR, 400 MHz, DMSO-$d_6$ $\delta$ = 9.42 (s, 1H), 9.03 (s, 1H), 8.81 (s, 1H), 8.25 - 8.32 (m, 2H), 7.15 - 7.08 (m, 2H), 6.58 (d, J = 2.25 Hz, 2H), 6.40 (t, J = 2.25 Hz, 1H), 5.71 - 5.57 (m, 1H), 4.80 (dd, J = 7.57, 4.57 Hz, 1H), 3.71 (s, 6H), 3.63 - 3.54 (m, 1H), 3.46 (ddd, J = 13.32, 7.63, 5.82 Hz, 1H). [M+H]$^+$: 413.2. |
| 94 | 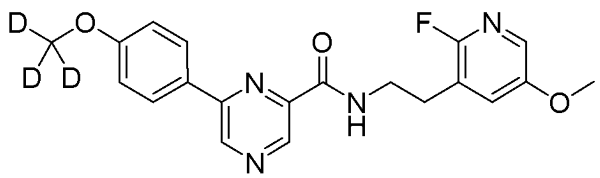 *N*-(2-(2-fluoro-5-methoxypyridine-3-yl)ethyl)-6-(4-(methoxy-$d_3$)phenyl)pyrazine-2-carboxamide | $^1$H-NMR, 400 MHz, DMSO-$d_6$ $\delta$ = 9.40 (s, 1H), 9.12 (t, J = 6.0 Hz, 1H), 8.99 (s, 1H), 8.36 - 8.31 (m, 2H), 7.76 - 7.70 (m, 1H), 7.49 (dd, J = 3.0, 8.0 Hz, 1H), 7.11 (s, 1H), 7.13 - 7.06 (m, 1H), 3.76 (s, 3H), 3.63 (q, J = 6.9 Hz, 2H), 3.32 (s, 3H), 2.92 (t, J = 7.0 Hz, 2H), 2.50 (s, 5H). [M+H]$^+$: 386.1. |
| 95 | 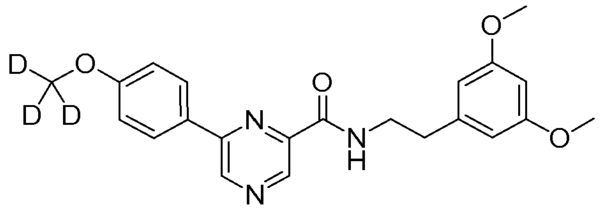 *N*-(3,5-dimethoxyphenethyl)-6-(4-(methoxy-$d_3$)phenyl)pyrazine-2-carboxamide | $^1$H-NMR, 400 MHz, DMSO-$d_6$ $\delta$ = 9.42 (s, 1H), 9.03 (s, 1H), 9.02 - 8.98 (m, 1H), 8.37 - 8.29 (m, 2H), 7.15 - 7.07 (m, 2H), 6.45 (d, J = 2.1 Hz, 2H), 6.36 (t, J = 2.3 Hz, 1H), 3.71 (s, 6H), 3.64 - 3.55 (m, 2H), 2.85 (t, J = 7.4 Hz, 2H). [M+H]$^+$: 397.2. |

(continued)

| Compound Nº | Structure | $^1$H-NMR / MS (m/z) [M+H]$^+$ |
|---|---|---|
| 96 | 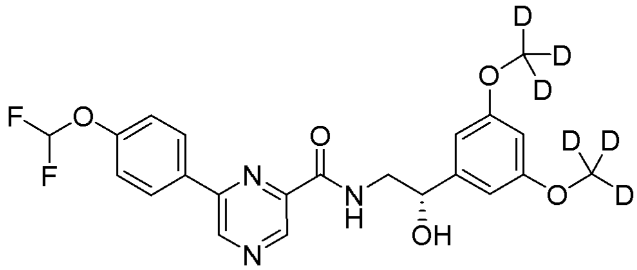 (*S*)-*N*-(2-(3,5-bis(methoxy-d$_3$)phenyl)-2-hydroxyethyl)-6-(4-(difluoromethoxy)phenyl)pyrazine-2-carboxamide | $^1$H-NMR, 400 MHz, DMSO-d$_6$ $\delta$ = 9.49 (s, 1H), 9.11 (s, 1H), 8.87 (t, J = 5.8 Hz, 1H), 8.41 (d, J = 8.8 Hz, 2H), 7.65 - 7.20 (m, 3H), 6.57 (d, J = 2.3 Hz, 2H), 6.39 (t, J = 2.2 Hz, 1H), 5.63 (d, J = 4.4 Hz, 1H), 4.89 - 4.72 (m, 1H), 3.62 - 3.42 (m, 2H). [M+H]$^+$: 452.2 |
| 97 | 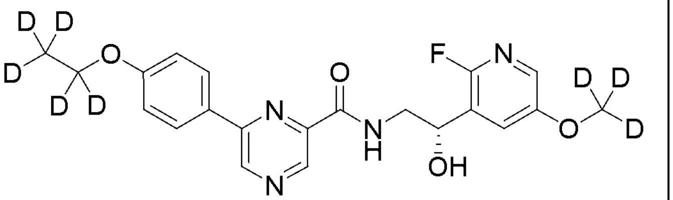 (*S*)-6-(4-(ethoxy-d$_5$)phenyl)-*N*-(2-(2-fluoro-5-(methoxy-d$_3$)pyridine-3-yl)-2-hydroxyethyl)pyrazine-2-carboxamide | $^1$H-NMR, 400 MHz, DMSO-d$_6$ $\delta$ = 3.51 - 3.68 (m, 2 H), 5.04 (br d, J=6.50 Hz, 1 H), 5.91 (d, J=5.00 Hz, 1 H), 7.09 (d, J=8.88 Hz, 2 H), 7.60 (dd, J=7.69, 3.06 Hz, 1 H), 7.77 - 7.80 (m, 1 H), 8.30 (d, J=8.75 Hz, 2 H), 8.94 - 8.98 (m, 1 H), 8.99 (s, 1 H), 9.40 (s, 1 H). [M+H]$^+$: 420.2. |
| 98 | 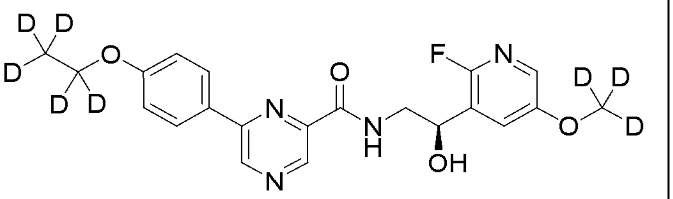 (*R*)-6-(4-(ethoxy-d$_5$)phenyl)-*N*-(2-(2-fluoro-5-(methoxy-d$_3$)pyridine-3-yl)-2-hydroxyethyl)pyrazine-2-carboxamide | $^1$H-NMR, 400 MHz, DMSO-d$_6$ $\delta$ = 3.52 - 3.70 (m, 2 H), 5.00 - 5.07 (m, 1 H), 5.91 (d, J=5.00 Hz, 1 H), 7.09 (d, J=8.88 Hz, 2 H), 7.60 (dd, J=7.75, 3.00 Hz, 1 H), 7.76 - 7.81 (m, 1 H), 8.30 (d, J=8.88 Hz, 2 H), 8.94 - 8.97 (m, 1 H), 8.99 (s, 1 H), 9.40 (s, 1 h). [M+H]$^+$: 420.2. |
| 99 | 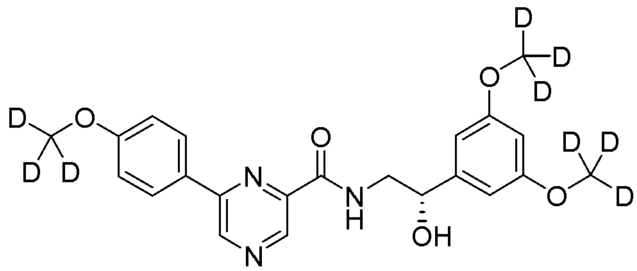 (S)-*N*-(2-(3,5-bis(methoxy-d$_3$)phenyl)-2-hydroxyethyl)-6-(4-(methoxy-d$_3$)phenyl)pyrazine-2-carboxamide | $^1$H-NMR, 400 MHz, DMSO-d$_6$ $\delta$ = 9.42 (s, 1H), 9.03 (s, 1H), 8.81 (br s, 1H), 8.29 (br d, J = 8.6 Hz, 2H), 7.11 (br d, J = 8.6 Hz, 2H), 6.57 (br d, J = 1.0 Hz, 2H), 6.39 (br s, 1H), 5.64 (br d, J = 4.3 Hz, 1H), 4.84 - 4.74 (m, 1H), 3.67 - 3.42 (m, 2H). [M+H]$^+$: 419.2. |
| 100 | 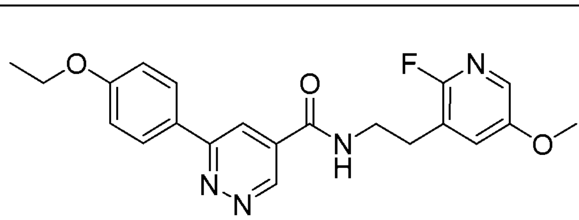 6-(4-ethoxyphenyl)-*N*-(2-(2-fluoro-5-methoxypyridine-3-yl)ethyl)pyridazine-4-carboxamide | $^1$H-NMR, 400 MHz, DMSO-d$_6$ $\delta$ = 9.35 (s, 1H), 9.13 (br t, J = 5.4 Hz, 1H), 8.35 (s, 1H), 8.14 (d, J = 8.5 Hz, 2H), 7.75 (br s, 1H), 7.53 (dd, J = 2.7, 7.8 Hz, 1H), 7.13 (d, J = 8.5 Hz, 2H), 4.14 (q, J = 6.9 Hz, 2H), 3.78 (s, 3H), 3.59 (q, J = 6.4 Hz, 2H), 2.89 (br t, J = 6.9 Hz, 2H), 1.37 (t, J = 6.9 Hz, 3H). [M+H]$^+$: 397.2. |

(continued)

| Compound №| Structure | $^1$H-NMR / MS (m/z) $[M+H]^+$ |
|---|---|---|
| 101 | 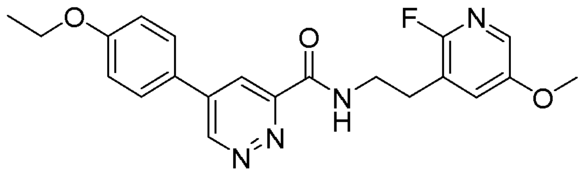 5-(4-ethoxyphenyl)-*N*-(2-(2-fluoro-5-methoxypyridine-3-yl) ethyl)pyridazine-3-carboxamide | $^1$H-NMR, 400 MHz, DMSO-$d_6$ $\delta$ = 9.77 (d, J = 2.4 Hz, 1H), 9.43 (t, J = 6.1 Hz, 1H), 8.30 (d, J = 2.4 Hz, 1H), 7.97 (d, J = 8.9 Hz, 2H), 7.74 - 7.68 (m, 1H), 7.49 (dd, J = 3.0, 8.1 Hz, 1H), 7.12 (d, J = 8.8 Hz, 2H), 4.13 (q, J = 7.0 Hz, 2H), 3.76 (s, 3H), 3.65 (q, J = 6.7 Hz, 2H), 2.93 (t, J = 6.8 Hz, 2H), 1.36 (t, J = 6.9 Hz, 3H). $[M+H]^+$: 397.1. |
| 102 | 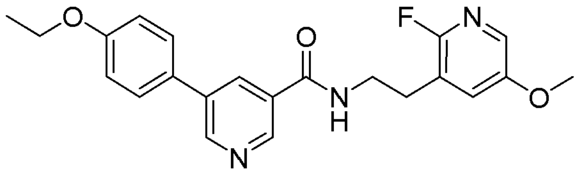 5-(4-ethoxyphenyl)-*N*-(2-(2-fluoro-5-methoxypyridine-3-yl) ethyl)nicotinamide | $^1$H-NMR, 400 MHz, DMSO-$d_6$ $\delta$ = 8.98 (d, J = 1.9 Hz, 1H), 8.89 - 8.80 (m, 2H), 8.31 (s, 1H), 7.75 (br s, 1H), 7.72 (d, J = 8.6 Hz, 2H), 7.55 - 7.48 (m, 1H), 7.08 (d, J = 8.5 Hz, 2H), 4.10 (q, J = 7.0 Hz, 2H), 3.78 (s, 3H), 3.57 (q, J = 6.3 Hz, 2H), 2.88 (t, J = 6.8 Hz, 2H), 1.36 (t, J = 6.9 Hz, 3H). $[M+H]^+$: 396.1. |
| 103 | 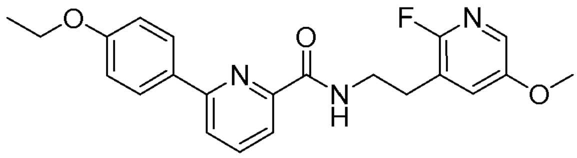 6-(4-ethoxyphenyl)-*N*-(2-(2-fluoro-5-methoxypyridine-3-yl) ethyl)picolinamide | $^1$H-NMR, 400 MHz, DMSO-$d_6$ $\delta$ = 8.96 (t, J = 5.9 Hz, 1H), 8.26 - 8.19 (m, 2H), 8.09 (dd, J = 0.8, 8.0 Hz, 1H), 8.00 (t, J = 7.8 Hz, 1H), 7.90 - 7.85 (m, 1H), 7.76 - 7.72 (m, 1H), 7.50 (dd, J = 3.0, 8.0 Hz, 1H), 7.08 - 7.01 (m, 2H), 4.13 (q, J = 7.0 Hz, 2H), 3.76 (s, 3H), 3.64 (q, J = 6.8 Hz, 2H), 2.93 (t, J = 6.9 Hz, 2H), 1.38 (t, J = 7.0 Hz, 3H). $[M+H]^+$: 396.2. |
| 104 | 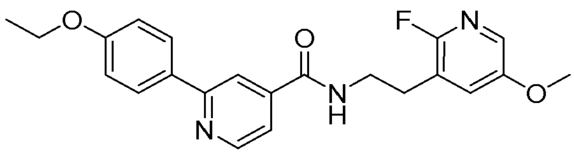 2-(4-ethoxyphenyl)-*N*-(2-(2-fluoro-5-methoxypyridine-3-yl) ethyl)isonicotinamide | $^1$H-NMR, 400 MHz, DMSO-$d_6$ $\delta$ = 8.93 (t, J = 5.5 Hz, 1H), 8.73 (d, J = 5.0 Hz, 1H), 8.14 (s, 1H), 8.10 - 8.03 (m, 2H), 7.77 - 7.73 (m, 1H), 7.57 (dd, J = 1.4, 5.0 Hz, 1H), 7.51 (dd, J = 3.1, 8.1 Hz, 1H), 7.07 (d, J = 8.9 Hz, 2H), 4.12 (q, J = 6.9 Hz, 2H), 3.79 (s, 3H), 3.58 (q, J = 6.6 Hz, 2H), 2.89 (t, J = 6.8 Hz, 2H), 1.37 (t, J = 6.9 Hz, 3H). $[M+H]^+$: 396.2. |

(continued)

| Compound № | Structure | $^1$H-NMR / MS (m/z) [M+H]$^+$ |
|---|---|---|
| 105 | 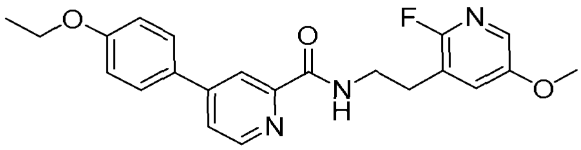 4-(4-ethoxyphenyl)-*N*-(2-(2-fluoro-5-methoxypyridine-3-yl) ethyl)picolinamide | $^1$H-NMR, 400 MHz, DMSO-$d_6$ $\delta$ = 8.97 (t, J = 6.1 Hz, 1H), 8.63 (d, J = 5.3 Hz, 1H), 8.21 (d, J = 1.5 Hz, 1H), 7.87 (dd, J = 1.9, 5.2 Hz, 1H), 7.81 (d, J = 8.8 Hz, 2H), 7.73 (t, J = 2.4 Hz, 1H), 7.47 (dd, J = 3.0, 8.0 Hz, 1H), 7.09 (d, J = 8.9 Hz, 2H), 4.11 (q, J = 6.9 Hz, 2H), 3.77 (s, 3H), 3.61 (q, J = 6.8 Hz, 2H), 2.90 (t, J = 6.8 Hz, 2H), 1.36 (t, J = 6.9 Hz, 3H). [M+H]$^+$: 395.9. |
| 106 | 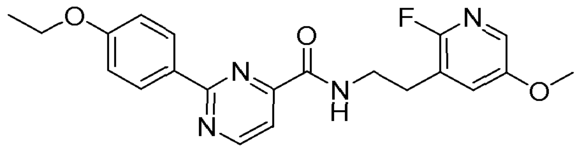 2-(4-ethoxyphenyl)-*N*-(2-(2-fluoro-5-methoxypyridine-3-yl) ethyl)pyrimidine-4-carboxamide | $^1$H-NMR, 400 MHz, DMSO-$d_6$ $\delta$ = 9.27 (t, J = 6.1 Hz, 1H), 9.05 (d, J = 5.0 Hz, 1H), 8.54 (d, J = 8.9 Hz, 2H), 7.78 (d, J = 4.9 Hz, 1H), 7.75 (t, J = 2.4 Hz, 1H), 7.50 (dd, J = 3.1, 8.1 Hz, 1H), 7.11 - 7.04 (m, 2H), 4.15 (q, J = 7.0 Hz, 2H), 3.76 (s, 3H), 3.63 (q, J = 6.5 Hz, 2H), 2.93 (t, J = 7.0 Hz, 2H), 1.38 (t, J = 6.9 Hz, 3H). [M+H]$^+$: 397.2. |
| 107 | 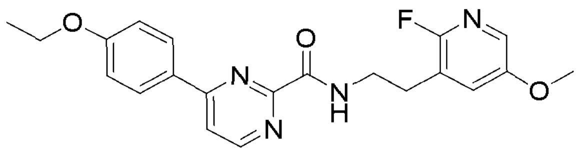 4-(4-ethoxyphenyl)-*N*-(2-(2-fluoro-5-methoxypyridine-3-yl) ethyl)pyrimidine-2-carboxamide | $^1$H-NMR, 400 MHz, DMSO-$d_6$ $\delta$ = 9.07 (t, J = 5.9 Hz, 1H), 8.89 (d, J = 5.4 Hz, 1H), 8.32 (d, J = 8.9 Hz, 2H), 8.13 (d, J = 5.4 Hz, 1H), 7.74 (t, J = 2.3 Hz, 1H), 7.51 (dd, J = 2.9, 8.1 Hz, 1H), 7.10 (d, J = 8.8 Hz, 2H), 4.15 (q, J = 7.0 Hz, 2H), 3.78 (s, 3H), 3.62 (q, J = 6.5 Hz, 2H), 2.92 (t, J = 6.9 Hz, 2H), 1.38 (t, J = 6.9 Hz, 3H). [M+H]$^+$: 397.1. |
| 108 | 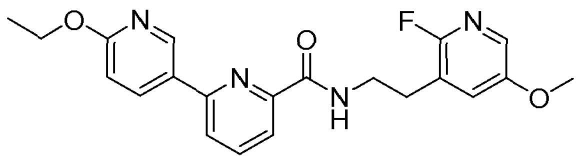 6'-ethoxy-*N*-(2-(2-fluoro-5-methoxypyridine-3-yl)ethyl)-[2,3'-bi-pyridine]-6-carboxamide | $^1$H-NMR, 400 MHz, DMSO-$d_6$ $\delta$ = 9.13 (d, J = 2.3 Hz, 1H), 9.07 (br t, J = 6.1 Hz, 1H), 8.59 (dd, J = 2.5, 8.8 Hz, 1H), 8.15 (d, J = 7.4 Hz, 1H), 8.03 (t, J = 7.8 Hz, 1H), 7.95 - 7.85 (m, 1H), 7.78 - 7.68 (m, 1H), 7.48 (dd, J = 3.0, 8.1 Hz, 1H), 6.92 (d, J = 8.8 Hz, 1H), 4.39 (q, J = 7.0 Hz, 2H), 3.79 - 3.71 (m, 3H), 3.70 - 3.56 (m, 2H), 3.33 (s, 2H), 2.92 (t, J = 7.0 Hz, 2H), 1.36 (t, J = 7.1 Hz, 3H). [M+H]$^+$: 397.1. |

(continued)

| Compound № | Structure | $^1$H-NMR / MS (m/z) [M+H]$^+$ |
|---|---|---|
| 109 | 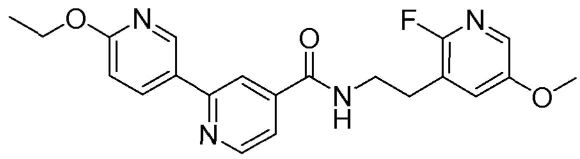 6'-ethoxy-*N*-(2-(2-fluoro-5-methoxypyridine-3-yl)ethyl)-[2,3'-bi-pyridine]-4-carboxamide | $^1$H-NMR, 400 MHz, DMSO-$d_6$ $\delta$ = 8.92 (br t, J = 5.7 Hz, 1H), 8.88 (d, J = 2.1 Hz, 1H), 8.77 (d, J = 5.1 Hz, 1H), 8.37 (dd, J = 2.5, 8.7 Hz, 1H), 8.20 (s, 1H), 7.81 - 7.70 (m, 1H), 7.62 (dd, J = 1.4, 5.0 Hz, 1H), 7.50 (dd, J = 3.0, 8.1 Hz, 1H), 6.94 (d, J = 8.6 Hz, 1H), 4.38 (q, J = 7.1 Hz, 2H), 3.78 (s, 3H), 3.58 (q, J = 6.7 Hz, 2H), 2.88 (t, J = 6.9 Hz, 2H), 1.35 (t, J = 7.1 Hz, 3H). [M+H]$^+$: 397.2. |
| 110 | 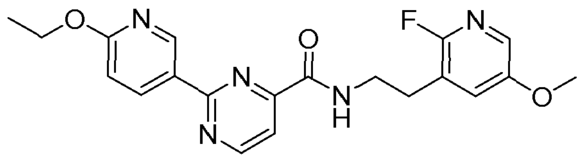 2-(6-ethoxypyridine-3-yl)-*N*-(2-(2-fluoro-5-methoxypyridine-3-yl)ethyl)pyrimidine-4-carboxamide | $^1$H-NMR, 400 MHz, DMSO-$d_6$ $\delta$ = 9.40 (d, J = 2.3 Hz, 1H), 9.35 (br t, J = 6.0 Hz, 1H), 9.08 (d, J = 5.0 Hz, 1H), 8.78 (dd, J = 2.4, 8.8 Hz, 1H), 7.84 (d, J = 5.0 Hz, 1H), 7.73 (t, J = 2.3 Hz, 1H), 7.49 (dd, J = 2.9, 8.0 Hz, 1H), 6.95 (d, J = 8.8 Hz, 1H), 4.42 (q, J = 7.1 Hz, 2H), 3.76 (s, 3H), 3.62 (q, J = 6.8 Hz, 2H), 2.92 (t, J = 6.9 Hz, 2H), 1.36 (t, J = 7.1 Hz, 3H). [M+H]$^+$: 398.1. |
| 111 | 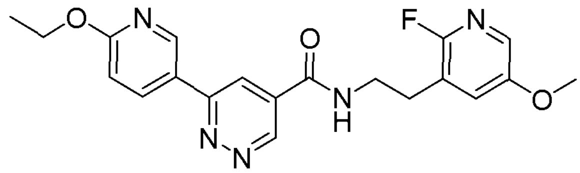 6-(6-ethoxypyridine-3-yl)-*N*-(2-(2-fluoro-5-methoxypyridine-3-yl)ethyl)pyridazine-4-carboxamide | $^1$H-NMR, 400 MHz, DMSO-$d_6$ $\delta$ = 9.41 (d, J = 2.0 Hz, 1H), 9.13 (t, J = 5.6 Hz, 1H), 8.96 (d, J = 2.4 Hz, 1H), 8.49 (dd, J = 6.0, 2.8 Hz, 1H), 8.43 (d, J = 2.0 Hz, 1H), 7.76 (t, J = 2.8 Hz, 1H), 7.55 (dd, J = 4.8, 3.2 Hz, 1H), 7.03 (d, J = 8.8 Hz, 1H), 4.43 (q, J = 7.2 Hz, 2H), 3.79 (s, 3H), 3.62 (q, J = 6.4 Hz, 2H), 2.90 (t, J = 6.8 Hz, 2H), 1.38 (t, J = 7.2 Hz, 3H). [M+H]$^+$: 398.2. |
| 112 | 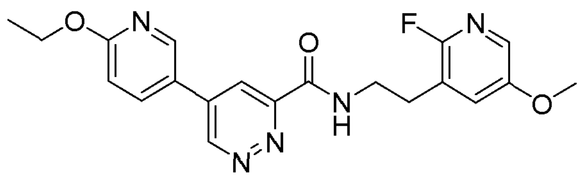 5-(6-ethoxypyridine-3-yl)-*N*-(2-(2-fluoro-5-methoxypyridine-3-IL)ethyl)pyridazine-3-carboxamide | $^1$H-NMR, 400 MHz, DMSO-$d_6$ $\delta$ = 9.82 (d, J = 2.4 Hz, 1H), 9.48 (t, J = 6.0 Hz, 1H), 8.83 (d, J = 2.8 Hz, 1H), 8.39 (d, J = 2.4 Hz, 1H), 8.36 (dd, J = 6.0, 2.8 Hz, 1H), 7.73 (t, J = 2.4 Hz, 1H), 7.50 (dd, J = 5.2, 3.2 Hz, 1H), 7.00 (d, J = 8.8 Hz, 1H), 4.42 (q, J = 6.8 Hz, 2H), 3.76 (s, 3H), 3.68 (q, J = 6.8 Hz, 2H), 2.94 (t, J = 6.8 Hz, 2H), 1.37 (t, J = 6.8 Hz, 3H). [M+H]$^+$: 398.2. |

(continued)

| Compound № | Structure | $^1$H-NMR / MS (m/z) [M+H]$^+$ |
|---|---|---|
| 113 | 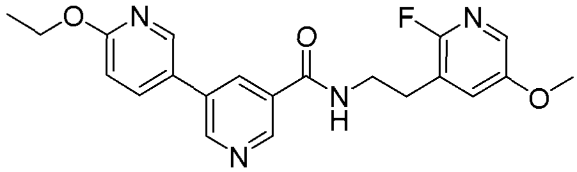 6'-ethoxy-*N*-(2-(2-fluoro-5-methoxypyridine-3-yl)ethyl)-[3,3'-bi-pyridine]-5-carboxamide | $^1$H-NMR, 400 MHz, DMSO-$d_6$ $\delta$ = 9.02 (d, J = 2.1 Hz, 1H), 8.90 (d, J = 2.0 Hz, 1H), 8.83 (br t, J = 5.7 Hz, 1H), 8.58 (d, J = 2.4 Hz, 1H), 8.36 (t, J = 2.1 Hz, 1H), 8.12 (dd, J = 2.6, 8.6 Hz, 1H), 7.78 - 7.71 (m, 1H), 7.51 (dd, J = 3.0, 8.0 Hz, 1H), 6.96 (d, J = 8.6 Hz, 1H), 4.37 (q, J = 7.0 Hz, 2H), 3.78 (s, 3H), 3.57 (q, J = 6.6 Hz, 2H), 2.88 (t, J = 6.8 Hz, 2H), 1.35 (t, J = 7.1 Hz, 3H). [M+H]$^+$: 397.1. |
| 114 | 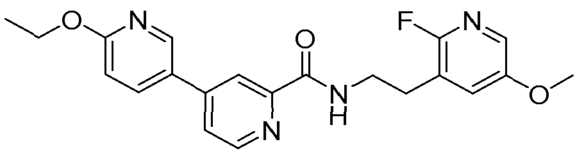 6-ethoxy-*N*-(2-(2-fluoro-5-methoxypyridine-3-yl)ethyl)-[3,4'-bi-pyridine]-2'-carboxamide | $^1$H-NMR, 400 MHz, DMSO-$d_6$ $\delta$ = 8.98 (t, J = 6.1 Hz, 1H), 8.73 - 8.61 (m, 2H), 8.23 (d, J = 1.4 Hz, 1H), 8.18 (dd, J = 2.6, 8.6 Hz, 1H), 7.92 (dd, J = 1.9, 5.3 Hz, 1H), 7.71 (t, J = 2.4 Hz, 1H), 7.46 (dd, J = 3.0, 8.0 Hz, 1H), 6.94 (d, J = 8.6 Hz, 1H), 4.37 (q, J = 7.1 Hz, 2H), 3.76 (s, 3H), 3.61 (q, J = 6.7 Hz, 2H), 2.89 (t, J = 6.9 Hz, 2H), 1.34 (t, J = 7.0 Hz, 3H). [M+H]$^+$: 397.1. |
| 115 | 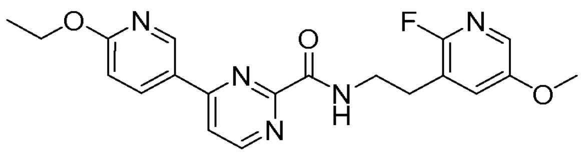 4-(6-ethoxypyridine-3-yl)-*N*-(2-(2-fluoro-5-methoxypyridine-3-yl)ethyl)pyrimidine-2-carboxamide | $^1$H-NMR, 400 MHz, DMSO-$d_6$ $\delta$ = 9.21 (d, J = 2.1 Hz, 1H), 9.16 (br t, J = 6.0 Hz, 1H), 8.96 (d, J = 5.4 Hz, 1H), 8.64 (dd, J = 2.5, 8.8 Hz, 1H), 8.21 (d, J = 5.4 Hz, 1H), 7.80 - 7.67 (m, 1H), 7.49 (dd, J = 2.9, 8.1 Hz, 1H), 6.99 (d, J = 8.6 Hz, 1H), 4.42 (q, J = 7.1 Hz, 2H), 3.77 (s, 3H), 3.60 (q, J = 6.7 Hz, 2H), 2.91 (t, J = 6.9 Hz, 2H), 1.36 (t, J = 7.1 Hz, 3H). [M+H]$^+$: 398.1. |

**EXAMPLE 8. TESTS *IN VITRO:***

Biological assays were performed on Chinese hamster ovary (CHO) cells that express human sodium channels Nav 1.8 or Nav 1.7 in a stable manner.

The experimental protocol *voltage-clamp* using the whole cell configuration was established on the automated ScreenPatch® 384P platform (SP384PE, Nanion Technologies, Livingston, NJ) and the results were recorded with a Nanion 384-well Patch Clamp chip (NPC) (Nanion Technologies, Livingston, NJ).

Compounds of Formula Ia and Formula Ib were diluted in eight concentrations in the extracellular solution composed of saline solution, buffered with HEPES (mM): NaCl, 137; KCl, 4; $CaCl_2$, 3,8; $MgCl_2$, 1; HEPES, 10; Glucose, 10; pH 7.4. The extracellular solution is composed of (mM) CsCl, 50; CsF, 90; $MgCl_2$, 5; EGTA, 5; HEPES, 10; pH 7.2. The duration of exposure of each compound with cells expressing Nav 1.7 or Nav 1.8 was at least five minutes, and the assays were performed at room temperature.

The measurements of the sodium currents of Nav 1.8 and Nav 1.7 were obtained using the voltage protocol described below:

A holding voltage of -100 mV was established followed by an inactivation voltage step at -40 mV for 8 seconds, followed by a step of -100 mV for 20 ms, followed by a 20 ms step for 10 mV for Nav 1.8 or 0 mV for Nav 1.7 (TP1A) before returning to

the holding voltage of -100 mV.

The protocol was repeated at a frequency of 0.05 Hz and the amplitude of the current was quantified throughout the recording of the TP1A phase. The variation in the amplitude of the peak current was evaluated according to the formula described below, after exposing the cells expressing the channels to each concentration of the different compounds:

$$\% \text{ Block} = (1 - (I_{TP1A,molecule} / I_{TP1A,basal}) \times 100\%,$$

wherein $I_{TP1A,basal}$ and $I_{TP1A,molecule}$ represent the peaks of sodium current input into TP1A before exposure to the compound and in the presence of the compound, respectively.

The decrease in the amplitude of the peak current, after the exposure of the cells to the compounds, was used to calculate the percentage of relative blockage of the channels in relation to the positive control according to the formula below:

$$\% \text{ Block'} = 100\% - ((\% \text{ Block} - \% \text{ CP}) * (100\% / (\%V - \% \text{ CP})),$$

wherein %V and %CP represent the means of the values of current inhibition with a vehicle (DMSO) and positive controls, respectively. The sodium currents of the positive control were considered as 100%.

$$\% \text{ Block'} = (100\% / [1 + ([\text{Test}] / CI_{50})^N],$$

where [Test] represents the concentration of the molecule evaluated, $CI_{50}$ is the concentration of the compound that generates half of the maximum inhibition, N is the Hill coefficient, and % Block' is the percentage of sodium channel current (Nav 1.8 and Nav 1.7) blocked at each concentration of the molecule evaluated. Data were obtained by nonlinear regression *(nonlinear least squares)* with XLfit for Excel (Microsoft, Redmond, WA).

The compounds were tested in at least one assay to obtain the value of $CI_{50}$. For compounds that were tested in two or more assays, the results are described as the mean of the $CI_{50}$ values.

Table 18 shows the efficacy *in vitro* of selected compounds against Nav1.8 and Nav1.7. $CI_{50}$ values less than 500 nM are represented with the legend (+++); $CI_{50}$ values between 500 nM and 1000 nM are represented with the legend (++), $CI_{50}$ values greater than 1000 nM are demonstrated with the symbology (+).

demonstrated with the symbology (+).

**TABLE 18. $CI_{50}$ VALUES OF THE COMPOUNDS OF THIS INVENTION IN NAV 1.8 AND NAV 1.7 CHANNELS.**

| Compound № | Nav1.8 $IC_{50}$ (nM) | Nav1.7 $IC_{50}$ (nM) |
|---|---|---|
| 1 | ++ | + |
| 2 | +++ | ++ |
| 3 | +++ | + |
| 4 | +++ | +++ |
| 5 | +++ | +++ |
| 6 | +++ | +++ |
| 7 | + | + |
| 8 | + | + |
| 9 | + | + |
| 10 | +++ | ++ |
| 11 | + | + |
| 12 | +++ | + |
| 13 | +++ | + |
| 14 | + | + |
| 15 | +++ | + |
| 16 | +++ | + |
| 17 | +++ | ++ |

(continued)

| Compound № | Nav1.8 $IC_{50}$ (nM) | Nav1.7 $IC_{50}$ (nM) |
|---|---|---|
| 18 | +++ | + |
| 22 | +++ | + |
| 24 | + | + |
| 25 | + | + |
| 26 | + | + |
| 27 | +++ | +++ |
| 28 | +++ | ++ |
| 29 | +++ | ++ |
| 30 | + | + |
| 31 | + | + |
| 32 | +++ | +++ |
| 33 | +++ | ++ |
| 34 | +++ | ++ |
| 39 | +++ | +++ |
| 40 | + | + |
| 41 | + | + |
| 42 | +++ | + |
| 43 | +++ | ++ |
| 44 | + | + |
| 45 | +++ | ++ |
| 47 | + | + |
| 48 | + | + |
| 49 | + | + |
| 50 | + | + |
| 51 | + | + |
| 52 | +++ | + |
| 53 | +++ | ++ |
| 54 | +++ | + |
| 55 | +++ | + |
| 56 | + | + |
| 57 | + | + |
| 58 | + | + |
| 59 | + | + |
| 60 | +++ | ++ |
| 61 | + | + |
| 62 | + | + |
| 63 | + | + |
| 64 | + | + |
| 65 | + | + |

(continued)

| Compound № | Nav1.8 $IC_{50}$ (nM) | Nav1.7 $IC_{50}$ (nM) |
|---|---|---|
| 66 | + | + |
| 67 | + | + |
| 68 | + | + |
| 69 | +++ | + |
| 70 | + | + |
| 71 | + | + |
| 72 | + | + |
| 73 | + | + |
| 74 | +++ | ++ |
| 75 | + | + |
| 76 | + | + |
| 77 | + | + |
| 78 | +++ | + |
| 79 | ++ | ++ |
| 80 | +++ | + |
| 81 | +++ | + |
| 82 | ++ | + |
| 83 | + | + |
| 84 | +++ | + |
| 85 | + | + |
| 86 | + | + |
| 87 | + | + |
| 88 | +++ | + |
| 89 | +++ | + |
| 90 | + | + |
| 91 | +++ | + |
| 92 | + | + |
| 93 | +++ | + |
| 94 | +++ | + |
| 95 | +++ | + |
| 96 | +++ | + |
| 97 | + | + |
| 98 | +++ | + |
| 99 | ++ | + |
| 100 | + | + |
| 101 | + | + |
| 102 | + | + |
| 103 | + | + |
| 104 | + | + |

(continued)

| Compound № | Nav1.8 $IC_{50}$ (nM) | Nav1.7 $IC_{50}$ (nM) |
|---|---|---|
| 105 | + | +++ |
| 106 | + | + |
| 107 | ++ | + |
| 108 | + | + |
| 109 | + | + |
| 110 | + | + |
| 111 | + | + |
| 112 | + | + |
| 113 | + | + |
| 114 | + | + |
| 115 | + | + |

From these results, the blocking activity of Nav 1.7 and/or 1.8 is proven, whose application is readily performed by those skilled in the art in pharmaceutical compositions, which may comprise one or more of the aforementioned Formula (Ia) or Formula (Ib) compounds, kits, in addition to uses in the treatment of pain-related pathologies.

In particular, these results indicate the possibility of using Formula (Ia) or Formula (Ib) compounds in the preparation of drugs for the treatment of conditions such as peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia, and postsurgical neuralgia.

It should be understood that the embodiments described above are merely illustrative and that several modifications can be made by a person skilled in the art without departing from the scope of this invention. Consequently, the present invention should not be regarded as being limited to the illustrative specifications described in this application.

**Claims**

**1.** COMPOUND, **characterized by** being of Formula (Ia)

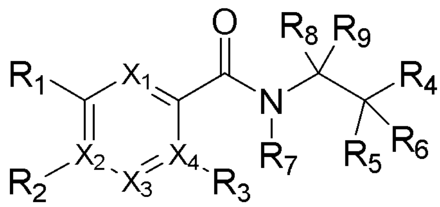

Formula (Ia)

or a pharmaceutically acceptable salt, hydrate, solvate, ester and isomer thereof, where:

- $X_1$, $X_2$, $X_3$, and $X_4$ are independently selected from the group consisting of carbon, CH, or nitrogen, wherein
- when at least one of the substituents $X_2$, $X_4$ is nitrogen or CH, the corresponding R ($R_2$, $R_3$) is null,
- when two of the substituents $X_1$, $X_2$, $X_3$ and $X_4$ are nitrogen, the remaining substituents $X_1$, $X_2$, $X_3$ or $X_4$ must be carbon or CH;
- $R_1$ is:

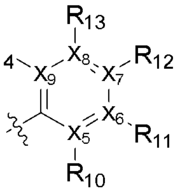

,

wherein

- $X_5$, $X_6$, $X_7$, $X_8$, and $X_9$ are independently selected from the group consisting of carbon, CH, or nitrogen, wherein
- when at least one of the substituents $X_5$-$X_9$ is nitrogen or CH, the corresponding R ($R_{10}$-$R_{14}$) is null,
- $R_{10}$, $R_{11}$, $R_{12}$, $R_{13}$, and $R_{14}$ are independently selected from the group consisting of hydrogen, halogen, linear or branched $C_1$-$C_6$ alkyl, alkoxy $C_1$-$C_6$ linear or branched, hydroxy, hydroxyalkyl $C_1$-$C_4$, haloalkoxy $C_1$-$C_6$ linear or branched, $-OCD_2CD_3$ or $-OCD_3$;
- $R_2$ and $R_3$ are independently selected from the group consisting of hydrogen, halogen, amine, linear or branched $C_1$-$C_6$ alkoxy, and linear or branched $C_1$-$C_6$ alkyl;
- $R_4$ is:

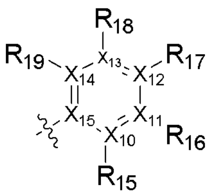

or

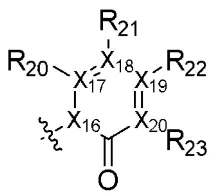

,

wherein:

- $X_{10}$, $X_{11}$, $X_{12}$, $X_{13}$, $X_{14}$, $X_{15}$, $X_{16}$, $X_{17}$, $X_{18}$, $X_{19}$ and $X_{20}$ are independently selected from the group consisting of carbon, CH or nitrogen, where when at least one of the substituents $X_{10}$, $X_{11}$, $X_{12}$, $X_{13}$, $X_{14}$, $X_{17}$, $X_{18}$, $X_{19}$ or $X_{20}$ is nitrogen or CH, the corresponding R ($R_{15-23}$) is null;
- $R_{13}$, $R_{16}$, $R_{17}$, $R_{18}$, $R_{19}$, $R_{20}$, $R_{21}$, $R_{22}$, $R_{23}$ are independently selected from the group consisting of hydrogen, halogen, oxygen, $C_1$-$C_6$ alkyl linear or branched, $C_1$-$C_6$ linear or branched alkoxy, $C_1$-$C_6$ linear or branched haloalkoxy, $C_1$-$C_4$ linear or branched hydroxyalkyl, $-OCD_2CD_3$ or $-OCD_3$;
- $R_5$ and $R_6$ are independently selected from the group consisting of hydrogen, hydroxy, carbonyl, amine, alkyl $C_1$-$C_6$ linear or branched, where when $R_5$ or $R_6$ are carbonyl, the other R must be null;
- or $R_4$, $R_5$, and $R_6$ together form one of the following bicycles:

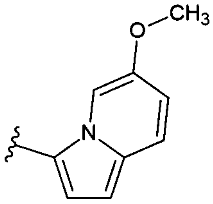

,

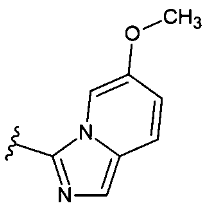

or

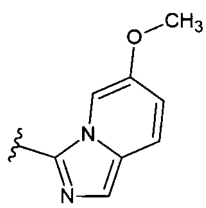

;

and

- $R_7$, $R_8$, and $R_9$ are independently selected from the group consisting of hydrogen and linear or branched alkyl $C_1$-$C_6$.

**2.** COMPOUND, **characterized by** being of Formula (Ib)

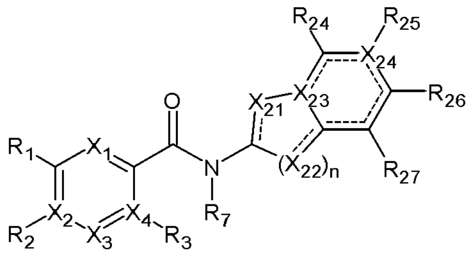

Formula (Ib)

or a pharmaceutically acceptable salt, hydrate, solvate, ester and isomer thereof, where:

- $X_1$, $X_2$, $X_3$, and $X_4$ are independently selected from the group consisting of carbon, CH, or nitrogen, wherein
- when at least one of the substituents $X_2$, $X_4$ is nitrogen or CH, the corresponding R ($R_2$, $R_3$) is null,
- when two of the substituents $X_1$, $X_2$, $X_3$ and $X_4$ are nitrogen, the remaining substituents $X_1$, $X_2$, $X_3$ or $X_4$ must be carbon or CH;
- $R_1$ is:

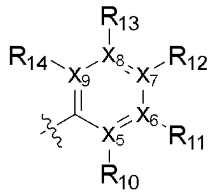

,

wherein

- $X_5$, $X_6$, $X_7$, $X_8$, and $X_9$ are independently selected from the group consisting of carbon, CH, or nitrogen, wherein
- when at least one of the substituents $X_5$-$X_9$ is nitrogen or CH, the corresponding R ($R_{10}$-$R_{14}$) is null,
- $R_{10}$, $R_{11}$, $R_{12}$, $R_{13}$, and $R_{14}$ are independently selected from the group consisting of hydrogen, halogen, linear or branched $C_1$-$C_6$ alkyl, alkoxy $C_1$-$C_6$ linear or branched, hydroxyalkyl $C_1$-$C_4$, haloalkoxy $C_1$-$C_6$ linear or branched, $-OCD_2CD_3$ or $-OCD_3$;
- $R_2$ and $R_3$ are independently selected from the group consisting of hydrogen, halogen, amine, $C_1$-$C_6$ linear or branched alkoxy, and $C_1$-$C_6$ linear or branched alkyl;
- $R_7$ is selected from a group consisting of hydrogen and $C_1$-$C_6$ linear or branched alkyl;
- $X_{21}$, $X_{22}$, $X_{23}$, and $X_{24}$ are independently selected from the group consisting of carbon, CH, $CH_2$, nitrogen, oxygen, sulfur, or $NCH_3$ wherein
- when $X_{24}$ is nitrogen or CH, $R_{25}$ is null;
- n is 1 or 2; and
- $R_{24}$, $R_{25}$, $R_{26}$, and $R_{27}$ are independently selected from the groups consisting of hydrogen, halogen, and alkoxy $C_1$-$C_6$ linear or branched, or null when the respective $X_{21}$, $X_{22}$, $X_{23}$, or $X_{24}$ is CH, $CH_2$, nitrogen, oxygen, or sulfur, or $NCH_3$.

**3.** COMPOUND, according to claims 1 or 2, **characterized by** being selected from the group consisting of:

*N*-(3,5-dimethoxyphenethyl)-6-(4-isopropoxyphenyl)pyrazine-2-carboxamide (Compound 1);
6-(4-(difluoromethoxy)phenyl)-*N*-(3,5-dimethoxyphenethyl)pyrazine-2-carboxamide (Compound 2);
*N*-(3,5-dimethoxyphenethyl)-6-(4-ethoxy-2-fluorophenyl)pyrazine-2-carboxamide (Compound 3);
*N*-(3,5-dimethoxyphenethyl)-6-(4-ethoxy-2-methylphenyl)pyrazine-2-carboxamide (Compound 4);
6-(4-ethoxyphenyl)-*N*-(3-methoxyphenethyl)pyrazine-2-carboxamide (Compound 5);
6-(4-ethoxyphenyl)-*N*-(2-fluorophenethyl)pyrazine-2-carboxamide (Compound 6);
6-(4-ethoxyphenyl)-*N*-(2-(2-oxopyridine-1(2H)-yl)ethyl)pyrazine-2-carboxamide (Compound 7);
6-(4-ethoxyphenyl)-*N*-(2-(5-methoxy-2-oxopyridine-1(2H)-yl)ethyl)pyrazine-2-carboxamide (Compound 8);
6-(4-ethoxyphenyl)-*N*-(2-(2-fluorophenyl)-2-oxoethyl)pyrazine-2-carboxamide (Compound 9);
*N*-(3,5-dimethoxyphenethyl)-6-(4-ethoxyphenyl)pyrazine-2-carboxamide (Compound 10);
6-(4-ethoxyphenyl)-*N*-(2-(2-fluoro-5-methoxyphenyl)-2-oxoethyl)pyrazine-2-carboxamide (Compound 11);
6-(4-ethoxyphenyl)-*N*-(3-(1-hydroxyethyl)-5-methoxyphenethyl)pyrazine-2-carboxamide (Compound 12);

6-(4-ethoxyphenyl)-*N*-(3-(1-hydroxyethyl)phenethyl)pyrazine-2-carboxamide (Compound 13);
6-(4-ethoxyphenyl)-*N*-(2-(2-fluoro-5-(1-hydroxyethyl)pyridine-3-yl)ethyl)pyrazine-2-carboxamide (Compound 14);
6-(4-ethoxyphenyl)-*N*-(2-fluoro-5-(1-hydroxyethyl)phenethyl)pyrazine-2-carboxamide (Compound 15);
6-(4-ethoxyphenyl)-*N*-(2-(2-fluoro-5-methoxypyridin-3-yl)ethyl)pyrazine-2-carboxamide (Compound 16);
6-(4-ethoxyphenyl)-*N*-(2-fluoro-5-methoxyphenethyl)pyrazine-2-carboxamide (Compound 17);
6-(4-ethoxyphenyl)-*N*-(2-(5-methoxy-2-methylpyridine-3-yl)ethyl)pyrazine-2-carboxamide (Compound 18);
6-(4-ethoxyphenyl)-*N*-(5-methoxy-2-methylphenethyl)pyrazine-2-carboxamide (Compound 19);
(*S*)-*N*-(1-(3,5-dimethoxyphenyl)propan-2-yl)-6-(4-ethoxyphenyl)pyrazine-2-carboxamide (Compound 20);
(*R*)-*N*-(1-(3,5-dimethoxyphenyl)propan-2-yl)-6-(4-ethoxyphenyl)pyrazine-2-carboxamide (Compound 21);
(*R*)-*N*-(2-(3,5-dimethoxyphenyl)propyl)-6-(4-ethoxyphenyl)pyrazine-2-carboxamide (Compound 22);
(*S*)-*N*-(2-(3,5-dimethoxyphenyl)propyl)-6-(4-ethoxyphenyl)pyrazine-2-carboxamide (Compound 23);
(*R*)-*N*-(2-(3,5-dimethoxyphenyl)-2-hydroxyethyl)-6-(4-ethoxyphenyl)pyrazine-2-carboxamide (Compound 24);
(*S*)-*N*-(2-(3,5-dimethoxyphenyl)-2-hydroxyethyl)-6-(4-ethoxyphenyl)pyrazine-2-carboxamide (Compound 25);
(*R*)-*N*-(2-amino-2-(3,5-dimethoxyphenyl)ethyl)-6-(4-ethoxyphenyl)pyrazine-2-carboxamide (Compound 26);
(*S*)-*N*-(2-amino-2-(3,5-dimethoxyphenyl)ethyl)-6-(4-ethoxyphenyl)pyrazine-2-carboxamide (Compound 27);
*N*-(3,5-dimethoxyphenethyl)-6-(6-isopropoxypyridin-3-yl)pyrazine-2-carboxamide (Compound 28);
6-(6-(difluoromethoxy)pyridine-3-yl)-*N*-(3,5-dimethoxyphenethyl)pyrazine-2-carboxamide (Compound 29);
*N*-(3,5-dimethoxyphenethyl)-6-(6-ethoxy-4-methylpyridine-3-yl)pyrazine-2-carboxamide (Compound 30);
*N*-(3,5-dimethoxyphenethyl)-6-(6-ethoxy-2-methylpyridine-3-yl)pyrazine-2-carboxamide (Compound 31);
6-(6-ethoxypyridine-3-yl)-*N*-(2-(2-oxopyridine-1(2H)-yl)ethyl)pyrazine-2-carboxamide (Compound 32);
6-(6-ethoxypyridine-3-yl)-*N*-(2-(5-methoxy-2-oxopyridine-1(2H)-yl)ethyl)pyrazine-2-carboxamide (Compound 33);
6-(6-ethoxypyridine-3-yl)-*N*-(2-(2-fluoro-5-methoxypyridine-3-yl)ethyl)pyrazine-2-carboxamide (Compound 34);
6-(6-ethoxypyridine-3-yl)-*N*-(2-fluoro-5-methoxyphenethyl)pyrazine-2-carboxamide (Compound 35);
6-(6-ethoxypyridine-3-yl)-*N*-(2-(5-methoxy-2-methylpyridine-3-yl)ethyl)pyrazine-2-carboxamide (Compound 36);
6-(6-ethoxypyridine-3-yl)-*N*-(5-methoxy-2-methylphenethyl)pyrazine-2-carboxamide (Compound 37);
6-(6-ethoxypyridine-3-yl)-*N*-(2-(2-fluorophenyl)-2-oxoethyl)pyrazine-2-carboxamide (Compound 38);
6-(6-ethoxypyridine-3-yl)-*N*-(2-(2-fluorophenyl)-2-oxoethyl)pyrazine-2-carboxamide (Compound 39);
6-(6-ethoxypyridine-3-yl)-*N*-(3-(1-hydroxyethyl)-5-methoxyphenethyl)pyrazine-2-carboxamide (Compound 40);
6-(6-ethoxypyridine-3-yl)-*N*-(3-(1-hydroxyethyl)phenethyl)pyrazine-2-carboxamide (Compound 41);
6-(6-ethoxypyridine-3-yl)-*N*-(2-(2-fluoro-5-(1-hydroxyethyl)pyridine-3-yl)ethyl)pyrazine-2-carboxamide (Compound 42);
6-(6-ethoxypyridine-3-yl)-*N*-(2-fluoro-5-(1-hydroxyethyl)phenethyl)pyrazine-2-carboxamide (Compound 43);
*N*-(3,5-dimethoxyphenethyl)-6-(6-ethoxypyridine-3-yl)pyrazine-2-carboxamide (Compound 44);
6-(6-ethoxypyridine-3-yl)-*N*-(3-methoxyphenethyl)pyrazine-2-carboxamide (Compound 45);
6-(6-ethoxypyridine-3-yl)-*N*-(2-fluorophenethyl)pyrazine-2-carboxamide (Compound 46);
6-(6-(difluoromethoxy)pyridine-3-yl)-*N*-(2-fluorophenethyl)pyrazine-2-carboxamide (Compound 47);
*N*-(2-fluorophenethyl)-6-(6-methoxypyridine-3-yl)pyrazine-2-carboxamide (Compound 48);
*N*-(3,5-dimethoxyphenethyl)-6-(4-methoxyphenyl)pyrazine-2-carboxamide (Compound 49);
6-(4-(difluoromethoxy)phenyl)-*N*-(2-(2-fluoro-5-methoxypyridine-3-yl)ethyl)pyrazine-2-carboxamide (Compound 50);
6-(6-(difluoromethoxy)pyridine-3-yl)-*N*-(2-(2-fluoro-5-methoxypyridine-3-yl)ethyl)pyrazine-2-carboxamide (Compound 51);
*N*-(2-(2-fluoro-5-methoxypyridine-3-yl)ethyl)-6-(4-methoxyphenyl)pyrazine-2-carboxamide (Compound 52);
*N*-(2-(2-fluoro-5-methoxypyridine-3-yl)ethyl)-6-(6-methoxypyridine-3-yl)pyrazine-2-carboxamide (Compound 53);
(*S*)-6-(4-(difluoromethoxy)phenyl)-N-(2-(3,5-dimethoxyphenyl)-2-hydroxyethyl)pyrazine-2-carboxamide (Compound 54);
(*S*)-N-(2-(3,5-dimethoxyphenyl)-2-hydroxyethyl)-6-(4-methoxyphenyl)pyrazine-2-carboxamide (Compound 55);
*N*-(2-(2-fluoro-5-methoxypyridine-3-yl)ethyl)-6-(4-(2-hydroxypropane-2-yl)phenyl)pyrazine-2-carboxamide (Compound 56);
*N*-(2-(2-fluoro-5-methoxypyridine-3-yl)ethyl)-6-(6-(2-hydroxypropane-2-yl)pyridine-3-yl)pyrazine-2-carboxamide (Compound 57);

*N*-(2-(2-fluoro-5-methoxypyridine-3-yl)ethyl)-6-(6-(1-hydroxyethyl)pyridine-3-yl)pyrazine-2-carboxamide (Compound 58);
N-(2-(2-fluoro-5-methoxypyridine-3-yl)ethyl)-6-(4-(1-hydroxyethyl)phenyl)pyrazine-2-carboxamide (Compound 59);
6-(4-ethoxyphenyl)-N-(4-methoxy-1H-indole-2-yl)pyrazine-2-carboxamide (Compound 60);
6-(6-ethoxypyridine-3-yl)-N-(4-fluoro-7-methoxybenzo[d]thiazole-2-yl)pyrazine-2-carboxamide (Compound 61);
6-(6-ethoxypyridine-3-yl)-N-(7-fluoro-4-methoxybenzo[d]oxazole-2-yl)pyrazine-2-carboxamide (Compound 62);
6-(6-ethoxypyridine-3-yl)-N-(5-fluoro-8-methoxyimidazo[1,2-a]pyridine-2-yl)pyrazine-2-carboxamide (Compound 63);
6-(6-ethoxypyridine-3-yl)-N-(4-fluoro-7-methoxy-1H-benzo[d]imidazole-2-yl)pyrazine-2-carboxamide (Compound 64);
6-(6-ethoxypyridine-3-yl)-N-(4-fluoro-7-methoxybenzo[d]oxazole-2-yl)pyrazine-2-carboxamide (Compound 65);
6-(6-ethoxypyridine-3-yl)-N-(7-fluoro-4-methoxy-1-methyl-1H-benzo[d]imidazole-2-yl)pyrazine-2-carboxamide (Compound 66);
6-(6-ethoxypyridine-3-yl)-N-(4-fluoro-7-methoxyoxazole[4,5-c]pyridine-2-yl)pyrazine-2-carboxamide (Compound 67);
6-(6-ethoxypyridine-3-yl)-N-(8-fluoro-5-methoxyimidazo[1,2-a]pyridine-2-yl)pyrazine-2-carboxamide (Compound 68);
6-(6-ethoxypyridine-3-yl)-N-(4-fluoro-7-methoxy-1H-indole-2-yl)pyrazine-2-carboxamide (Compound 69);
6-(6-ethoxypyridine-3-yl)-N-(4-fluoro-7-methoxy-1-methyl-1H-indole-2-yl)pyrazine-2-carboxamide (Compound 70);
6-(6-ethoxypyridine-3-yl)-N-(4-fluoro-7-methoxy-1H-indole-2-yl)-N-methylpyrazine-2-carboxamide (Compound 71);
6-(6-ethoxypyridine-3-yl)-N-(7-methoxy-1H-indole-2-yl)pyrazine-2-carboxamide (Compound 72);
6-(6-ethoxypyridine-3-yl)-N-(4-methoxy-1H-indole-2-yl)pyrazine-2-carboxamide (Compound 73);
6-(6-ethoxypyridine-3-yl)-N-(7-fluoro-4-methoxy-1H-indole-2-yl)pyrazine-2-carboxamide (Compound 74);
6-(6-ethoxypyridine-3-yl)-*N*-(7-fluoro-4-methoxy-1-methyl-1H-indole-2-yl)pyrazine-2-carboxamide (Compound 75);
6-(4-ethoxyphenyl)-*N*-(4-fluoro-7-methoxy-1H-indole-2-yl)pyrazine-2-carboxamide (Compound 76);
6-(4-ethoxyphenyl)-*N*-(7-fluoro-4-methoxy-1H-indole-2-yl)pyrazine-2-carboxamide (Compound 77);
(S)-6-(6-ethoxypyridine-3-yl)-*N*-(8-fluoro-5-methoxy-1,2,3,4-tetrahydronaphthalene-2-yl)pyrazine-2-carboxamide (Compound 78);
(*R*)-6-(6-ethoxypyridine-3-yl)-*N*-(8-fluoro-5-methoxy-1,2,3,4-tetrahydronaphthalene-2-yl)pyrazine-2-carboxamide (Compound 79);
6-(6-ethoxypyridine-3-yl)-*N*-((6-methoxyindolizine-3-yl)methyl)pyrazine-2-carboxamide (Compound 80);
(S)-6-(6-ethoxypyridine-3-yl)-*N*-(4-fluoro-7-methoxy-2,3-dihydro-1*H*-inden-2-yl)pyrazine-2-carboxamide (Compound 81);
(R)-6-(6-ethoxypyridine-3-yl)-*N*-(4-fluoro-7-methoxy-2,3-dihydro-1H-inden-2-yl)pyrazine-2-carboxamide (Compound 82);
(S)-6-(6-ethoxypyridine-3-yl)-*N*-(1-fluoro-4-methoxy-6,7-dihydro-5*H*-cyclopenta[c]pyridine-6-yl)pyrazine-2-carboxamide (Compound 83);
(*R*)-6-(6-ethoxypyridine-3-yl)-*N*-(1-fluoro-4-methoxy-6,7-dihydro-5H-cyclopenta[c]pyridin-6-yl)pyrazine-2-carboxamide (Compound 84);
6-(6-ethoxypyridine-3-yl)-*N*-((6-methoxyimidazo[1,5-a]pyridine-3-yl)methyl)pyrazine-2-carboxamide (Compound 85);
6-(6-ethoxypyridine-3-yl)-*N*-((6-methoxyimidazo[1,2-a]pyridine-3-yl)methyl)pyrazine-2-carboxamide (Compound 86);
(*S*)-6-(6-(ethoxy-$d_5$)pyridine-3-yl)-*N*-(2-(2-fluorophenyl)-2-hydroxyethyl)pyrazine-2-carboxamide (Compound 87);
(*R*)-6-(6-(ethoxy-$d_5$)pyridine-3-yl)-*N*-(2-(2-fluorophenyl)-2-hydroxyethyl)pyrazine-2-carboxamide (Compound 88);
*N*-(2-fluorophenethyl)-6-(6-(methoxy-$d_3$)pyridine-3-yl)pyrazine-2-carboxamide (Compound 89);
*N*-(2-(2-fluoro-5-methoxypyridine-3-yl)ethyl)-6-(6-(methoxy-$d_3$)pyridine-3-yl)pyrazine-2-carboxamide (Compound 90);
(*S*)-*N*-(2-(3,5-bis(methoxy-$d_3$)phenyl)-2-hydroxyethyl)-6-(6-(difluoromethoxy)pyridine-3-yl)pyrazine-2-carboxamide (Compound 91);
(*S*)-*N*-(2-(3,5-bis(methoxy-$d_3$)phenyl)-2-hydroxyethyl)-6-(6-(methoxy-$d_3$)pyridine-3-yl)pyrazine-2-carboxamide (Compound 92);
(*S*)-*N*-(2-(3,5-dimethoxyphenyl)-2-hydroxyethyl)-6-(4-(methoxy-$d_3$)phenyl)pyrazine-2-carboxamide (Compound 93);
*N*-(2-(2-fluoro-5-methoxypyridine-3-yl)ethyl)-6-(4-(methoxy-$d_3$)phenyl)pyrazine-2-carboxamide (Compound 94);
*N*-(3,5-dimethoxyphenethyl)-6-(4-(methoxy-$d_3$)phenyl)pyrazine-2-carboxamide (Compound 95);
(*S*)-N-(2-(3,5-bis(methoxy-$d_3$)phenyl)-2-hydroxyethyl)-6-(4-(difluoromethoxy)phenyl)pyrazine-2-carboxamide (Compound 96);
(*S*)-6-(4-(ethoxy-$d_5$)phenyl)-N-(2-(2-fluoro-5-(methoxy-$d_3$)pyridine-3-yl)-2-hydroxyethyl)pyrazine-2-carboxamide (Compound 97);
(*R*)-6-(4-(ethoxy-$d_5$)phenyl)-*N*-(2-(2-fluoro-5-(methoxy-$d_3$)pyridine-3-yl)-2-hydroxyethyl)pyrazine-2-carboxamide (Compound 98);
(*S*)-*N*-(2-(3,5-bis(methoxy-$d_3$)phenyl)-2-hydroxyethyl)-6-(4-(methoxy-$d_3$)phenyl)pyrazine-2-carboxamide (compound 99);
6-(4-ethoxyphenyl)-*N*-(2-(2-fluoro-5-methoxypyridine-3-yl)ethyl)pyridazine-4-carboxamide (Compound 100);
5-(4-ethoxyphenyl)-*N*-(2-(2-fluoro-5-methoxypyridine-3-yl)ethyl)pyridazine-3-carboxamide (Compound 101);
5-(4-ethoxyphenyl)-*N*-(2-(2-fluoro-5-methoxypyridine-3-yl)ethyl)nicotinamide (Compound 102);
6-(4-ethoxyphenyl)-*N*-(2-(2-fluoro-5-methoxypyridine-3-yl)ethyl)picolinamide (Compound 103);
2-(4-ethoxyphenyl)-*N*-(2-(2-fluoro-5-methoxypyridine-3-yl)ethyl)isonicotinamide (Compound 104);
4-(4-ethoxyphenyl)-*N*-(2-(2-fluoro-5-methoxypyridine-3-yl)ethyl)picolinamide (Compound 105);
2-(4-ethoxyphenyl)-*N*-(2-(2-fluoro-5-methoxypyridine-3-yl)ethyl)pyrimidine-4-carboxamide (Compound 106);
4-(4-ethoxyphenyl)-N-(2-(2-fluoro-5-methoxypyridine-3-yl)ethyl)pyrimidine-2-carboxamide (Compound 107);
6'-ethoxy-N-(2-(2-fluoro-5-methoxypyridine-3-yl)ethyl)-[2,3'-bipyridine]-6-carboxamide (Compound 108);
6'-ethoxy-N-(2-(2-fluoro-5-methoxypyridine-3-yl)ethyl)-[2,3'-bipyridine]-4-carboxamide (Compound 109);
2-(6-ethoxypyridine-3-yl)-N-(2-(2-fluoro-5-methoxypyridine-3-yl)ethyl)pyrimidine-4-carboxamide (Compound 110);
6-(6-ethoxypyridine-3-yl)-N-(2-(2-fluoro-5-methoxypyridine-3-yl)ethyl)pyridazine-4-carboxamide (Compound 111);
5-(6-ethoxypyridine-3-yl)-N-(2-(2-fluoro-5-methoxypyridine-3-yl)ethyl)pyridazine-3-carboxamide (Compound 112);
6'-ethoxy-N-(2-(2-fluoro-5-methoxypyridine-3-yl)ethyl)-[3,3'-bipyridine]-5-carboxamide (Compound 113);
6-ethoxy-N-(2-(2-fluoro-5-methoxypyridine-3-yl)ethyl)-[3,4'-bipyridine]-2'-carboxamide (Compound 114), and
4-(6-ethoxypyridine-3-yl)-N-(2-(2-fluoro-5-methoxypyridine-3-yl)ethyl)pyrimidine-2-carboxamide (Compound 115).

4. COMPOUND, according to claim 1 or 2, **characterized by** being a blocker of voltage-gated sodium channels Nav 1.7 and/or Nav 1.8.

5. PHARMACEUTICAL COMPOSITION, **characterized by** comprising a therapeutically effective amount of one or more compounds of Formula (I) or of a pharmaceutically acceptable salt, hydrate, solvate, and isomer thereof, as defined in any of claims 1 to 3, and one or more pharmaceutically acceptable excipients.

6. PHARMACEUTICAL COMPOSITION, according to claim 4, **characterized by** being formulated as an oral, sublingual, nasal, parenteral, injectable, submuscular, topical, transdermal, ocular, or rectal composition.

7. USE OF FORMULA (I) COMPOUND(S), as defined in any of claims 1 to 3, **characterized by** being to prepare a drug to treat pathologies related to neuropathic pain.

8. USE, according to claim 6, **characterized by** said pathologies being selected from the group consisting of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia, and postsurgical neuralgia.

9. METHOD OF TREATMENT, PREVENTION, RELIEF, SUPPRESSION AND/OR CONTROL of pathologies related to neuropathic pain, **characterized by the** administration of an effective amount of at least one compound of Formula (I) or a salt, hydrate, solvate and pharmaceutically acceptable isomer thereof, as defined in any of claims 1 to 3.

**10.** METHOD, according to claim 8, **characterized by** being for the treatment or prophylaxis of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia, and postsurgical neuralgia.

**11.** METHOD, according to claim 8 or 9, **characterized by** administration of at least one compound of Formula (I) being selected from the group comprising oral, sublingual, nasal, parenteral, injectable, submuscular, topical, transdermal, ocular, and rectal routes.

**12.** PROCESS OF OBTAINING GENERAL FORMULA COMPOUND (I), as defined in any of the claims 1 to 3, **characterized by** comprising the steps:

(a) Formation of the Formula III intermediate:

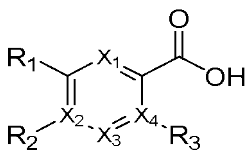

Formula III

from the basic hydrolysis reaction of a Formula IV intermediate:

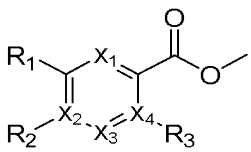

Formula IV

(b) obtaining Formula Ia and Formula Ib compounds:

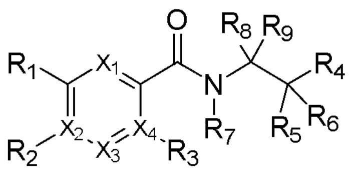

Formula (Ia)

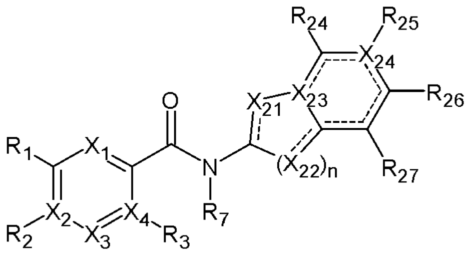

Formula (Ib)

from the amide coupling of Formula II intermediates:

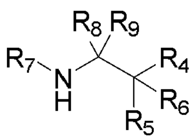

Formula II

and Formula III with or without the presence of a catalyst and an aprotic solvent;
wherein,

- $X_1$, $X_2$, $X_3$, and $X_4$ are independently selected from the group consisting of carbon, CH, or nitrogen, wherein
- when at least one of the substituents $X_2$, $X_4$ is nitrogen or CH, the corresponding R ($R_2$, $R_3$) is null,
- when two of the substituents $X_1$, $X_2$, $X_3$ and $X_4$ are nitrogen, the remaining substituents $X_1$, $X_2$, $X_3$ or $X_4$ must be carbon or CH;
- $R_1$ is:

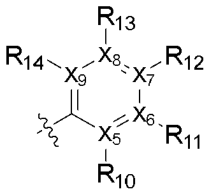
,

wherein

- $X_5$, $X_6$, $X_7$, $X_8$, and $X_9$ are independently selected from the group consisting of carbon, CH, or nitrogen, wherein
- when at least one of the substituents $X_5$-$X_9$ is nitrogen or CH, the corresponding R ($R_{10}$-$R_{14}$) is null,
- $R_{10}$, $R_{11}$, $R_{12}$, $R_{13}$, and $R_{14}$ are independently selected from the group consisting of hydrogen, halogen, linear or branched $C_1$-$C_6$ alkyl, alkoxy $C_1$-$C_6$ linear or branched, hydroxy, hydroxyalkyl $C_1$-$C_4$, haloalkoxy $C_1$-$C_6$ linear or branched, $-OCD_2CD_3$ or $-OCD_3$;
- $R_2$ and $R_3$ are independently selected from the group consisting of hydrogen, halogen, amine, $C_1$-$C_6$ linear or branched alkoxy, and $C_1$-$C_6$ linear or branched alkyl;
- $R_4$ is:

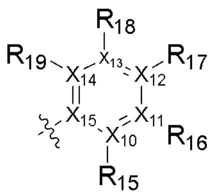
or
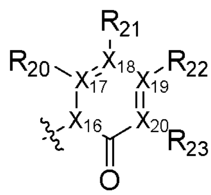
,

wherein

- $X_{10}$, $X_{11}$, $X_{12}$, $X_{13}$ $X_{14}$, $X_{15}$, $X_{16}$, $X_{17}$, $X_{18}$, $X_{19}$ and $X_{20}$ are independently selected from the group consisting of carbon, CH or nitrogen, wherein
- when at least one of the substituents $X_{10}$, $X_{11}$, $X_{12}$, $X_{13}$, $X_{14}$, $X_{17}$, $X_{18}$, $X_{19}$ or $X_{20}$ is N or CH, the corresponding R ($R_{15}$-$R_{23}$) is null;
- $R_{13}$, $R_{16}$, $R_{17}$, $R_{18}$, $R_{19}$, $R_{20}$, $R_{21}$, $R_{22}$, $R_{23}$ are independently selected from the group consisting of hydrogen, halogen, oxygen, alkyl $C_{10\ 1}$-$C_6$ linear or branched, $C_1$-$C_6$ linear or branched alkoxy, hydroxyalkyl $C_1$-$C_4$ linear or branched, - $OCD_2CD_3$ or $-OCD_3$;
- $R_5$ and $R_6$ are independently selected from the group consisting of hydrogen, hydroxy, carbonyl $C_1$-$C_6$, amine, $C_1$-$C_6$ linear or branched alkyl, where- when $R_5$ or $R_6$ are carbonyl, the other R must be null;
- or $R_4$, $R_5$, and $R_6$ together form one of the following bicycles:

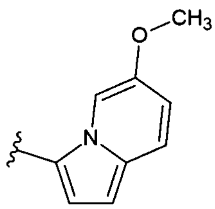
,
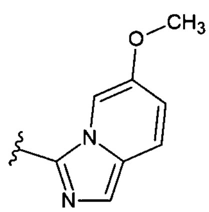
or
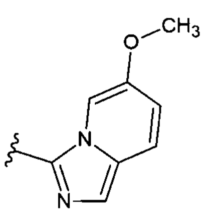
;

- $R_7$, $R_8$, and $R_9$ are independently selected from the group consisting of hydrogen and alkyl

$C_1$-$C_6$ linear or branched;
- $X_{21}$, $X_{22}$, $X_{23}$, and $X_{24}$ are independently selected from the group consisting of carbon, CH, $CH_2$, nitrogen, oxygen, sulfur, or $NCH_3$ wherein
- when $X_{24}$ is nitrogen or CH, $R_{25}$ is null;
- n is 1 or 2; and
- $R_{24}$, $R_{25}$, $R_{26}$, and $R_{27}$ are independently selected from the groups consisting of hydrogen, halogen, and alkoxy $C_1$-$C_6$, or null when the respective $X_{21}$, $X_{22}$, $X_{23}$, or $X_{24}$ is CH, $CH_2$, nitrogen, oxygen, or sulfur.

**13.** PROCESS, according to claim 11, **characterized by** further comprising a compound formation step (c) of Formula (I) where $R_7$ is linear or branched $C_{1-}C_6$ alkyl from the nucleophilic substitution reaction of a compound obtained in step (b), with linear or branched $C_{1-}C_6$ alkyl halides in the presence of inorganic base and polar aprotic solvents.

**14.** PROCESS, according to claim 11 or 12, **characterized by** in step (b) said catalyst being selected from concentrated hydrochloric acid, acetic acid, trifluoroacetic acid, formic acid, or combinations of these and said solvent being selected from dimethylformamide, dimethylsulfoxide, alcohols, or combinations thereof.

**15.** PROCESS, according to claim 12 or 13, **characterized by** in step (c) the said inorganic base being selected from among $K_2CO_3$ or NaH.

**16.** KIT, **characterized by** comprising a pharmaceutical composition, as defined in claim 5, and an application device.

**INTERNATIONAL SEARCH REPORT**

International application No.
**PCT/BR2024/050028**

**A. CLASSIFICATION OF SUBJECT MATTER**

***C07C 233/64***(2006.01)i; **C07C 15/00**(2006.01)i; ***C07C 15/12***(2006.01)i; **C07D 213/00**(2006.01)i; **C07D 401/00**(2006.01)i; **C07D 403/00**(2006.01)i; **A61K 31/166**(2006.01)i; **A61K 31/435**(2006.01)i; **A61K 31/495**(2006.01)i; ***A61K 31/609***(2006.01)i; ***A61P 25/02***(2006.01)i; ***A61P 25/04***(2006.01)i; **A61P 29/00**(2006.01)i
CPC: C07C 233/64, C07C 15/00, C07C 15/12, C07D 213/00, C07D 401/00; C07D 403/00, A61K 31/166, A61K 31/435, A61K 31/495, A61K 31/609; A61P 25/02, A61P 25/04, A61P 29/00

According to International Patent Classification (IPC) or to both national classification and IPC

**B. FIELDS SEARCHED**

Minimum documentation searched (classification system followed by classification symbols)

C07C 233/64; C07C 15/00; C07C 15/12; C07D 213/00; C07D 401/00; C07D 403/00; A61K 31/166; A61K 31/435; A61K 31/495; A61K 31/609; A61P 25/02; A61P 25/04; A61P 29/00
CPC: C07C 233/64, C07C 15/00, C07C 15/12, C07D 213/00, C07D 401/00; C07D 403/00, A61K 31/166, A61K 31/435, A61K 31/495, A61K 31/609; A61P 25/02, A61P 25/04, A61P 29/00

Documentation searched other than minimum documentation to the extent that such documents are included in the fields searched

Database INPI-BR; Periódicos Capes

Electronic data base consulted during the international search (name of data base and, where practicable, search terms used)

Derwent Innovation; REGISTRY, CAPLUS, MARPAT (STN®)

**C. DOCUMENTS CONSIDERED TO BE RELEVANT**

| Category* | Citation of document, with indication, where appropriate, of the relevant passages | Relevant to claim No. |
|---|---|---|
| X | Scanio MJ, Shi L, Drizin I, Gregg RJ, Atkinson RN, Thomas JB, Johnson MS, Chapman ML, Liu D, Krambis MJ, Liu Y, Shieh CC, Zhang X, Simler GH, Joshi S, Honore P, Marsh KC, Knox A, Werness S, Antonio B, Krafte DS, Jarvis MF, Faltynek CR, Marron BE, Kort ME. Discovery and biological evaluation of potent, selective, orally bioavailable, pyrazine-based blockers of the Na(v)1.8 sodium channel with efficacy in a model of neuropathic pain. Bioorg Med Chem. 2010 Nov 15;18(22):7816-25. doi: 10.1016/j.bmc.2010.09.057. Epub 2010 Sep 29. PMID: 20965738. See the whole document, especially compounds 18, 21 and 28. | 1-8, 12-16 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 April 2024** | **29 April 2024** |
| Name and mailing address of the ISA/BR<br>**National Institute of Industrial Property (Brazil)**<br>**Rua Mayrink Veiga, 9, 6º andar, CEP 20.090-910 Rio de Janeiro – RJ**<br>**Brazil** | Authorized officer<br>**Rodinelli OLIVEIRA** |
| Telephone No. **(55 21) 3037-3742, 3037-3984** | Telephone No. **+55 21 3037 4528 - 3037 3319** |

Form PCT/ISA/210 (second sheet) (July 2022)

**INTERNATIONAL SEARCH REPORT**

International application No.
**PCT/BR2024/050028**

**C. DOCUMENTS CONSIDERED TO BE RELEVANT**

| Category* | Citation of document, with indication, where appropriate, of the relevant passages | Relevant to claim No. |
|---|---|---|
| X | Owen DR, Dodd PG, Gayton S, Greener BS, Harbottle GW, Mantell SJ, Maw GN, Osborne SA, Rees H, Ringer TJ, Rodriguez-Lens M, Smith GF. Structure-activity relationships of novel non-competitive mGluR1 antagonists: a potential treatment for chronic pain. Bioorg Med Chem Lett. 2007 Jan 15;17(2):486-90. doi: 10.1016/j.bmcl.2006.10.015. Epub 2006 Oct 11. PMID: 17064898. See the whole document, especially compound 15. | 1-8, 12-16 |
| X | WO 2009058298 A1 (MERCK & CO INC [US]) 07 May 2009 (2009-05-07) See abstract and claims. | 1-8, 12-16 |
| X | WO 2008000645 A1 (HOFFMANN LA ROCHE [CH]) 03 January 2008 (2008-01-03) See abstract and claims. | 1-8, 12-16 |
| X | WO 2009077371 A1 (HOFFMANN LA ROCHE [CH]) 25 June 2009 (2009-06-25) See abstract and claims. | 1-8, 12-16 |
| X | WO 2006053109 A1 (SYNTA PHARMACEUTICALS CORP [US]) 18 May 2006 (2006-05-18) See abstract and claims. | 1-8, 12-16 |
| X | Liu M, Liang Y, Zhu Z, Wang J, Cheng X, Cheng J, Xu B, Li R, Liu X, Wang Y. Discovery of Novel Aryl Carboxamide Derivatives as Hypoxia-Inducible Factor 1α Signaling Inhibitors with Potent Activities of Anticancer Metastasis. J Med Chem. 2019 Oct 24;62(20):9299-9314. doi: 10.1021/acs.jmedchem.9b01313. Epub 2019 Oct 4. PMID: 31556611. See the whole document, especially compounds 27a and 27b. | 1-8, 12-16 |
| X | CN 104876912 A (SUZHOU YUNXUAN MEDICINE SCIENCE & TECHNOLOGY CO LTD) 02 September 2015 (2015-09-02) See abstract and claims. | 1-8, 12-16 |
| X | CN 110407824 A (UNIV ANHUI MEDICAL) 05 November 2019 (2019-11-05) See abstract and claims. | 1-8, 12-16 |
| X | WO 2012129562 A2 (SCRIPPS RESEARCH INST [US]) 27 September 2012 (2012-09-27) See abstract and claims. | 1-8, 12-16 |
| X | WO 2015048547 A2 (RIGEL PHARMACEUTICALS INC [US]) 02 April 2015 (2015-04-02) See abstract and claims. | 1-8, 12-16 |
| X | WO 03051366 A2 (ABBOTT LAB [US]) 26 June 2003 (2003-06-26) See abstract and claims. | 1-8, 12-16 |
| X<br>A | WO 02064545 A1 (AVENTIS PHARMA GMBH [DE]) 22 August 2002 (2002-08-22) See abstract and claims. | 1-6, 12-16<br>7-8 |
| X<br>A | WO 2006006569 A1 (NIHON NOHYAKU CO LTD [JP]) 19 January 2006 (2006-01-19) See abstract and claims. | 1-6, 12-16<br>7-8 |
| X<br>A | WO 2012151567 A1 (ST JUDE CHILDRENS RES HOSPITAL) 08 November 2012 (2012-11-08) See abstract and claims. | 1-6, 12-16<br>7-8 |
| X<br>A | WO 2006065204 A1 (ASTRAZENECA AB [SE]) 22 June 2006 (2006-06-22) See abstract and claims. | 1-6, 12-16<br>7-8 |

Form PCT/ISA/210 (second sheet) (July 2022)

INTERNATIONAL SEARCH REPORT

International application No.
**PCT/BR2024/050028**

**C. DOCUMENTS CONSIDERED TO BE RELEVANT**

| Category* | Citation of document, with indication, where appropriate, of the relevant passages | Relevant to claim No. |
|---|---|---|
| X<br>A | WO 0151456 A2 (TULARIK INC [US]) 19 July 2001 (2001-07-19)<br>See abstract and claims. | 1-6, 12-16<br>7-8 |
| X<br>A | WO 2008096005 A1 (BASF SE [DE]) 14 August 2008 (2008-08-14)<br>See abstract and claims. | 1-6, 12-16<br>7-8 |
| X<br>A | WO 2010099279 A1 (DOW AGROSCIENCES LLC [US]) 02 September 2010 (2010-09-02)<br>See abstract and claims. | 1-6, 12-16<br>7-8 |
| X<br>A | JP 2004203871 A (YAMANOUCHI PHARMA CO LTD) 22 July 2004 (2004-07-22)<br>See abstract and claims. | 1-6, 12-16<br>7-8 |
| X<br>A | US 4607053 A (SQUIBB & SONS INC [US]) 19 August 1986 (1986-08-19)<br>See abstract and claims. | 1-6, 12-16<br>7-8 |
| X<br>A | CN 108503501 A (UNIV ZHEJIANG TECHNOLOGY) 07 September 2018 (2018-09-07)<br>See abstract and claims. | 1-6, 12-16<br>7-8 |
| Y | WO 2010077624 A1 (MERCK SHARP & DOHME [US]) 08 July 2010 (2010-07-08)<br>See abstract and claims. | 1-8, 12-16 |
| Y | Schenkel LB, DiMauro EF, Nguyen HN, Chakka N, Du B, Foti RS, Guzman-Perez A, Jarosh M, La DS, Ligutti J, Milgram BC, Moyer BD, Peterson EA, Roberts J, Yu VL, Weiss MM. Discovery of a biarylamide series of potent, state-dependent NaV1.7 inhibitors. Bioorg Med Chem Lett. 2017 Aug 15;27(16):3817-3824. doi: 10.1016/j.bmcl.2017.06.054. Epub 2017 Jun 26. PMID: 28684121. See the whole document. | 1-8, 12-16 |
| Y | WO 2012007836 A1 (PURDUE PHARMA LP [US]) 19 January 2012 (2012-01-19)<br>See abstract and claims. | 1-8, 12-16 |
| Y | WO 2008130322 A1 (ASTRAZENECA AB [SE]) 30 October 2008 (2008-10-30)<br>See abstract and claims. | 1-8, 12-16 |
| Y | WO 2008130323 A1 (ASTRAZENECA AB [SE]) 30 October 2008 (2008-10-30)<br>See abstract and claims. | 1-8, 12-16 |
| A | WO 2020138271 A1 (RAQUALIA PHARMA INC [JP]) 02 July 2020 (2020-07-02)<br>The whole document | 1-8, 12-16 |
| A | WO 2018235851 A1 (RAQUALIA PHARMA INC [JP]) 27 December 2018 (2018-12-27)<br>The whole document | 1-8, 12-16 |
| A | Lopes AB, Miguez E, Kümmerle AE, Rumjanek VM, Fraga CA, Barreiro EJ. Characterization of amide bond conformers for a novel heterocyclic template of N-acylhydrazone derivatives. Molecules. 2013 Sep 25;18(10):11683-704. doi: 10.3390/molecules181011683. PMID: 24071978; PMCID: PMC6270085. | 1-8, 12-16 |
| A | da Silva YK, Augusto CV, de Castro Barbosa ML, de Albuquerque Melo GM, de Queiroz AC, de Lima Matos Freire Dias T, Júnior WB, Barreiro EJ, Lima LM, Alexandre-Moreira MS. Synthesis and pharmacological evaluation of pyrazine N-acylhydrazone derivatives designed as novel analgesic and anti-inflammatory drug candidates. Bioorg Med Chem. 2010 Jul 15;18(14):5007-15. doi: 10.1016/j.bmc.2010.06.002. Epub 2010 Jun 8. PMID: 20598893. | 1-8, 12-16 |

Form PCT/ISA/210 (second sheet) (July 2022)

**INTERNATIONAL SEARCH REPORT**

International application No. **PCT/BR2024/050028**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **9-11**
because they relate to subject matter not required to be searched by this Authority, namely:
Claims 9-11 contain subject matter that falls under the provisions of PCT Rule 39.1(iv) concerning methods for treatment of the human or animal body by surgery or therapy.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/BR2024/050028**

| Patent document cited in search report | Publication date (day/month/year) | Patent family member(s) | Publication date (day/month/year) |
|---|---|---|---|
| WO 2009058298 A1 | 07 May 2009 | AU 2008319308 A1 | 07 May 2009 |
| | | AU 2008319308 B2 | 31 January 2013 |
| | | CA 2703909 A1 | 07 May 2009 |
| | | EP 2215049 A1 | 11 August 2010 |
| | | EP 2215049 A4 | 27 June 2012 |
| | | EP 2215049 B1 | 12 June 2019 |
| | | JP 2011502148 A | 20 January 2011 |
| | | US 2010266714 A1 | 21 October 2010 |
| | | US 8247401 B2 | 21 August 2012 |
| WO 2008000645 A1 | 03 January 2008 | AR 061667 A1 | 10 September 2008 |
| | | AU 2007263809 A1 | 03 January 2008 |
| | | AU 2007263809 B2 | 18 July 2013 |
| | | BR 122019017036 B1 | 09 June 2020 |
| | | BR 122019017036 B8 | 27 July 2021 |
| | | BR PI0714315 A2 | 28 May 2013 |
| | | BR PI0714315 B1 | 24 March 2020 |
| | | BR PI0714315 B8 | 25 May 2021 |
| | | CA 2654915 A1 | 03 January 2008 |
| | | CA 2654915 C | 28 July 2015 |
| | | CL 2007001887 A1 | 08 February 2008 |
| | | CN 101479250 A | 08 July 2009 |
| | | CN 101479250 B | 22 May 2013 |
| | | CN 103159692 A | 19 June 2013 |
| | | CN 103159692 B | 18 March 2015 |
| | | EP 2038264 A1 | 25 March 2009 |
| | | EP 2038264 B1 | 26 October 2016 |
| | | EP 2592070 A2 | 15 May 2013 |
| | | EP 2592070 A3 | 22 May 2013 |
| | | EP 2592070 B1 | 31 August 2016 |
| | | ES 2596706 T3 | 11 January 2017 |
| | | ES 2604542 T3 | 07 March 2017 |
| | | HK 1184453 A1 | 24 January 2014 |
| | | IL 195786 A | 26 February 2015 |
| | | JP 2009541415 A | 26 November 2009 |
| | | JP 5021734 B2 | 12 September 2012 |
| | | JP 2012197303 A | 18 October 2012 |
| | | JP 5456846 B2 | 02 April 2014 |
| | | KR 20090014221 A | 06 February 2009 |
| | | KR 101103143 B1 | 04 January 2012 |
| | | MX 2008016423 A | 22 January 2009 |
| | | TW 200808747 A | 16 February 2008 |
| | | TW I334414 B | 11 December 2010 |
| | | US 2017143672 A1 | 25 May 2017 |
| | | US 10201525 B2 | 12 February 2019 |
| | | US 2008004442 A1 | 03 January 2008 |
| | | US 7595405 B2 | 29 September 2009 |
| | | US 2009326220 A1 | 31 December 2009 |
| | | US 8193368 B2 | 05 June 2012 |
| | | US 2012214789 A1 | 23 August 2012 |
| | | US 8846945 B2 | 30 September 2014 |

Form PCT/ISA/210 (patent family annex) (July 2022)

**INTERNATIONAL SEARCH REPORT**
**Information on patent family members**

International application No.
**PCT/BR2024/050028**

| Patent document cited in search report | Publication date (day/month/year) | Patent family member(s) | Publication date (day/month/year) |
|---|---|---|---|
| | | US 2015191487 A1 | 09 July 2015 |
| | | US 9593128 B2 | 14 March 2017 |
| WO 2009077371 A1 | 25 June 2009 | AU 2008337665 A1 | 25 June 2009 |
| | | AU 2008337665 B2 | 16 January 2014 |
| | | BR 122019016794 B1 | 25 August 2020 |
| | | BR 122019016794 B8 | 27 July 2021 |
| | | BR PI0820838 A2 | 16 June 2015 |
| | | BR PI0820838 B1 | 24 December 2019 |
| | | BR PI0820838 B8 | 25 May 2021 |
| | | CA 2708228 A1 | 25 June 2009 |
| | | CA 2708228 C | 21 June 2016 |
| | | CN 101903362 A | 01 December 2010 |
| | | CN 101903362 B | 17 July 2013 |
| | | CN 103214464 A | 24 July 2013 |
| | | CN 103214464 B | 18 February 2015 |
| | | EP 2234989 A1 | 06 October 2010 |
| | | EP 2234989 B1 | 13 August 2014 |
| | | EP 2570407 A1 | 20 March 2013 |
| | | EP 2570407 B1 | 13 August 2014 |
| | | ES 2517518 T3 | 03 November 2014 |
| | | ES 2517602 T3 | 03 November 2014 |
| | | HK 1186465 A1 | 14 March 2014 |
| | | IL 206105 A | 30 September 2014 |
| | | IL 234251 A | 31 May 2015 |
| | | IL 238702 A | 30 November 2016 |
| | | JP 2011506523 A | 03 March 2011 |
| | | JP 5456691 B2 | 02 April 2014 |
| | | JP 2014058539 A | 03 April 2014 |
| | | JP 5745596 B2 | 08 July 2015 |
| | | KR 20100077219 A | 07 July 2010 |
| | | KR 101290385 B1 | 29 July 2013 |
| | | US 2009163502 A1 | 25 June 2009 |
| | | US 8048905 B2 | 01 November 2011 |
| | | US 2012015944 A1 | 19 January 2012 |
| | | US 8841336 B2 | 23 September 2014 |
| | | US 2015065506 A1 | 05 March 2015 |
| | | US 9512089 B2 | 06 December 2016 |
| WO 2006053109 A1 | 18 May 2006 | TW 200628463 A | 16 August 2006 |
| | | US 2006122156 A1 | 08 June 2006 |
| | | US 7919487 B2 | 05 April 2011 |
| CN 104876912 A | 02 September 2015 | CN 104876912 B | 21 July 2017 |
| CN 110407824 A | 05 November 2019 | CN 110407824 B | 02 July 2021 |
| WO 2012129562 A2 | 27 September 2012 | WO 2012129562 A3 | 31 January 2013 |
| | | US 2017112843 A1 | 27 April 2017 |
| | | US 10166237 B2 | 01 January 2019 |
| | | US 2014113012 A1 | 24 April 2014 |
| | | US 9464065 B2 | 11 October 2016 |
| WO 2015048547 A2 | 02 April 2015 | WO 2015048547 A3 | 18 June 2015 |
| | | US 2015087673 A1 | 26 March 2015 |

Form PCT/ISA/210 (patent family annex) (July 2022)

**INTERNATIONAL SEARCH REPORT**
**Information on patent family members**

International application No.
**PCT/BR2024/050028**

| Patent document cited in search report | Publication date (day/month/year) | Patent family member(s) | Publication date (day/month/year) |
|---|---|---|---|
| WO 03051366 A2 | 26 June 2003 | WO 03051366 A3 | 25 March 2004 |
| | | US 2003187026 A1 | 02 October 2003 |
| | | US 2003199511 A1 | 23 October 2003 |
| | | US 6831175 B2 | 14 December 2004 |
| WO 02064545 A1 | 22 August 2002 | AR 035430 A1 | 26 May 2004 |
| | | AU 2002253010 B2 | 28 February 2008 |
| | | AU 2002253010 B9 | 31 July 2008 |
| | | AU 2002253010 B8 | 02 April 2009 |
| | | BG 108076 A | 31 May 2005 |
| | | BR 0207211 A | 27 January 2004 |
| | | CA 2437944 A1 | 22 August 2002 |
| | | CA 2437944 C | 14 December 2010 |
| | | CN 1491207 A | 21 April 2004 |
| | | CN 1259307 C | 14 June 2006 |
| | | CR 7042 A | 15 April 2004 |
| | | CZ 20032155 A3 | 12 November 2003 |
| | | DK 1373191 T3 | 26 August 2013 |
| | | DZ 3491 A1 | 22 August 2002 |
| | | EC SP034729 A | 28 October 2003 |
| | | EE 200300369 A | 15 October 2003 |
| | | EE 05217 B1 | 15 October 2009 |
| | | EP 1373191 A1 | 02 January 2004 |
| | | EP 1373191 B1 | 05 June 2013 |
| | | ES 2425939 T3 | 18 October 2013 |
| | | HK 1061015 A1 | 03 September 2004 |
| | | HR P20030644 A2 | 31 August 2005 |
| | | HR P20030644 B1 | 31 March 2012 |
| | | HU P0303256 A2 | 28 January 2004 |
| | | HU P0303256 A3 | 28 April 2010 |
| | | IL 157209 A | 15 April 2010 |
| | | JP 2004518719 A | 24 June 2004 |
| | | JP 5073149 B2 | 14 November 2012 |
| | | JP 2010100622 A | 06 May 2010 |
| | | JP 5215975 B2 | 19 June 2013 |
| | | KR 20030077617 A | 01 October 2003 |
| | | KR 100998161 B1 | 06 December 2010 |
| | | MA 26981 A1 | 20 December 2004 |
| | | MX PA03006974 A | 02 April 2004 |
| | | MY 148640 A | 15 May 2013 |
| | | NO 20033565 D0 | 12 August 2003 |
| | | NO 20033565 L | 13 October 2003 |
| | | NO 328709 B1 | 03 May 2010 |
| | | NZ 527470 A | 29 April 2005 |
| | | OA 12444 A | 23 May 2006 |
| | | PE 20020858 A1 | 07 November 2002 |
| | | PL 366660 A1 | 07 February 2005 |
| | | PL 209114 B1 | 29 July 2011 |
| | | PT 1373191 E | 17 July 2013 |
| | | RU 2003127682 A | 10 March 2005 |
| | | RU 2339614 C2 | 27 November 2008 |

Form PCT/ISA/210 (patent family annex) (July 2022)

**INTERNATIONAL SEARCH REPORT**
**Information on patent family members**

International application No.
**PCT/BR2024/050028**

| Patent document cited in search report | Publication date (day/month/year) | Patent family member(s) | Publication date (day/month/year) |
|---|---|---|---|
| | | SK 10102003 A3 | 08 January 2004 |
| | | SV 2006000883 A | 28 September 2006 |
| | | TN SN03044 A1 | 23 December 2005 |
| | | TW I243164 B | 11 November 2005 |
| | | UA 75121 C2 | 15 March 2006 |
| | | US 2003055093 A1 | 20 March 2003 |
| | | US 7179839 B2 | 20 February 2007 |
| | | US 2007082897 A1 | 12 April 2007 |
| | | US 7713963 B2 | 11 May 2010 |
| | | US 2010183712 A1 | 22 July 2010 |
| | | US 8163751 B2 | 24 April 2012 |
| | | UY 27166 A1 | 31 July 2002 |
| | | YU 63003 A | 25 May 2006 |
| | | ZA 200305413 B | 28 April 2004 |
| WO 2006006569 A1 | 19 January 2006 | NONE | |
| WO 2012151567 A1 | 08 November 2012 | US 2014079666 A1 | 20 March 2014 |
| | | US 9328075 B2 | 03 May 2016 |
| WO 2006065204 A1 | 22 June 2006 | EP 1831170 A1 | 12 September 2007 |
| | | EP 1831170 A4 | 14 October 2009 |
| | | JP 2008523139 A | 03 July 2008 |
| | | US 2008287399 A1 | 20 November 2008 |
| WO 0151456 A2 | 19 July 2001 | WO 0151456 A3 | 20 December 2001 |
| | | AT E376996 T1 | 15 November 2007 |
| | | AU 3972501 A | 24 July 2001 |
| | | CA 2397575 A1 | 19 July 2001 |
| | | DE 60131138 D1 | 13 December 2007 |
| | | DE 60131138 T2 | 14 August 2008 |
| | | EP 1246795 A2 | 09 October 2002 |
| | | EP 1246795 B1 | 31 October 2007 |
| | | ES 2293980 T3 | 01 April 2008 |
| | | JP 2003519676 A | 24 June 2003 |
| | | US 2002045749 A1 | 18 April 2002 |
| | | US 6780858 B2 | 24 August 2004 |
| | | US 2004235911 A1 | 25 November 2004 |
| | | US 7053234 B2 | 30 May 2006 |
| | | US 2006270651 A1 | 30 November 2006 |
| | | US 7148259 B1 | 12 December 2006 |
| WO 2008096005 A1 | 14 August 2008 | AR 065288 A1 | 27 May 2009 |
| | | AT E542421 T1 | 15 February 2012 |
| | | AU 2008212764 A1 | 14 August 2008 |
| | | AU 2008212764 B2 | 29 November 2012 |
| | | BR PI0807864 A2 | 17 June 2014 |
| | | BR PI0807864 A8 | 08 March 2016 |
| | | BR PI0807864 B1 | 02 February 2021 |
| | | CA 2675627 A1 | 14 August 2008 |
| | | CA 2675627 C | 22 April 2014 |
| | | CL 2008000395 A1 | 04 July 2008 |
| | | CR 10984 A | 15 October 2009 |
| | | CY 1112576 T1 | 10 February 2016 |

Form PCT/ISA/210 (patent family annex) (July 2022)

**INTERNATIONAL SEARCH REPORT**
**Information on patent family members**

International application No.
**PCT/BR2024/050028**

| Patent document cited in search report | Publication date (day/month/year) | Patent family member(s) | Publication date (day/month/year) |
|---|---|---|---|
| | | DK 2117297 T3 | 21 May 2012 |
| | | EA 200901081 A1 | 26 February 2010 |
| | | EA 017040 B1 | 28 September 2012 |
| | | EC SP099615 A | 30 October 2009 |
| | | EP 2117297 A1 | 18 November 2009 |
| | | EP 2117297 B1 | 25 January 2012 |
| | | ES 2378909 T3 | 19 April 2012 |
| | | HR P20120198 T1 | 31 March 2012 |
| | | JP 2010518050 A | 27 May 2010 |
| | | JP 5523838 B2 | 18 June 2014 |
| | | KR 20090118962 A | 18 November 2009 |
| | | KR 101488034 B1 | 30 January 2015 |
| | | MX 2009007733 A | 08 October 2009 |
| | | PE 20081754 A1 | 31 January 2009 |
| | | PL 2117297 T3 | 31 July 2012 |
| | | PT 2117297 E | 08 February 2012 |
| | | RS 52230 B | 31 October 2012 |
| | | SI 2117297 T1 | 30 March 2012 |
| | | TW 200901889 A | 16 January 2009 |
| | | UA 97973 C2 | 10 April 2012 |
| | | US 2010113543 A1 | 06 May 2010 |
| | | US 8859607 B2 | 14 October 2014 |
| | | ZA 200906209 B | 24 November 2010 |
| WO 2010099279 A1 | 02 September 2010 | AR 075635 A1 | 20 April 2011 |
| | | BR PI1008770 A2 | 25 August 2015 |
| | | BR PI1008770 B1 | 21 February 2017 |
| | | BR PI1008770 B8 | 31 July 2018 |
| | | CA 2750539 A1 | 02 September 2010 |
| | | EP 2421832 A1 | 29 February 2012 |
| | | EP 2421832 B1 | 18 December 2013 |
| | | MX 2011009024 A | 29 September 2011 |
| | | PL 2421832 T3 | 30 May 2014 |
| | | US 2010222221 A1 | 02 September 2010 |
| | | US 8536331 B2 | 17 September 2013 |
| | | US 2013324412 A1 | 05 December 2013 |
| | | US 8889694 B2 | 18 November 2014 |
| JP 2004203871 A | 22 July 2004 | NONE | |
| US 4607053 A | 19 August 1986 | AU 4254685 A | 21 November 1985 |
| | | AU 586973 B2 | 03 August 1989 |
| | | DE 3587460 D1 | 26 August 1993 |
| | | DE 3587460 T2 | 17 February 1994 |
| | | DK 217385 D0 | 15 May 1985 |
| | | DK 217385 A | 18 November 1985 |
| | | EP 0161939 A2 | 21 November 1985 |
| | | EP 0161939 A3 | 08 October 1986 |
| | | EP 0161939 B1 | 21 July 1993 |
| | | ES 8609201 A1 | 01 September 1986 |
| | | ES 8704872 A1 | 16 April 1987 |
| | | ES 8800129 A1 | 16 October 1987 |
| | | ES 8802378 A1 | 16 May 1988 |

Form PCT/ISA/210 (patent family annex) (July 2022)

**INTERNATIONAL SEARCH REPORT**
**Information on patent family members**

International application No.
**PCT/BR2024/050028**

| Patent document cited in search report | Publication date (day/month/year) | Patent family member(s) | Publication date (day/month/year) |
|---|---|---|---|
| | | ES 8706604 A1 | 01 July 1987 |
| | | ES 8706605 A1 | 01 July 1987 |
| | | ES 8706606 A1 | 01 July 1987 |
| | | FI 851970 L | 18 November 1985 |
| | | GR 851203 B | 25 November 1985 |
| | | HU T37742 A | 28 February 1986 |
| | | HU 200594 B | 28 July 1990 |
| | | IL 75223 A | 31 May 1988 |
| | | KR 850008660 A | 21 December 1985 |
| | | KR 910000855 B1 | 11 February 1991 |
| | | NZ 212099 A | 29 August 1989 |
| | | PH 21267 A | 28 September 1987 |
| CN 108503501 A | 07 September 2018 | CN 108503501 B | 21 September 2021 |
| WO 2010077624 A1 | 08 July 2010 | EP 2375904 A1 | 19 October 2011 |
| | | EP 2375904 A4 | 12 September 2012 |
| | | EP 2375904 B1 | 28 May 2014 |
| | | US 2011230498 A1 | 22 September 2011 |
| | | US 8367679 B2 | 05 February 2013 |
| WO 2012007836 A1 | 19 January 2012 | EP 2593434 A1 | 22 May 2013 |
| | | JP 2013531687 A | 08 August 2013 |
| | | US 2013345211 A1 | 26 December 2013 |
| | | US 9120752 B2 | 01 September 2015 |
| | | US 2015344465 A1 | 03 December 2015 |
| | | US 9765029 B2 | 19 September 2017 |
| WO 2008130322 A1 | 30 October 2008 | NONE | |
| WO 2008130323 A1 | 30 October 2008 | NONE | |
| WO 2020138271 A1 | 02 July 2020 | BR 112021008995 A2 | 10 August 2021 |
| | | CA 3120268 A1 | 02 July 2020 |
| | | CN 113164461 A | 23 July 2021 |
| | | EP 3902544 A1 | 03 November 2021 |
| | | JP 2022515630 A | 21 February 2022 |
| | | KR 20210107698 A | 01 September 2021 |
| | | MX 2021007425 A | 05 August 2021 |
| | | TW 202038950 A | 01 November 2020 |
| | | US 2022048892 A1 | 17 February 2022 |
| WO 2018235851 A1 | 27 December 2018 | AU 2018289731 A1 | 14 November 2019 |
| | | AU 2018289731 B2 | 18 November 2021 |
| | | BR 112019021736 A2 | 05 May 2020 |
| | | CA 3059687 A1 | 27 December 2018 |
| | | CN 110612285 A | 24 December 2019 |
| | | CN 110612285 B | 04 April 2023 |
| | | DK 3487839 T3 | 18 January 2021 |
| | | EP 3487839 A1 | 29 May 2019 |
| | | EP 3487839 A4 | 18 March 2020 |
| | | EP 3487839 B1 | 23 December 2020 |
| | | ES 2851004 T3 | 02 September 2021 |
| | | HU E053460 T2 | 28 June 2021 |
| | | JP 6592702 B1 | 23 October 2019 |
| | | JP 2019532911 A | 14 November 2019 |

Form PCT/ISA/210 (patent family annex) (July 2022)

**INTERNATIONAL SEARCH REPORT**
**Information on patent family members**

International application No.
**PCT/BR2024/050028**

| Patent document cited in search report | Publication date (day/month/year) | Patent family member(s) | Publication date (day/month/year) |
|---|---|---|---|
| | | KR 20200015888 A | 13 February 2020 |
| | | KR 102577137 B1 | 11 September 2023 |
| | | MX 2019012635 A | 11 December 2019 |
| | | PH 12019502850 A1 | 28 September 2020 |
| | | PL 3487839 T3 | 14 June 2021 |
| | | PT 3487839 T | 11 February 2021 |
| | | RU 2019133530 A | 20 July 2021 |
| | | RU 2019133530 A3 | 04 August 2021 |
| | | RU 2768149 C2 | 23 March 2022 |
| | | SG 11201909793Y A | 28 November 2019 |
| | | TW 201904944 A | 01 February 2019 |
| | | TW I769266 B | 01 July 2022 |
| | | US 2020016135 A1 | 16 January 2020 |
| | | US 11154544 B2 | 26 October 2021 |
| | | ZA 201906888 B | 24 February 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 10550080 B
- US 9765029 B
- US 10000475 B
- WO 2020261114 A
- WO 2020092667 A
- US 9163042 B
- WO 2014120808 A
- WO 2014120815 A
- WO 2018213426 A
- WO 2019014352 A
- WO 2015006280 A
- US 7928107 B
- WO 2018235851 A
- US 8629149 B
- JP 2017001991 B

### Non-patent literature cited in the description


- **SMITH**. *Pain*, 2020, vol. 161 (1), S127
- **CAVALLI**. *Int. J. Immunopathol. Pharmacol.*, 2019, vol. 33, 2058738419838383
- **BOUHASSIRA**. *Rev Neurol (Paris).*, 2019, vol. 175 (1-2), 16
- **SCHOLZ**. *Nature Neurosci.*, 2002, vol. 5, 1062
- **COSTIGAN**. *Annu. Rev. Neurosci.*, 2009, vol. 32, 1
- **DWORKIN**. *Clin. J. Pain*, 2002, vol. 18 (6), 343
- **DUCREUX**. *Brain*, 2006, vol. 129, 963
- **PAK**. *Curr. Pain Headache Rep.*, 2018, vol. 22 (2), 9
- **KUSHNAREV**. *Expert Opin. Investig. Drugs*, 2020, vol. 29 (3), 259
- **EMERY**. *Expert Opin. Ther. Targets*, 2016, vol. 20 (8), 975
- **CATTERALL**. *Nat. Chem. Biol.*, 2020, vol. 16, 1314
- **WISEDCHAISRI**. *Cell*, 2019, vol. 178 (4), 993
- **CLAIRFEUILLE**. *Science*, 2019, vol. 363, 1302
- **LERA-RUIZ**. *J. Med. Chem.*, 2015, vol. 58 (18), 7093
- **BAGAL**. *J. Med. Chem.*, 2013, vol. 56 (3), 593
- **BAGAL**. *Channels*, 2015, vol. 9 (6), 360
- **LAW**. *Drug Discovery Today*, 2019, vol. 24 (7), 1389
- **BAGAL**. *Channels (Austin)*, 2015, vol. 9 (6), 360
- **VETTER**. *Pharmacology & Therapeutics*, 2017, vol. 172, 73
- **AHUJA**. *Science*, 2015, vol. 350 (6267), 1491
- **KINGWELL**. *Nat. Rev. Drug Discov.*, 2019, vol. 18, 321
- **SAFINA**. *J. Med. Chem.*, 2021, vol. 64, 2953
- **LUO**. *J. Med. Chem.*, 2019, vol. 62, 831
- **BANKAR**. *Cell Reports*, 2018, vol. 24, 3133
- **BROWN**. *Bioorg. Med. Chem.*, 2019, vol. 27 (1), 230
- **PAYNE**. *Br. J. Pharmacol.*, 2015, vol. 172 (10), 2654
- **BAGAL**. *Med. Chem. Lett.*, 2015, vol. 6 (6), 650
- **KORT**. *J. Med. Chem.*, 2008, vol. 51, 407
- **ZHANG**. *Neuropharmacology*, 2010, vol. 59, 201-207
- **KORNECOOK**. *J. Pharmacol. Exp. Ther.*, 2017, vol. 362, 146
- **DEUIS**. *Neuropharmacology*, 2017, vol. 127, 87-108
- **MCKERRALL**. *Bioorganic & Medicinal Chemistry Lett.*, 2018, vol. 28, 3141
- **BAGAL**. *Bioorganic & Medicinal Chemistry Lett.*, 2014, vol. 24, 3690